# EUROPEAN PATENT APPLICATION

(11) **EP 4 717 762 A1**
(43) Date of publication of application: **01.04.2026**
(21) Application number: 24202899.1
(22) Date of filing: 26.09.2024
(51) Int. Cl.: C12N 1/20, C12N 9/10, C12P 7/26

(54) **CURCUMIN PRODUCING GRAM POSITIVE BACTERIA**

(71) Applicant: Universität des Saarlandes, eine Körperschaft des Öffentlichen Rechts, 66123 Saarbrücken (DE)
(72) Inventor: Wittmann, Christoph, 66740 Saarlouis (DE); Beganovic, Selma, 66123 Saarbrücken (DE); Weiland, Fabia, 66806 Ensdorf (DE)
(74) Representative: Schiweck Weinzierl Koch Patentanwälte Partnerschaft mbB

(57) **Abstract**

The present invention relates to a gram-positive bacterial host cell, which naturally has a phenylpropanoid pathway, wherein said host cell is genetically engineered to express curcuminoid biosynthetic genes, thereby being capable of producing curcumin. The present invention further relates to a method for producing curcumin, comprising culturing the bacterial host cell being capable of producing curcumin according to the present invention under suitable conditions in liquid medium, and harvesting curcumin. Furthermore, the present invention relates to the use of the bacterial host cell being capable of producing curcumin according to the present invention for the production of curcumin

## Description

The present invention relates to a gram-positive bacterial host cell, which naturally has a phenylpropanoid pathway, wherein said host cell is genetically engineered to express curcuminoid biosynthetic genes, thereby being capable of producing curcumin. The present invention further relates to a method for producing curcumin, comprising culturing the bacterial host cell being capable of producing curcumin according to the present invention under suitable conditions in liquid medium, and harvesting curcumin. Furthermore, the present invention relates to the use of the bacterial host cell being capable of producing curcumin according to the present invention for the production of curcumin.

Curcumin is a plant-derived polyphenol of recognized commercial value. Naturally, the compound is found in the roots of the perennial herb turmeric (Curcuma longa L.) to which it gives a bright orange colour and which led to its fame as "golden spice". Besides curcumin, there exist two other curcuminoids, namely demethoxycurcumin, and bisdemethoxycurcumin, and all three compounds are often simplified as a curcumin. The biosynthesis involves the joint activities of the phenylpropanoid pathway and a type III polyketide synthase (PKS) and proceeds via phenylpropanoid-derived CoA-thioesters as metabolic intermediates (Rodrigues et al., Microbiol Mol Biol Rev (2015), 79: 39-60). Over the years, curcumin has revealed multiple benefits for human. It suppresses cancer growth, protects against inflammation and helps to treat and prevent infectious and non-infectious diseases including malaria, tuberculosis, diabetes, Alzheimer's disease and Parkinson's disease. Furthermore, curcumin actively supports wound healing and mediates the appropriate modulation of our immune system, opening a wide range of therapeutic applications. In addition, curcumin is extensively used in the food (Prasad et al., Biotechnol Adv (2014), 32: 1053-1064) and cosmetic industries (Rafiee et al., Trends in Food Sci Technol (2019), 88: 445-458). Overall, this has created a rapidly increasing nutraceutical and pharmaceutical market that is projected to reach US$ 181 million by 2030 (2022).

Traditionally, curcumin is obtained as powdered dried rhizomes of turmeric. Agricultural production of the exhaustive crop, however, is restricted to tropical and subtropical regions, requires extensive farming with up to 40 irrigations per year and heavy manuring, while facing a high risk of harvest loss by fungal rhizome and root rot, several foliar diseases, and nematodes. During the growth and storage of the harvested rhizomes, turmeric is susceptible to diseases caused by fungi, bacteria and nematodes, resulting in decay and loss of biomass that can be used for subsequent extraction Altogether, the conventional techniques have a number of disadvantages: low yield, long operational times, high costs and the dependence on seasonal harvest. The final powdered products contain only 2-5% curcumin. Chemical synthesis of curcumin is also possible, but suffers from low yields and contamination of the product with the organic reagents, excluding a safe use in food and therapy. In this regard, microbial biosynthesis has emerged as an attractive alternative to derive curcumin (Rodrigues et al., Microbiol Mol Biol Rev (2015), 79: 39-60).

A rich set of biochemical studies unravelled the native biosynthetic pathways of curcumin (Katsuyama et al., FEBS Lett (2009), 583: 2799-2803; Katsuyama et al., J Biol Chem (2009), 284: 11160-11170; Katsuyama et al., J Biol chem (2011), 286: 6659-6668). Inter alia, this enabled the reconstruction of the metabolic routes in microbial hosts for heterologous curcumin production. Interestingly, the first microbial-based production of curcumin was accomplished by using a curcuminoid synthase (CUS) from rice (Oryza sativa L.) (Katsuyama et al., Micribiol (Reading) (2008), 154: 2620-2628), an enzyme that shares 51% identity with diketide synthase (DCS) and 45% with curcumin synthase 1 (CURS1) from turmeric (Rodrigues et al., Microbiol Mol Biol Rev (2015), 79: 39-60). In this study, 113 mg L-1 of curcumin was produced by recombinant *E. coli* using ferulate as a feeding substrate.

The first implementation of the biosynthetic genes from turmeric (DCS and CURS1) resulted in a titer of 70 mg L-1. Further optimization of the *E. coli* chassis and the bioprocess was done (Rodrigues et al., Front Bioeng Biotechnol (2020), 8: 59; Wu et al., J Agric Food Chem (2020), 68: 10772-10779).

Also other microorganisms were used for the production of curcumin, such as *Saccharomyces cerevisiae* (Rainha et al., Biotechnol J (2022), 17: e2100400), *Aspergillus oryze* (Kan et al., Biosci Biotechnol Biochem (2019), 83: 1372-1381), *Yarrowia lipolytica* (Palmer et al., Metab Eng (2020), 57: 174-181) were used (see also Beganovic and Wittmann (2024), Curr. Opin. Biotechnol. 87: 1031112; doi.org/10.1016/j.copbio.2024.103112). Titers for curcumin have been increased through further modifying thus far used microorganisms, but there is still room for further increasing titers.

Accordingly, there is still a need for further means and methods for the biotechnological production of curcumin.

The present invention therefore addresses this need and provides a solution as described herein, defined in the claims, demonstrated in the examples and illustrated in the figures herein.

The present invention relates to a gram-positive bacterial host cell, which naturally has a phenylpropanoid pathway, wherein said host cell is genetically engineered to express curcuminoid biosynthetic genes, thereby being capable of producing curcumin.

Up to the present invention, curcumin was produced in the typical model organism *E. coli.* Alternatively, *S*. *cerevisiae, A. oryze* or *Y. lipolytica* was used for the production of curcumin. However, no other microorganisms were used, let alone suggested in the prior art.

The present inventors, however, found that gram-positive bacterial host cells - exemplified by using corynebacteria, in particular C. glutamicum - are suitable microbial factories for the production of curcumin provided that these gram-positive bacterial host cells naturally have a phenyl propanoid pathway. The latter is an advantageous property observed by the present inventors. Such gram-positive bacterial host cells can then be genetically engineered with curcuminoid biosynthetic genes, thereby rendering such gram-positive bacterial host cells capable of producing curcumin.

The present inventors also observed that due to its pronounced lipophilic nature, curcumin may anchor deep into the cell membrane in a transbilayer orientation which may disturb integrity of a microorganism used for the production of curcumin. However, in case of gram-positive bacteria, particularly Corynebacterium, e.g. C. glutamicum, integrity was not disturbed to such an extent that, e.g. growth or vitality was compromised.

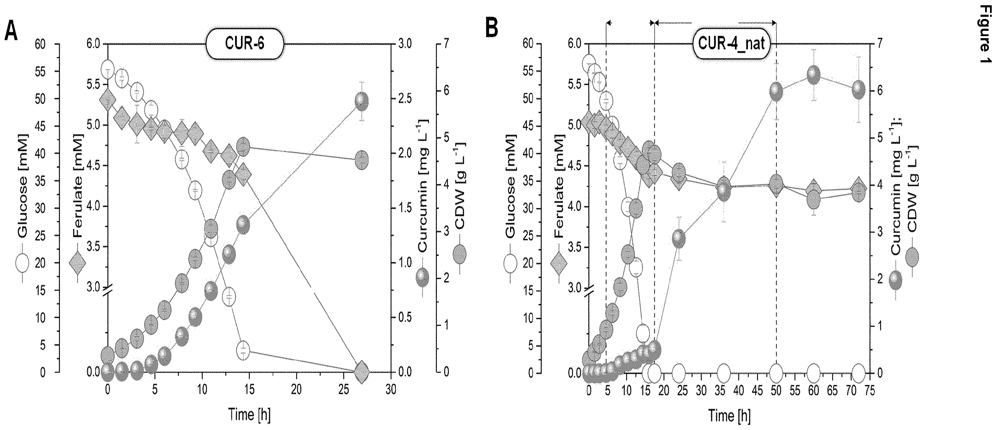

In addition to establishing gram-positive bacterial host cells, in particular corynebacteria, e.g. C. *glutamicum,* the present inventors found that inactivation of *phdBCDE* genes enabled an increase in formed curcumin, as compared to wild type expressing the dcs and *curs1* genes (see Figure 1). Also, the present inventors found that native curcumin biosynthetic genes from, e.g turmeric, dcs and *curs1,* provided for a further increase in the titer of curcumin (see Figure 1).

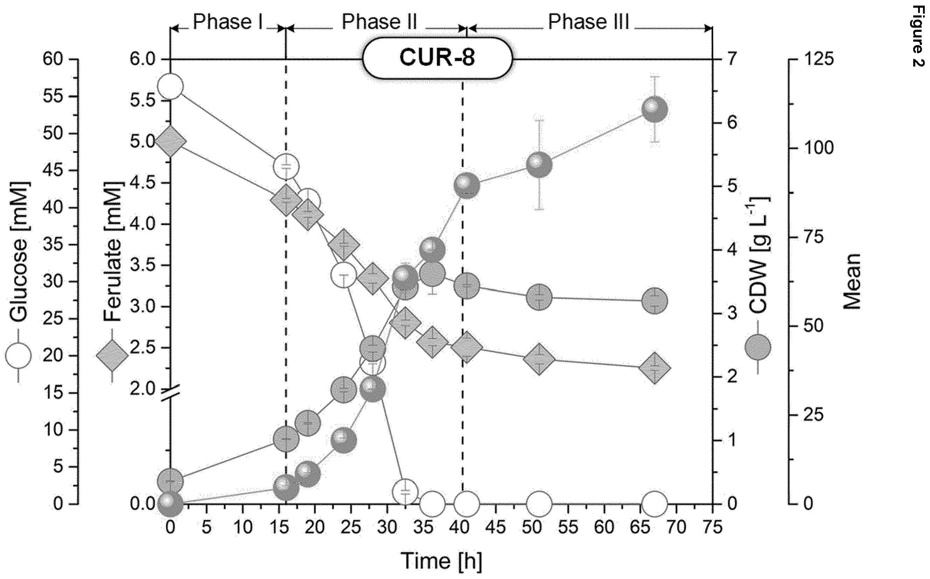

A further surprising finding is that the inactivation of *phdR* regulator provided for a further increase in production of curcumin (Figure 2).

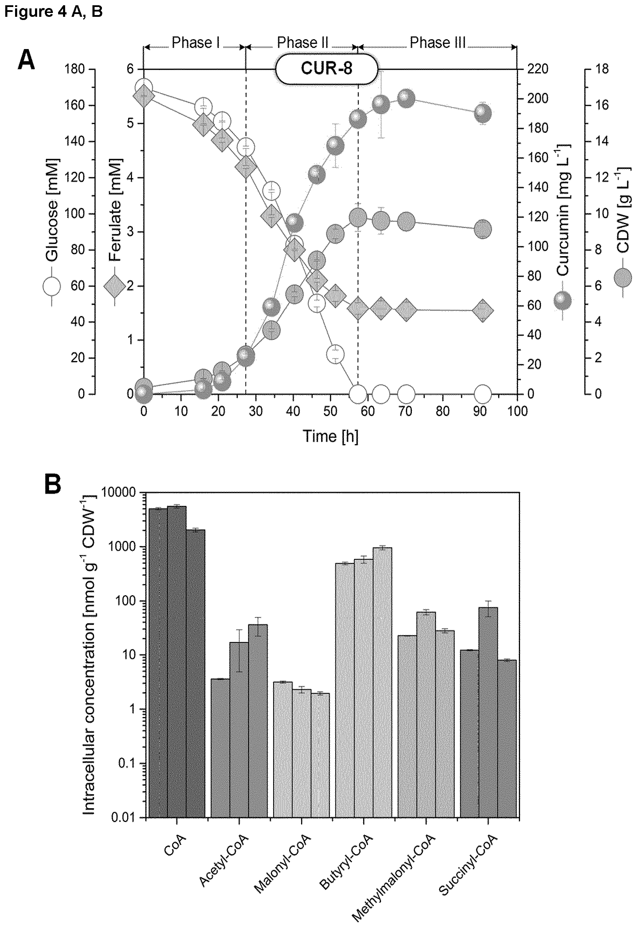

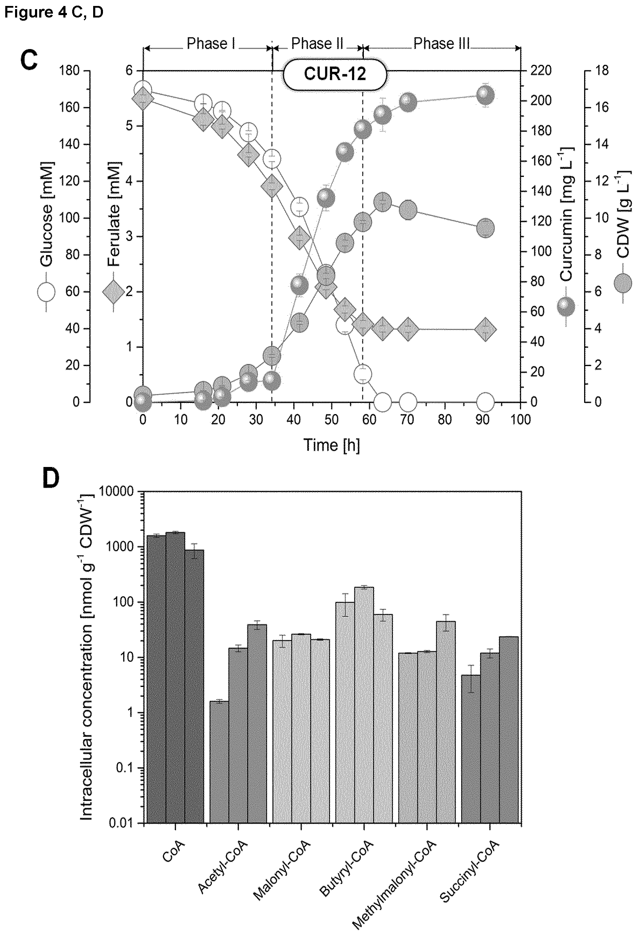

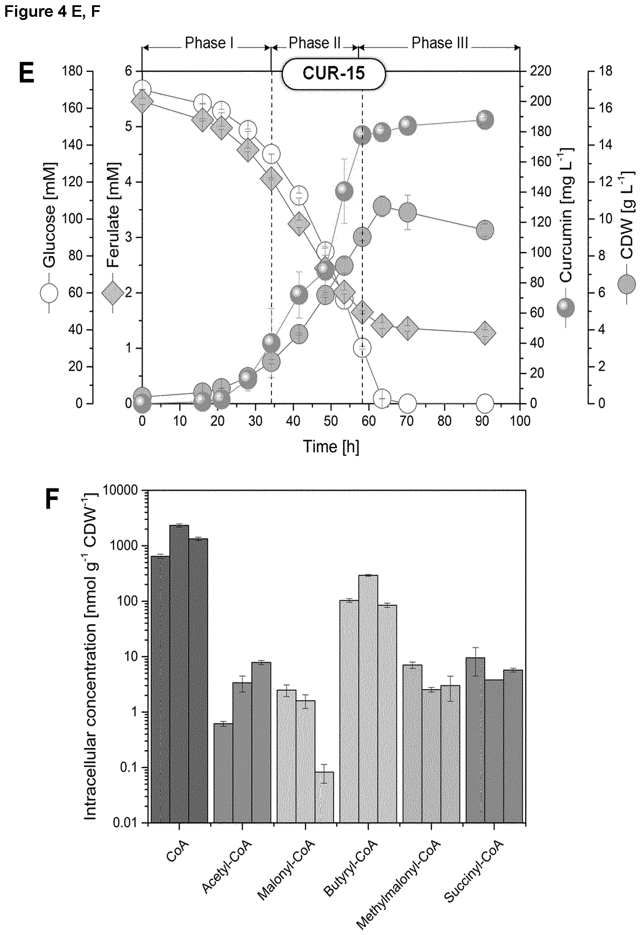

Attenuation of the expression of citrate synthase also contributed to an increase in curcumin production (Figure 4).

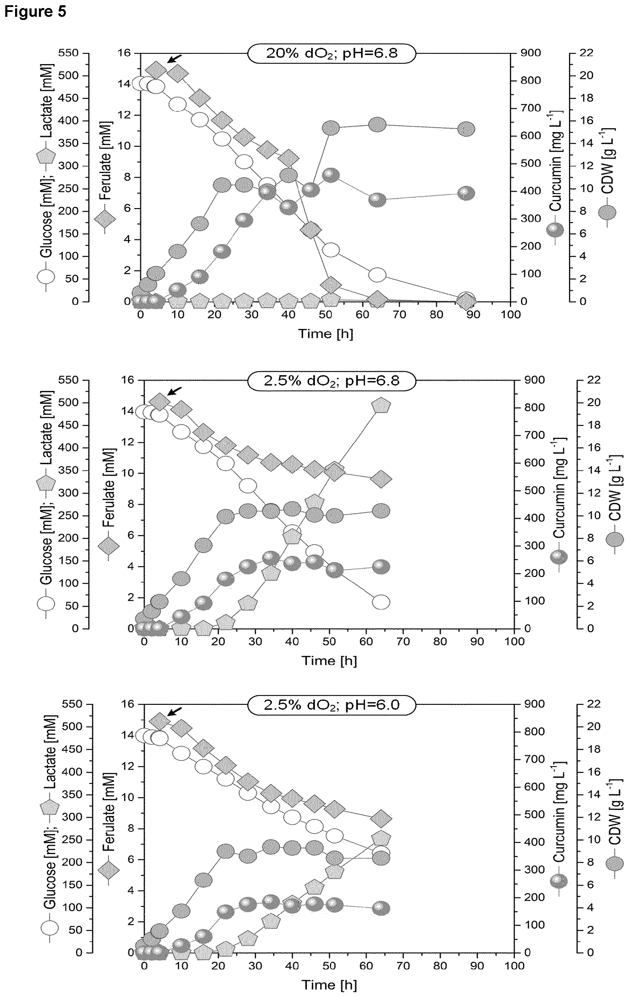

A yet further increase in the production of curcumin was achieved by optimization of glucose, ferulate, pH and/or oxygen supply. Given that ferulate, as curcumin precursor, is a common component of lingo-cellulose of monocot grains, valorisation of lignin or food waste streams would enable establishing sustainable production of curcumin (Figure 5).

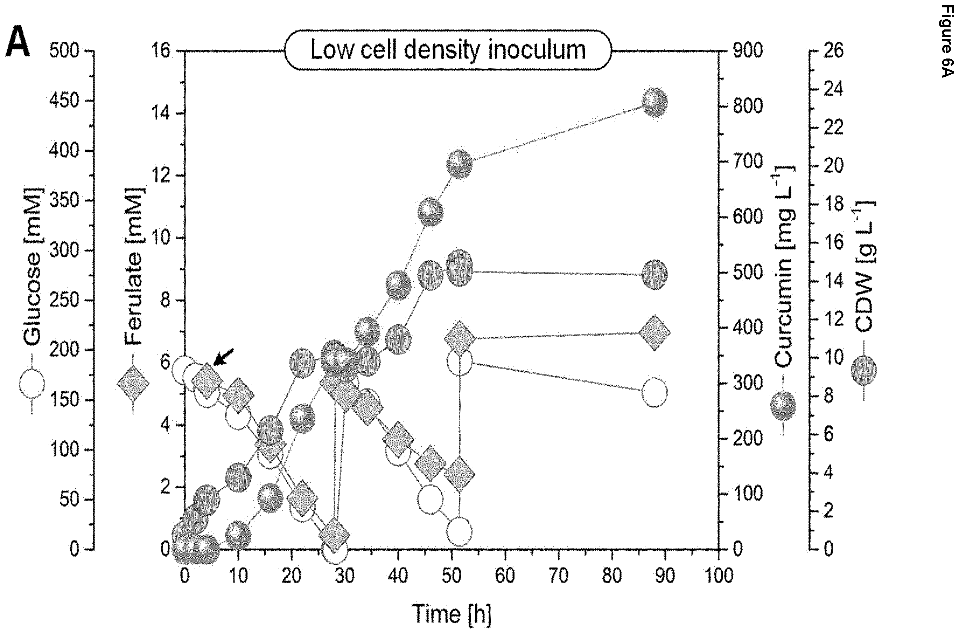

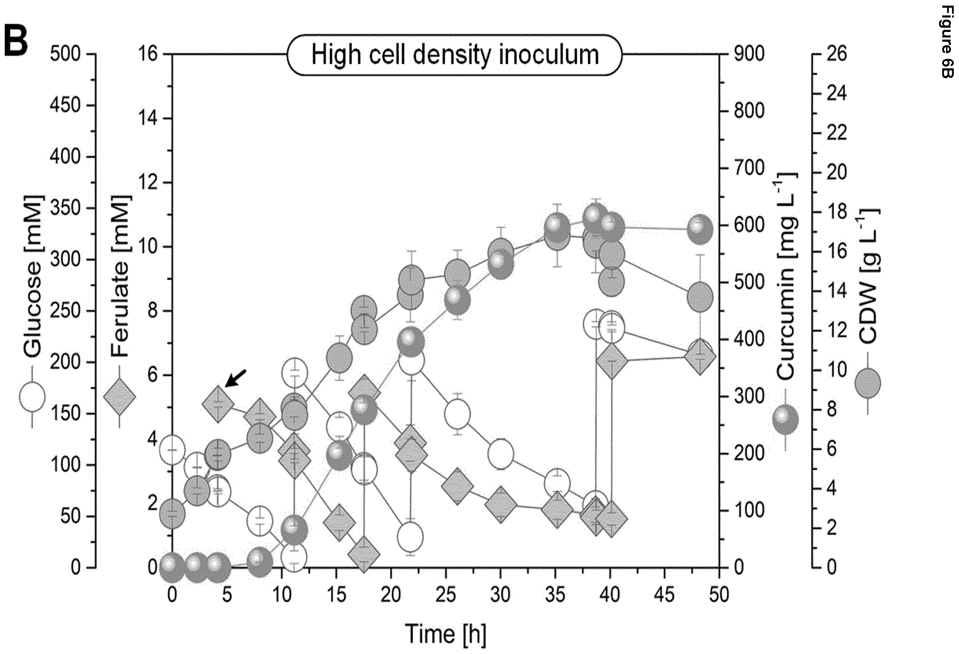

Finally, production of curcumin in fed-batch mode enabled achieving, to the best of our knowledge, the highest known titre of curcumin (Figure 6).

In context with the present invention, the term "curcuminoid" refers to curcumin and related compounds known in the art (Di Meo et al., Nutrients (2019), 11). Preferably, in the context of of the present invention, the term "curcuminoid" refers to curcumin, demethoxycurcumin and bisdemethoxycurcumin, particularly curcumin. Accordingly, a bacterial host cell of the present invention equipped with curcuminoid biosynthetic genes is capable of producing curcumin, demethoxycurcumin and bisdemethoxycurcumin, with curcumin being preferred. The skilled person is aware which curcuminoid biosynthetic genes are required such that a bacterial host cell of the present invention preferably produces curcumin.

Preferably, in the context of the present invention, the bacterial host cell is a Corynebacterium host cell, Bacillus host cell, Actinobacter host cell, or Streptomycetes host cell, with a Corynebacterium host cell, such as *C*. *glutamicum being preferred.*

When used herein, the term "bacterial host cell" may be sometimes abbreviated as "host cell" or "bacterial host" or simply "host" or the like, however, these terms may be interchangeably used herein and mean a gram-positive bacterial host cell, which naturally has a phenylpropanoid pathway, wherein said bacterial host cell is genetically engineered to express curcuminoid biosynthetic genes, thereby being capable of producing curcumin

The bacterial host cell of the present invention is characterized to naturally have a phenylpropanoid pathway. "Naturally" in this context means that a bacterial host cell has - without genetic engineering - genes which express proteins that convert metabolites to cinnamic acid, p-coumaric acid, caffeic acid and/or ferulic acid. These metabolites are from the shikimate pathway and/or aromatic amino acid biosynthesis pathway, particularly tyrosine (having been produced from chorismate and shikimate, respectively) and phenylalanine (having been produced from the aromatic amino acid biosynthesis); see Figure 2 of Rainha et al. (2022). Microbial Production of Curcumin. In: Jafari, S.M., Harzevili, F.D. (eds) Microbial Production of Food Bioactive Compounds. Springer, Cham. https://doi.org/10.1007/978-3-030-81403-8_8-1.

Accordingly, by "phenylpropanoid pathway", it is preferably meant that a bacterial host cell of the present invention naturally produces the phenylpropanoids cinnamic acid, p-coumaric acid, caffeic acid and/or ferulic acid, preferably ferulic acid.

That said, the present invention does not exclude that a bacterial host cell as described herein which naturally has a phenylpropanoid pathway may additionally be genetically engineered to contain - in addition to genes which express proteins that convert metabolites to cinnamic acid, p-coumaric acid, caffeic acid and ferulic acid - such genes which express proteins that convert metabolites to cinnamic acid, p-coumaric acid, caffeic acid and/or ferulic acid, preferably ferulic acid. Thus, a bacterial host cell which already naturally has a phenylpropanoid pathway may be equipped with one or more additional genes which provide for members of the phenylpropanoid pathway. Also, it is included within the scope of the present invention that genes of the phenylpropanoid pathway which a bacterial host cell naturally has may be modified to increase their expression, e.g. by replacing a native promoter with a promoter which is stronger than the native promoter.

The term "genetically engineered" means that a bacterial host cell of the present invention is one that has a genetic background which is at least partially due to manipulation, e.g. by transformation by the hand of man through the use of genetic engineering. Accordingly, a genetically engineered bacterial host cell as described herein refers to a bacterial host cell whose DNA contains an exogenous nucleic acid not originally present in the non-genetically engineered (or transformed) bacterial host cell.

Accordingly, said term includes (unless specified otherwise herein) that the respective bacterial host cell encodes and is capable to (stably) express a gene that it does naturally not contain and/or express. For example, such polynucleotide has been introduced into the respective bacterial host cell. Preferably, such polynucleotide represents an entire gene or ORF, encoding a complete or relevant fragment of a protein as described herein which a bacterial host cell of the present invention may not naturally harbor. The respective bacterial cell which is "genetically engineered" in accordance with this invention may contain the introduced polynucleotide either on an extrachromosomal molecule (e.g., on a plasmid) or the polynucleotide may be (stably) integrated into the or a cell chromosome. Preferably, it is introduced on a plasmid. The introduced polynucleotide may stand under the control of its own promoter or regulator or the like (introduced together with the polynucleotide), or under the control of a promoter or regulator or the like of the bacterial host cell (either alone or und common control together with an endogenous gene of the host cell). The latter promoter is a homologous promoter. However, in the context of the present invention, also a heterologous promoter may be used. Such a promoter is not from the bacterial host cell as described herein, but from another source, e.g. it may even be artificial.

Preferably, in the context of the present invention, the curcuminoid biosynthetic genes to be expressed in a genetically engineered bacterial host cell described and provided herein are under the control of a homologous or heterologous promoter. Such a promoter may preferably be a constitutive promoter, but may also be an inducible promoter.

A preferred homologous promoter used in the context of the present invention is the tuf promoter (Ptuf), sod promoter (Psod), gapA promoter (PgapA), sodA promoter (PsodA) or pgk promoter (Ppgk), with the tuf promoter being preferred.

The aforementioned promoters are also preferred constitutive promoters in the context of the present invention.

A preferred heterologous promoter used in the context of the present invention is the trc promoter (Ptrc). The trc promoter is also a preferred constitutive promoter in the context of the present invention.

A preferred inducible promoter used in the context of the present invention is the tac promoter (Ptac) or bad promoter (Pbad).

In context with the present invention, bacterial host cells, particularly corynebacterium (particularly *C. glutamicum*) host cells were genetically engineered to both allow and optimize curcumin production. Also, cultivation methods were adapted to further improve curcumin production of such genetically engineered bacterial host cells. As has been surprisingly found in context with the present invention, gram-positive bacterial host cell, exemplified by corynebacterium host cells, particularly *C. glutamicum,* exhibit superior features for increased curcumin production, e.g. increased tolerance to educts and curcumin itself, making them suitable as curcumin production host cell. In addition to genetic engineering of such bacterial host cells to express curcuminoid biosynthetic genes as provided in accordance with the present invention, further adaptations of the bacterial host cells like impacting the uptake of educts (e.g., ferulate, e.g., by deletion of *phdR*), inhibition of production of competing gene products (e.g., Δ*phdBCDE, ΔbenABCD*), increased expression of *phdA,* attenuation of the expression of citrate synthase (*gltA*), codon-optimizations, plasmid-selection and other modifications as described and provided herein add to the present invention for providing superior host cells for curcumin production.

Preferably, in the context of the present invention, said curcuminoid biosynthetic genes expressed in the bacterial host cell encode one or more selected from the group consisting of phenylalanine hydroxylase (PAH), phenylalanine ammonia lyase (PAL), tyrosine ammonia lyase (TAL), Cinnamate 4-hydroxylase (C4H), 4-coumarate 3-hydroxylase (C3H), Caffeic acid O-methlytransferase (COMT), Caffeoyl-CoA O-methyltransferase (CCoAOMT), 4-coumarate-CoA ligase (4CL), diketide-CoA synthase (DCS), curcuminoid synthase (CUS), curcumin synthase 1 (CURS1), curcumin synthase 2 (CURS2), and curcumin synthase 3 (CURS3).

Also preferred in the context of the present invention, said curcuminoid biosynthetic genes expressed in the bacterial host cell encode one or more selected from the group consisting of phenylalanine hydroxylase (PAH), phenylalanine ammonia lyase (PAL), tyrosine ammonia lyase (TAL), Cinnamate 4-hydroxylase (C4H), 4-coumarate 3-hydroxylase (C3H), Caffeic acid O-methlytransferase (COMT), Caffeoyl-CoA O-methyltransferase (CCoAOMT), 4-coumarate-CoA ligase (4CL), diketide-CoA synthase (DCS), curcumin synthase 1 (CURS1), curcumin synthase 2 (CURS2), and curcumin synthase 3 (CURS3).

More preferably, in the context of the present invention of the present invention, said curcuminoid biosynthetic genes expressed in the corynebacterium (e.g., *C. glutamicum)* host cell encode one or more selected from the group consisting of 4-coumarate 3-hydroxylase (C3H), Caffeic acid O-methlytransferase (COMT), Caffeoyl-CoA O-methyltransferase (CCoAOMT), 4-coumarate-CoA ligase (4CL), diketide-CoA synthase (DCS), curcuminoid synthase (CUS), curcumin synthase 1 (CURS1), curcumin synthase 2 (CURS2), and curcumin synthase 3 (CURS3).

Also, more preferably, in the context of the present invention of the present invention, said curcuminoid biosynthetic genes expressed in the bacterial host cell encode one or more selected from the group consisting of 4-coumarate 3-hydroxylase (C3H), Caffeic acid O-methlytransferase (COMT), Caffeoyl-CoA O-methyltransferase (CCoAOMT), 4-coumarate-CoA ligase (4CL), diketide-CoA synthase (DCS), curcumin synthase 1 (CURS1), curcumin synthase 2 (CURS2), and curcumin synthase 3 (CURS3).

Even more preferably in the context of the present invention of the present invention, said curcuminoid biosynthetic genes expressed in the corynebacterium (e.g., *C. glutamicum)* host cell encode one or more selected from the group consisting of 4-coumarate-CoA ligase (4CL), diketide-CoA synthase (DCS), curcuminoid synthase (CUS), curcumin synthase 1 (CURS1), curcumin synthase 2 (CURS2), and curcumin synthase 3 (CURS3),m preferably selected from the group consisting of 4-coumarate-CoA ligase (4CL), diketide-CoA synthase (DCS), curcuminoid synthase (CUS), and curcumin synthase 1 (CURS1), more preferably selected from the group consisting of 4-coumarate-CoA ligase (4CL), diketide-CoA synthase (DCS), and curcumin synthase 1 (CURS1).

Among the curcumin synthases 1, 2, and 3 (CURS1, CURS2, CURS3, respectively), in accordance with the present invention, curcumin synthase 1 (CURS1) is preferred for optimized curcumin production. That is, in context with the present invention, preferably the bacterial host cell as described and provided herein is genetically engineered to express curcumin synthase 1 (CURS1) (and optionally additionally DCS and 4CL as described herein).

Even more specifically preferred in the context of the present invention, said curcuminoid biosynthetic genes expressed in the bacterial host cell encode one or more selected from the group consisting of 4-coumarate-CoA ligase (4CL), diketide-CoA synthase (DCS), curcumin synthase 1 (CURS1), curcumin synthase 2 (CURS2), and curcumin synthase 3 (CURS3). For example, said curcuminoid biosynthetic genes expressed in the corynebacterium (e.g., C. *glutamicum)* host cell encode 4-coumarate-CoA ligase (4CL), diketide-CoA synthase (DCS), curcumin synthase 1 (CURS1), curcumin synthase 2 (CURS2), and curcumin synthase 3 (CURS3). As a more specific example, said curcuminoid biosynthetic genes expressed in the bacterial host cell (e.g., *C. glutamicum)* encode 4-coumarate-CoA ligase (4CL), diketide-CoA synthase (DCS), and curcumin synthase 1 (CURS1).

Preferably, in the context of the present invention, said curcuminoid biosynthetic genes expressed in the bacterial host cell encode one or more selected from the group consisting of 4-coumarate-CoA ligase (4CL), diketide-CoA synthase (DCS), and curcumin synthase 1 (CURS1). For example, said curcuminoid biosynthetic genes expressed in the corynebacterium (e.g., *C. glutamicum)* host cell encode 4-coumarate-CoA ligase (4CL), diketide-CoA synthase (DCS), and curcumin synthase 1 (CURS1).

Also, preferably in the context of the present invention, said curcuminoid biosynthetic genes expressed in the bacterial host cell encode one or more selected from the group consisting of 4-coumarate-CoA ligase (4CL), diketide-CoA synthase (DCS), and curcumin synthase 2 (CURS2). For example, said curcuminoid biosynthetic genes expressed in the bacterial host cell (e.g., *C. glutamicum)* encode 4-coumarate-CoA ligase (4CL), diketide-CoA synthase (DCS), and curcumin synthase 2 (CURS2).

Also, preferably in the context of the present invention, said curcuminoid biosynthetic genes expressed in the bacterial host cell encode one or more selected from the group consisting of 4-coumarate-CoA ligase (4CL), diketide-CoA synthase (DCS), and curcumin synthase 3 (CURS3). For example, said curcuminoid biosynthetic genes expressed in the bacterial host cell (e.g., *C. glutamicum)* encode 4-coumarate-CoA ligase (4CL), diketide-CoA synthase (DCS), and curcumin synthase 3 (CURS3).

Preferably in the context of the present invention, said curcuminoid biosynthetic genes expressed in the genetically engineered bacterial host cell described and provided in accordance with the present invention are codon optimized. Methods and tools for codon optimization are generally known in the art (see, e.g., Menzella, Microbial Cell Factories (2011), 10: 15; Rehbein et al., Prot expression and Purification (2019), 160: 84-93; Wang et al., LWT (2022), 165: 113744). Examples for codon-optimized genes are also provided herein in accordance with the present invention.

The curcuminoid biosynthesis pathway is known to the skilled person (cf. Beganovic and Wittmann, Curr Opinion Biotechnol (2024), 87: 103112). In accordance with the present invention, it has been found that enzymes of the curcuminoid biosynthesis pathway are particularly useful to be expressed in a genetically engineered bacterial host cell, preferably corynebacterium (e.g., *C. glutamicum)* host cell described and provided in accordance with the present invention for curcumin production. Such enzymes of the curcuminoid biosynthesis pathway include 4-coumarate-CoA ligase (4CL), diketide-CoA synthase (DCS), curcumin synthase 1 (CURS1), curcumin synthase 2 (CURS2), and curcumin synthase 3 (CURS3). Alternative to CURS 1, 2 and 3, curcuminoid synthase (CUS) may be employed in accordance with the present invention.

As shown and exemplified herein, curcuminoid synthase (CUS) from *Oryza sativa,* 4-coumarate-CoA ligase (4CL) from *Arabidopsis thaliana,* diketide-CoA synthase (DCS) from *Curcuma longa,* and curcumin synthase 1, 2, and 3 from *Curcuma longa* are particularly suitable for curcumin production and, thus, to be expressed in the genetically engineered bacterial host cell described and provided in accordance with the present invention.

Accordingly, in context with the present invention, preferably, the genetically engineered bacterial host cell expresses genes encoding curcuminoid synthase (CUS) from *Oryza sativa,* 4-coumarate-CoA ligase (4CL) from *Arabidopsis thaliana,* diketide-CoA synthase (DCS) from *Curcuma longa,* and/or curcumin synthase 1, 2, and/or 3 from *Curcuma longa.*

Preferably, in context with the present invention, the genetically engineered bacterial host cell expresses genes encoding 4-coumarate-CoA ligase (4CL) from *Arabidopsis thaliana,* diketide-CoA synthase (DCS) from *Curcuma longa,* and either curcuminoid synthase (CUS) from *Oryza sativa* or any one (or two or all) of curcumin synthase 1, 2, and/or 3 from *Curcuma longa.* In each case, for 4-coumarate-CoA ligase (4CL), diketide-CoA synthase (DCS), curcumin synthase 1 (CURS1), curcumin synthase 2 (CURS2), curcumin synthase 3 (CURS3), and curcuminoid synthase (CUS), the respective codon optimized version of the sequence is preferred. In particular, in accordance with the present invention, for 4-coumarate-CoA ligase (4CL) from *Arabidopsis thaliana,* diketide-CoA synthase (DCS) from *Curcuma longa,* curcumin synthase 1 (CURS1) from *Curcuma longa,* curcumin synthase 2 (CURS2) from *Curcuma longa,* curcumin synthase 3 (CURS3) from *Curcuma longa,* and curcuminoid synthase (CUS) from *Oryza sativa,* the respective codon optimized version of the sequence is preferred.

The polypeptide sequence for curcuminoid synthase (CUS) from *Oryza sativa* is shown in SEQ ID NO: 1 and 2.

The polypeptide sequence for diketide-CoA synthase (DCS) from *Curcuma longa* is shown in SEQ ID NO: 3 and 4.

The polypeptide sequence for curcumin synthase 1 (CURS1) from *Curcuma longa* is shown in SEQ ID NO: 5 and 6.

The polypeptide sequence for curcumin synthase 2 (CURS2) from *Curcuma longa* is shown in SEQ ID NO: 7.

The polypeptide sequence for curcumin synthase 3 (CURS3) from *Curcuma longa* is shown in SEQ ID NO: 8.

The polypeptide sequence for 4-coumarate-CoA ligase (4CL) from *Arabidopsis thaliana* is shown in SEQ ID NO: 9 and 10.

Preferably in the context of the present invention, the curcuminoid synthase (CUS) encoded by the curcuminoid biosynthetic gene expressed by the bacterial host cell has a polypeptide sequence at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99%, or 100% similar or (preferably) identical to the polypeptide sequence shown in SEQ ID: 1 or 2.

Preferably in the context of the present invention, the diketide-CoA synthase (DCS) encoded by the curcuminoid biosynthetic gene expressed by the bacterial host cell has a polypeptide sequence at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99%, or 100% similar or (preferably) identical to the polypeptide sequence shown in SEQ ID: 3 or 4.

Preferably in the context of the present invention, the curcumin synthase 1 (CURS1) encoded by the curcuminoid biosynthetic gene expressed by the bacterial host cell has a polypeptide sequence at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99%, or 100% similar or (preferably) identical to the polypeptide sequence shown in SEQ ID: 5 or 6.

Preferably in the context of the present invention, the curcumin synthase 2 (CURS2) encoded by the curcuminoid biosynthetic gene expressed by the bacterial host cell has a polypeptide sequence at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99%, or 100% similar or (preferably) identical to the polypeptide sequence shown in SEQ ID: 7.

Preferably in the context of the present invention, the curcumin synthase 3 (CURS3) encoded by the curcuminoid biosynthetic gene expressed by the bacterial host cell (e.g., *C. glutamicum)* has a polypeptide sequence at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99%, or 100% similar or (preferably) identical to the polypeptide sequence shown in SEQ ID: 8.

Preferably in the context of the present invention, the 4-coumarate-CoA ligase (4CL) encoded by the curcuminoid biosynthetic gene expressed by the bacterial host cell has a polypeptide sequence at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99%, or 100% similar or (preferably) identical to the polypeptide sequence shown in SEQ ID: 9 or 10.

Preferably in the context of the present invention, said curcuminoid biosynthetic genes expressed in the bacterial host cell encode diketide-CoA synthase (DCS) (preferably from *Curcuma longa)* and curcumin synthase 1 (CURS1) (preferably from *Curcuma longa).*

Preferably in the context of the present invention, said curcuminoid biosynthetic genes expressed in the bacterial host cell encode diketide-CoA synthase (DCS) (preferably from *Curcuma longa),* curcumin synthase 1 (CURS1) (preferably from *Curcuma longa)* and 4-coumarate-CoA ligase (4CL) (preferably from *Arabidopsis thaliana*)*.*

Preferably in the context of the present invention, said curcuminoid biosynthetic genes expressed in the bacterial host cell encode a polypeptide sequence at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99%, or 100% similar or (preferably) identical to the polypeptide sequence shown in SEQ ID: 3 or 4 (preferably 4), and a polypeptide sequence at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99%, or 100% similar or (preferably) identical to the polypeptide sequence shown in SEQ ID: 5 or 6 (preferably 6).

Preferably in the context of the present invention, said curcuminoid biosynthetic genes expressed in the bacterial host cell encode a polypeptide sequence at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99%, or 100% similar or (preferably) identical to the polypeptide sequence shown in SEQ ID: 3 or 4 (preferably 4), a polypeptide sequence at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99%, or 100% similar or (preferably) identical to the polypeptide sequence shown in SEQ ID: 5 or 6 (preferably 6), and a polypeptide sequence at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99%, or 100% similar or (preferably) identical to the polypeptide sequence shown in SEQ ID: 9 or 10 (preferably 10).

In context with the present invention, the curcuminoid biosynthetic genes expressed in the bacterial host cell as described and provided herein may include any gene encoding an enzyme involved in the curcuminoid biosynthesis pathway (cf. Beganovic and Wittmann, Curr Opinion Biotechnol (2024), 87: 103112). For example, in accordance with the present invention, such gene may encode one or more selected from the group consisting of 4-coumarate-CoA ligase (4CL), diketide-CoA synthase (DCS), curcumin synthase 1 (CURS1), curcumin synthase 2 (CURS2), and curcumin synthase 3 (CURS3). For example, said curcuminoid biosynthetic genes expressed in the bacterial host cell encode 4-coumarate-CoA ligase (4CL), diketide-CoA synthase (DCS), curcumin synthase 1 (CURS1), curcumin synthase 2 (CURS2), and curcumin synthase 3 (CURS3).

Preferably in the context of the present invention, the gene encoding 4-coumarate-CoA ligase (4CL) is from *Arabidopsis thaliana,* diketide-CoA synthase (DCS) is from *Curcuma longa,* curcumin synthase 1 (CURS1) is from *Curcuma longa,* curcumin synthase 2 (CURS2) is from *Curcuma longa,* and curcumin synthase 3 (CURS3) is from *Curcuma longa.* In each case, a codon optimized version of the nucleotide sequences encoding 4-coumarate-CoA ligase (4CL) (preferably from *Arabidopsis thaliana*)*,* diketide-CoA synthase (DCS) (preferably from *Curcuma longa),* curcumin synthase 1 (CURS1) (preferably from *Curcuma longa),* curcumin synthase 2 (CURS2) (preferably from *Curcuma longa),* and curcumin synthase 3 (CURS3) (preferably from *Curcuma longa),* respectively, is preferred.

The native nucleotide sequence for curcuminoid synthase (CUS) from *Oryza sativa* is shown in SEQ ID NO: 11. The respective codon optimized nucleotide sequence is shown in SEQ ID NO: 12.

The native nucleotide sequence for diketide-CoA synthase (DCS) from *Curcuma longa* is shown in SEQ ID NO: 13. The respective codon optimized nucleotide sequence is shown in SEQ ID NO: 14.

The native nucleotide sequence for curcumin synthase 1 (CURS1) from *Curcuma longa* is shown in SEQ ID NO: 15. The respective codon optimized nucleotide sequence is shown in SEQ ID NO: 16.

The native nucleotide sequence for curcumin synthase 2 (CURS2) from *Curcuma longa* is shown in SEQ ID NO: 17.

The native nucleotide sequence for curcumin synthase 3 (CURS3) from *Curcuma longa* is shown in SEQ ID NO: 18.

The native nucleotide sequence for 4-coumarate-CoA ligase (4CL) from *Arabidopsis thaliana* is shown in SEQ ID NO: 19. The respective codon optimized nucleotide sequence is shown in SEQ ID NO: 20.

Accordingly in the context of the present invention, the gene encoding curcuminoid synthase (CUS) has a nucleotide sequence at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99%, or 100% identical to the nucleotide sequence shown in SEQ ID: 11 or 12 (preferably 12). If deviating from the respective reference nucleotide sequence, silent mutations are preferred.

Accordingly, in the context of the present invention, the gene encoding diketide-CoA synthase (DCS) has a nucleotide sequence at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99%, or 100% identical to the nucleotide sequence shown in SEQ ID: 13 or 14 (preferably 14). If deviating from the respective reference nucleotide sequence, silent mutations are preferred.

Accordingly, in the context of the present invention, the gene encoding curcumin synthase 1 (CURS1) has a nucleotide sequence at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99%, or 100% identical to the nucleotide sequence shown in SEQ ID: 15 or 16 (preferably 16). If deviating from the respective reference nucleotide sequence, silent mutations are preferred.

Accordingly, in the context of the present invention, the gene encoding curcumin synthase 2 (CURS2) has a nucleotide sequence at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99%, or 100% identical to the nucleotide sequence shown in SEQ ID: 17. If deviating from the respective reference nucleotide sequence, silent mutations are preferred.

Accordingly, in the context of the present invention, the gene encoding curcumin synthase 3 (CURS3) has a nucleotide sequence at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99%, or 100% identical to the nucleotide sequence shown in SEQ ID: 18. If deviating from the respective reference nucleotide sequence, silent mutations are preferred.

Accordingly in the context of the present invention, the gene encoding 4-coumarate-CoA ligase (4CL) has a nucleotide sequence at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99%, or 100% identical to the nucleotide sequence shown in SEQ ID: 19 or 20 (preferably 20). If deviating from the respective reference nucleotide sequence, silent mutations are preferred.

In context with the present invention, preferred combinations of genes to be expressed in the host cell described and provided herein include genes encoding diketide-CoA synthase (DCS) and curcumin synthase 1 (CURS1) as described herein, or diketide-CoA synthase (DCS), curcumin synthase 1 (CURS1) and 4-coumarate-CoA ligase (4CL) as described herein.

In accordance with the present invention, enzymes named herein - including those enzymes which do not have 100% sequence identity with a corresponding native polypeptide sequence - preferably exhibit the same functions as the respective native polypeptides. Such functions of the respective enzymes are kwon in the art (cf., e.g., Beganovic and Wittmann, Curr Opinion Biotechnol (2024), 87: 103112). For example,
- PAL is able to convert L-phenylalanine to cinnamate,
- TAL is able to convert L-tyrosine to *p*-coumarate,
- C4H is able to convert cinnamate to *p*-coumarate,
- C3H is able to convert *p*-coumarate to caffeate,
- COMT is able to convert caffeate to ferulate,
- CCoAOMT is able to convert caffoyl-CoA to feruloyl-CoA,
- 4CL is able to convert
   ∘ *p*-coumarate to *p*-coumaroyl-CoA,
   ∘ caffeate to caffeoyl-CoA,
   ∘ ferulate to feruloyl-CoA,
- DCS is able to convert
   ∘ *p*-coumaroyl-CoA to *p*-coumaroyl-diketide-CoA (with malonyl-CoA),
   ∘ feruloyl-CoA to feruloyl-diketide-CoA (with malonyl-CoA)
- CURS 1, 2 and 3 are each able to convert
   ∘ feruloyl-diketide-CoA to β-keto acid,
   ∘ β-keto acid to curcumin (with feruloyl-CoA),
   ∘ β-keto acid to demethoxycurcumin (with feruloyl-CoA),
- CURS3 is (in addition to the above) able to convert
   ∘ *p*-coumaroyl-diketide-CoA to β-keto acid,
   ∘ β-keto acid to demethoxycurcumin (with *p*-coumaroyl-CoA),
   ∘ *p*-coumaroyl-CoA to bis demethoxycurcumin (with *p*-coumaroyl-CoA).

In accordance with the present invention, as used herein in context with polypeptide sequences (used herein synonymously with the term "amino acid sequences"), the term "similar" means that a given amino acid sequence comprises identical amino acids or only conservative or highly conservative substitutions compared to the amino acid sequence of the respective SEQ ID NO. As used herein, "conservative" substitutions mean substitutions as listed as "Exemplary Substitutions" in Table I herein. "Highly conservative" substitutions as used herein mean substitutions as shown under the heading "Preferred Substitutions" in Table I herein.

**TABLE I Amino Acid Substitutions**

| Original | Exemplary Substitutions | Preferred Substitutions |
|---|---|---|
| Ala (A) | val; leu; ile | Val |
| Arg (R) | lys; gln; asn | lys |
| Asn (N) | gln; his; asp, lys; arg | gln |
| Asp (D) | glu; asn | glu |
| Cys (C) | ser; ala | ser |
| Gln (Q) | asn; glu | asn |
| Glu (E) | asp; gln | asp |
| Gly (G) | ala | ala |
| His (H) | asn; gln; lys; arg | arg |
| Ile (I) | leu; val; met; ala; phe; | leu |
| Leu (L) | norleucine; ile; val; met; ala; | ile |
| Lys (K) | arg; gin; asn | arg |
| Met (M) | leu; phe; ile | leu |
| Phe (F) | leu; val; ile; ala; tyr | tyr |
| Pro (P) | ala | ala |
| Ser (S) | thr | thr |
| Thr (T) | ser | ser |
| Trp (W) | tyr; phe | tyr |
| Tyr (Y) | trp; phe; thr; ser | Phe |
| Val (V) | ile; leu; met; phe; ala; | leu |

As used herein, "silent" mutations or substitutions mean base substitutions within a nucleic acid sequence which do not change the amino acid sequence encoded by the nucleic acid sequence. "Conservative" mutations or substitutions mean substitutions vis-à-vis the encoded polypeptide as listed as "Exemplary Substitutions" in Table I. "Highly conservative" substitutions as used herein mean substitutions vis-à-vis the encoded polypeptide as shown under the heading "Preferred Substitutions" in Table I.

The term "position" when used in accordance with the present invention means the position of an amino acid within an amino acid sequence depicted herein. The term "corresponding" in this context also includes that a position is not only determined by the number of the preceding nucleotides/amino acids.

The level of identity between two or more sequences (e.g., nucleic acid sequences or amino acid sequences) can be easily determined by methods known in the art, e.g., by BLAST analysis. Generally, in context with the present invention, if two sequences (e.g., polynucleotide sequences or amino acid sequences) to be compared by, e.g., sequence comparisons differ in identity, then the term "identity" may refer to the shorter sequence and that part of the longer sequence that matches said shorter sequence. Therefore, when the sequences which are compared do not have the same length, the degree of identity may preferably either refer to the percentage of nucleotide residues in the shorter sequence which are identical to nucleotide residues in the longer sequence or to the percentage of nucleotides in the longer sequence which are identical to nucleotide sequence in the shorter sequence. In this context, the skilled person is readily in the position to determine that part of a longer sequence that matches the shorter sequence. Furthermore, as used herein, identity levels of nucleic acid sequences or amino acid sequences may refer to the entire length of the respective sequence and is preferably assessed pair-wise, wherein each gap is to be counted as one mismatch. These definitions for sequence comparisons (e.g., establishment of "identity" values) are to be applied for all sequences described and disclosed herein.

Moreover, the term "identity" as used herein means that there is a functional and/or structural equivalence between the corresponding sequences. Nucleic acid/amino acid sequences having the given identity levels to the herein-described particular nucleic acid/amino acid sequences may represent derivatives/variants of these sequences which, preferably, have the same biological function. They may be either naturally occurring variations, for instance sequences from other varieties, species, etc., or mutations, and said mutations may have formed naturally or may have been produced by deliberate mutagenesis. Furthermore, the variations may be synthetically produced sequences. The variants may be naturally occurring variants or synthetically produced variants or variants produced by recombinant DNA techniques.

"Deviations" from sequences (e.g., amino acid or nucleic acid sequences) as used herein may comprise, e.g., deletions, substitutions, additions, insertion and/or recombination. The term "addition" refers to adding a nucleic acid residue/amino acid to the end or beginning of the given sequence, whereas "insertion" refers to inserting a nucleic acid residue/amino acid within a given sequence. The term "deletion" refers to deleting or removal of a nucleic acid residue or amino acid residue in a given sequence. The term "substitution" refers to the replacement of a nucleic acid residue/amino acid residue in a given sequence. Again, these definitions as used here apply, *mutatis mutandis,* for all sequences provided and described herein unless specified otherwise.

As used herein, unless specifically defined otherwise, the term "nucleic acid" or "nucleic acid molecule" is used synonymously with "oligonucleotide", "nucleic acid strand", or the like, and means a polymer comprising one, two, or more nucleotides, arranged in single- or double stranded nucleotide chains.

Generally, as used herein, the terms "polynucleotide", "nucleic acid" and "nucleic acid molecule" are to be construed synonymously. Generally, nucleic acid molecules may comprise *inter alia* DNA molecules, RNA molecules, oligonucleotide thiophosphates, substituted ribo-oligonucleotides or PNA molecules. Furthermore, the term "nucleic acid molecule" may refer to DNA or RNA or hybrids thereof or any modification thereof that is known in the art (see, e.g., US 5525711, US 471 1955, US 5792608 or EP 302175 for examples of modifications). The nucleic acid molecule may be single- or double- stranded, linear or circular, natural or synthetic, and without any size limitation. For instance, the nucleic acid molecule may be genomic DNA, cDNA, mitochondrial DNA, mRNA, antisense RNA, ribozymal RNA or a DNA encoding such RNAs or chimeroplasts (Gamper, Nucleic Acids Research, 2000, 28, 4332 - 4339). Said nucleic acid molecule may be in the form of a vector, plasmid, cosmid, or of viral DNA or RNA. Also described herein are nucleic acid molecules which are complementary to the nucleic acid molecules described above and nucleic acid molecules which are able to hybridize to nucleic acid molecules described herein. A nucleic acid molecule described herein may also be a fragment of the nucleic acid molecules in context of the present invention. Particularly, such a fragment is a functional fragment. Examples for such functional fragments are nucleic acid molecules which can serve as primers.

In accordance with the present invention, the curcuminoid biosynthetic genes to be expressed in the genetically engineered bacterial host cell, particularly a corynebacterium (e.g., C. *glutamicum)* host cell described and provided herein may be introduced into the host cell with any suitable means known in the art. Such genes (polynucleotides) may be brought into the cell via technical or biological means, e.g., via transduction or transformation, or any other means that is suitable to introduce a polynucleotide into a cell, allowing (preferably stable) expression of that polynucleotide in said cell. Such methods are well known in the art. In accordance with the present invention, such polynucleotide may be introduced into the cell alone, i.e. solely the desired polynucleotide, or together with other polynucleotides (e.g., other genes, ORFs, promoter-, regulator- or enhancer-sequences, or other functional or non-functional nucleotides), either within a single molecule (e.g., a plasmid, cosmid, vector, or other forms) or on separate molecules, preferably a plasmid, such as low, medium or high copy number plasmids, e.g. pClik, pCES, PK18, PK19, pVWEx1 oder pECXT99A.

Furthermore, the bacterial host cell which is genetically engineered to express curcuminoid biosynthetic genes as described and provided herein is preferably further genetically engineered to be reduced, preferably inactivated in catabolizing phenylpropanoids into further metabolic products for energy production of said bacterial host cell.

As described herein, it is a prerequisite that a gram-positive bacterial host cell has naturally a phenylpropanoid pathway, since phenylpropanoids, in particular cinnamic acid, p-coumaric acid, caffeic acid and/or ferulic acid are the source for precursors of curcuminoids, particularly curcumin. Hence, ideally a bacterial host cell of the present invention uses phenylpropanoids for the production of curcuminoids, particularly for curcumin, but not for other purposes, e.g. energy production. Accordingly, a bacterial host cell of the present invention is genetically engineered to be reduced, preferably inactivated in catabolizing phenylpropanoids into further metabolic products for energy production.

By "reduced" or "reduction" it is meant that a bacterial host cell of the present invention predominantly, i.e., in more than half of all cases, preferably in 60%, 70%, 80%, 90% or all of all cases uses phenylpropanoids for the production of curcuminoids, particularly for curcumin. If in all of the cases a bacterial host cell of the present invention uses phenylpropanoids for the production of curcuminoids, such a bacterial host cell of the present invention is genetically engineered to be inactivated in catabolizing phenylpropanoids into further metabolic products for energy production

Preferably, for reducing a bacterial host cell of the present invention in catabolizing phenylpropanoids into further metabolic products for energy production, in said bacterial host cell *phdBCDE* genes encoding 3-hydroxyacyl-CoA dehydrogenase, metal-dependent hydrolase, acyl-CoA dehydrogenase, and/or enoyl-CoA hydratase, respectively, are reduced in their expression, preferably inactivated. In this context, preferably all *phdBCDE* genes encoding 3-hydroxyacyl-CoA dehydrogenase, metal-dependent hydrolase, acyl-CoA dehydrogenase, and enoyl-CoA hydratase, respectively, are reduced in their expression, preferably inactivated.

SEQ ID NO: 21 is the preferred nucleotide sequence encoding PhdB.

SEQ ID NO: 22 is the preferred nucleotide sequence encoding PhdC.

SEQ ID NO: 23 is the preferred nucleotide sequence encoding PhdD.

SEQ ID NO: 24 is the preferred nucleotide sequence encoding PhdE.

The skilled person is able on the basis of the nucleotide sequence of *phdB* as shown in SEQ ID NO: 21, *phdC* as shown in SEQ ID NO: 22, *phdD* as shown in SEQ ID NO: 23, or *phdE* as shown in SEQ ID NO: 24, respectively, to identify the corresponding gene in another gram-positive bacterial host cell as referred to herein.

"Inactivated" or "inactivation" as used herein in accordance with the present invention means that expression of such genes is inhibited or blocked, or the respective nucleotide sequences of such genes are mutated in a way that the respective genes cannot be transcribed or translated properly (i.e. to result in a functional enzyme exhibiting the enzymatic capabilities of the native sequence), or the respective genes are deleted partially or in total. Corresponding methods to inactivate genes are generally known in the art and comprise, for example, spot mutations (e.g., to change the reading frame, to introduce stop codons, to change one or more codon triplets to result in a non-functional polypeptide that does not exhibit the enzymatic capabilities of the native enzyme,), insertions or deletions to interrupt the open reading frame, silencing methods (e.g., RNAi like siRNA, miRNA), and others.

As has further been found in context with the present invention, deregulation of ferulate uptake in a bacterial host cell as described and provided herein can significantly boost the production of curcumin. Ferulate serves as a prime building block of the curcumin molecule. However, as has been found in context with the present invention, high concentrations of ferulate can impair growth of the corynebacterium host cells. Surprisingly, upon elimination (inactivation) of the PhdR-mediated repression of the ferulate catabolism, curcumin production could be increased. PhdR is a MarR-type repressor known in the art (Kallscheuer et al., Metab Eng (2016), 38: 47-55). Accordingly, in one embodiment of the present invention, the bacterial host cell as described and provided herein is further genetically engineered to be incapable of expressing PhdR. In other words, in accordance with the present invention, in one embodiment, in the corynebacterium host cell as described and provided herein, the gene encoding PhdR (*phdR*) is inactivated (preferably deleted). SEQ ID NO: 25 is the preferred nucleotide sequence encoding PhdR.

In accordance with the present invention, the inactivation of *phdR* can be alone or in addition with the inactivation of any (or, preferably, all) *phdBCDE* genes. In a preferred embodiment, both *phdR* and all *phdBCDE* genes are inactivated (preferably deleted or silenced, more preferably deleted) in the host cell described and provided herein.

The skilled person is able on the basis of either the nucleotide sequence of *phdR* as shown in SEQ ID NO: 25 to identify the corresponding gene in another gram-positive bacterial host cell as referred to herein.

As has also been found in context with the present invention, it is beneficial for curcumin production if *phdT* and *phdA* genes are left intact to allow proper expression of PhdT and PhdA, respectively. The *phdA* gene encodes long-chain fatty acid-CoA ligase. SEQ ID NO: 26 is the preferred nucleotide sequence encoding PhdA.

The skilled person is able on the basis of either the nucleotide sequence of *phdA* as shown in SEQ ID NO: 26 to identify the corresponding gene in another gram-positive bacterial host cell as referred to herein.

Accordingly, it is preferred that in the bacterial host cell as described and provided herein, the gene encoding PhdT and/or PhdA is not inactivated, preferably neither the gene encoding PhdT nor PhdA is inactivated in the bacterial host cell described and provided herein. Also, in context with the present invention, it is preferred that *phdT* and *phdA* are under native promoter control.

More preferably, the *phdA* gene is over-expressed, i.e., its expression is increased in a bacterial host cell of the present invention. "Increased" expression means that a gene is higher expressed than it is naturally expressed through its native promoter. Such increased expression may be achieved by having the gene under the control of a promoter which is stronger than the native promoter. In case of *phdA* as described herein the tuf promoter may be used for the over-expression of *phdA.*

"Increased" expression may also be achieved by genetically engineering a bacterial host cell of the present invention by introducing one or more further copies of the gene whose expression should be increased. In case of *phdA,* a bacterial host cell of the present invention may be genetically engineered to be equipped with a one or more copies of the *phdA* gene. Such additional copies of the *phdA* gene may be under the control of the native promoter, a homologous promoter which is stronger than the native promoter or a heterologous promoter which is stronger than the native promoter. A preferred promoter for the expression of such an additional copy of the *phdA* gene is the tuf promoter.

Accordingly, it is preferred that a bacterial host cell of the present invention may be genetically engineered to have increased expression of the phdA gene encoding long-chain fatty acid-CoA ligase. SEQ ID NO: 26 is the preferred nucleotide sequence encoding PhdA

The present inventors have observed that phenylpropanoids may be degraded by a bacterial host cell of the present invention via the benzoate degradation pathway. Hence, in context with the present invention, it may be advantageous that a bacterial host cell as described and provided herein is genetically engineered to inactivate *benABCD* genes encoding benzoate 1,2-dioxygenase large subunit, benzoate 1,2-dioxygenase small subunit, benzoate 1,2-dioxygenase electron transfer component, 1,6-dihydroxycyclohexa-2,4-diene-1-carboxylate dehydrogenase, respectively.

SEQ ID NO: 27 is the preferred nucleotide sequence encoding BenA.

SEQ ID NO: 28 is the preferred nucleotide sequence encoding BenB.

SEQ ID NO: 29 is the preferred nucleotide sequence encoding BenC.

SEQ ID NO: 30 is the preferred nucleotide sequence encoding BenD.

The skilled person is able on the basis of either the nucleotide sequence of *benA* as shown in SEQ ID NO: 27, *benB* as shown in SEQ ID NO: 28, *benC* as shown in SEQ ID NO: 29, or *benD* as shown in SEQ ID NO: 30, respectively, to identify the corresponding gene in another gram-positive bacterial host cell as referred to herein.

As has further been found in context with the present invention, attenuation of the expression of the gene encoding citrate synthase can increase the formation of curcumin. Such attenuation can be achieved by means known in the art, e.g. by equipping the citrate synthase gene with a promoter which is weaker than its native promoter or by modifying the start codon of the citrate synthase gene. The latter, i.e., modification of the start codon of the citrate synthase *gltA* genes is preferred. A citrate synthase gene with a modified start codon is attenuated in its expression. Attenuated expression of citrate synthase results in increased availability of acetyl-CoA and formation of curcumin in a bacterial host cell as described and provided herein.

Accordingly, the bacterial host cell of the present invention s genetically engineered to have attenuated expression of the gene encoding citrate synthase.

A preferred way for attenuating the expression of the gene encoding citrate synthase is to genetically engineer a bacterial host cell of the present invention to have GTG as start codon (instead of ATG) of the gene encoding citrate synthase (*gltA*). SEQ ID NO: 31 is the preferred nucleotide sequence encoding GltA (citrate synthase).

The skilled person is able on the basis of either the nucleotide sequence of *gltA* as shown in SEQ ID NO: 31 to identify the corresponding gene in another gram-positive bacterial host cell as referred to herein.

Further findings that have been made in context with the present invention relate to the cultivation of the bacterial host cells (e.g., *C. glutamicum)* as described and provided herein. As mentioned, ferulate serves as a prime building block of the curcumin molecule, and as has further been found in context with the present invention, high concentrations of ferulate can impair growth of the corynebacterium host cells. While growth of the host cells was still possible at high ferulate concentrations of 30 mM, it has been shown that curcumin production was increased at lower ferulate concentrations.

Accordingly, bacterial the host cells genetically engineered to express curcuminoid biosynthetic genes as described and provided herein are preferably cultivated in the presence of ferulate. Preferably, the bacterial host cells are cultivated in the presence of up to about 10 mM ferulate, preferably up to about 8 mM ferulate, more preferably up to about 7 mM ferulate, more preferably up to about 6 mM ferulate, most preferably up to about 5 mM ferulate.

Similarly, it has been found in context with the present invention that also the presence and concentration of glucose has an impact on the curcumin production yields. While curcumin production is possible with different glucose concentrations, it has been surprisingly found that the bacterial host cells genetically engineered to express curcuminoid biosynthetic genes as described and provided herein produced higher amounts of curcumin when cultivated up to about 30 g/l glucose in the medium.

Accordingly, the bacterial host cells genetically engineered to express curcuminoid biosynthetic genes as described and provided herein are preferably cultivated in the presence of glucose. Preferably, the bacterial host cells are cultivated in the presence of about 5 to about 45 g/l glucose, preferably about 10 to about 40 g/l glucose, more preferably about 15 to about 35 g/l glucose, more preferably about 20 to about 30 g/l glucose, most preferably about 30 g/l glucose.

It is also preferred that the bacterial host cells of the present invention are cultivated in the presence of about 5 mM ferulate and about 30 g/l glucose.

As shown and exemplified herein, very good curcumin production results could be achieved using a fed-batch fermentation.

Accordingly, it is preferred that bacterial host cells genetically engineered to express curcuminoid biosynthetic genes as described and provided herein are cultivated using fed-batch fermentation. In this context, preferably, the starting concentration of ferulate is about 5 mM and the starting concentration of glucose is about 30 g/l glucose. Furthermore, in accordance with the present invention, subsequent ferulate pulses may again be about 5 mM ferulate, and subsequent glucose pulses may again be about 30 g/l glucose.

As further found out in context with the present invention, specific O₂ and pH conditions can lead to even improved curcumin production yields when using the host cell described and provided herein. As has been found, pH levels between about 6 and 7 resulted in higher yields, and O₂ level of about 15-20%.

Accordingly, bacterial host cells genetically engineered to express curcuminoid biosynthetic genes as described and provided herein are cultivated at a pH of about 6 to 7, preferably about 6.2-6.9, more preferably 6.4-6.8, more preferably 6.6-6.8, most preferably about 6.8.

Furthermore, the bacterial host cells genetically engineered to express curcuminoid biosynthetic genes as described and provided herein are preferably cultivated at an O₂ concentration of about 10-22%, preferably about 15-20%, more preferably about 18-20%, most preferably about 20%.

The above being said, it is preferred that bacterial host cells genetically engineered to express curcuminoid biosynthetic genes as described and provided herein are cultivated at a pH of about 6.8 and an O₂ concentration of about 20%.

In accordance with the present invention, applying a fed-batch process for cultivation of the bacterial host cells described and provided herein together with the preferred O₂ conditions and pH levels as described herein may be particularly beneficial. This also applies for the method for producing curcumin as well as for the use of the host cell genetically engineered to express curcuminoid biosynthetic genes as described and provided herein for the production of curcumin as described and provided herein.

The present invention further relates to a method for producing curcumin, comprising culturing the bacterial host cell genetically engineered to express curcuminoid biosynthetic genes as described and provided herein under suitable conditions in liquid medium and harvesting curcumin.

Analogously to the description above, in a preferred embodiment of the method for producing curcumin as described hand provided herein, the bacterial host cells genetically engineered to express curcuminoid biosynthetic genes as described and provided herein are cultivated in the presence of ferulate. Preferably, the host cells are cultivated in the presence of up to about 10 mM ferulate, preferably up to about 8 mM ferulate, more preferably up to about 7 mM ferulate, more preferably up to about 6 mM ferulate, most preferably up to about 5 mM ferulate.

Furthermore, analogously to the description above, in a preferred embodiment of the method for producing curcumin as described hand provided herein, the bacterial host cells genetically engineered to express curcuminoid biosynthetic genes as described and provided herein are cultivated in the presence of glucose. Preferably, the host cells are cultivated in the presence of about 5 to about 45 g/l glucose, preferably about 10 to about 40 g/l glucose, more preferably about 15 to about 35 g/l glucose, more preferably about 20 to about 30 g/l glucose, most preferably about 30 g/l glucose.

The above being said, it is preferred that, in context with the method for producing curcumin, bacterial host cells are cultivated in the presence of about 5 mM ferulate and about 30 g/l glucose.

Analogously, preferably in context with the method for producing curcumin, the bacterial host cells genetically engineered to express curcuminoid biosynthetic genes as described and provided herein are cultivated using fed-batch fermentation. In this context, preferably, the starting concentration of ferulate is about 5 mM and the starting concentration of glucose is about 30 g/l glucose. Furthermore, in accordance with the present invention, subsequent ferulate pulses may again be about 5 mM ferulate, and subsequent glucose pulses may again be about 30 g/l glucose.

Furthermore, analogously to the description above, in a preferred embodiment of the present invention and in context with the method described and provided herein, the host cells genetically engineered to express curcuminoid biosynthetic genes as described and provided herein are cultivated at a pH of about 6 to 7, preferably about 6.2-6.9, more preferably 6.4-6.8, more preferably 6.6-6.8, most preferably about 6.8.

In another preferred embodiment of method described and provided herein, analogously to the description above and in context with the method described and provided herein, the host cells genetically engineered to express curcuminoid biosynthetic genes as described and provided herein are cultivated at an O₂ concentration of about 10-22%, preferably about 15-20%, more preferably about 18-20%, most preferably about 20%.

Preferably, the bacterial host cells genetically engineered to express curcuminoid biosynthetic genes as described and provided herein are cultivated at a pH of about 6.8 and an O₂ concentration of about 20%.

In another preferred embodiment of method described and provided herein, culturing comprises a two-phase fermentation. For the purpose of the invention, the term "two phase fermentation medium" or "biphasic fermentation medium" is meant to include a two-phase medium comprising a fermentation medium with the aqueous medium forming an aqueous phase and a second phase which can be liquid, solid, or a combination of both. For example, two phase systems are typically classified into liquid-liquid and liquid-solid systems. For example, the second phase may be a water-immiscible organic solvent forming an organic phase.

The present invention further relates to the use of the bacterial host cell genetically engineered to express curcuminoid biosynthetic genes as described and provided herein for the production of curcumin, preferably under conditions as described herein, particularly in context with ferulate concentration, glucose concentration, O₂ concentration, pH and cultivation method (e.g., fed-batch) as described herein.

It must be noted that as used herein, the singular forms "a", "an", and "the", include plural references unless the context clearly indicates otherwise. Thus, for example, reference to "a reagent" includes one or more of such different reagents and reference to "the method" includes reference to equivalent steps and methods known to those of ordinary skill in the art that could be modified or substituted for the methods described herein.

Unless otherwise indicated, the term "at least" preceding a series of elements is to be understood to refer to every element in the series. Those skilled in the art will recognize, or be able to ascertain using no more than routine experimentation, many equivalents to the specific embodiments of the invention described herein. Such equivalents are intended to be encompassed by the present invention.

The term "and/or" wherever used herein includes the meaning of "and", "or" and "all or any other combination of the elements connected by said term".

The term "about" or "approximately" as used herein means within 20%, preferably within 10%, and more preferably within 5% or 2% of a given value or range, and also comprise the respective exact numeric value.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having".

When used herein "consisting of" excludes any element, step, or ingredient not specified in the claim element. When used herein, "consisting essentially of" does not exclude materials or steps that do not materially affect the basic and novel characteristics of the claim.

In each instance herein any of the terms "comprising", "consisting essentially of" and "consisting of" may be replaced with either of the other two terms.

When used herein, the term "essentially", e.g., "essentially free of" or "essentially non-[...]" or the like, the term also includes the absolute condition, i.e. "free of", "non-[...]" or the like, respectively.

It should be understood that this invention is not limited to the particular methodology, protocols, and reagents, etc., described herein and as such can vary. The terminology used herein is for the purpose of describing particular embodiments only, and is not intended to limit the scope of the present invention, which is defined solely by the claims.

All publications and patents cited throughout the text of this specification (including all patents, patent applications, scientific publications, manufacturer's specifications, instructions, etc.), whether supra or infra, are hereby incorporated by reference in their entirety. Nothing herein is to be construed as an admission that the invention is not entitled to antedate such disclosure by virtue of prior invention. To the extent the material incorporated by reference contradicts or is inconsistent with this specification, the specification will supersede any such material.

The following sequences are referred to in this application:
SEQ ID NO: 1
   curcuminoid synthase (CUS)
   Amino acid
   *Oryza sativa* 
MAPTTTMGSALYPLGEMRRSQRADGLAAVLAIGTANPPNCVTQEEFPDFYFRVTNSDHLT
ALKDKFKRICQEMGVQRRYLHHTEEMLSAHPEFVDRDAPSLDARLDIAADAVPELAAEAA
KKAIAEWGRPAADITHLVVTTNSGAHVPGVDFRLVPLLGLRPSVRRTMLHLNGCFAGCAA
LRLAKDLAENSRGARVLVVAAELTLMYFTGPDEGCFRTLLVQGLFGDGAAAVIVGADADD
VERPLFEIVSAAQTIIPESDHALNMRFTERRLDGVLGRQVPGLIGDNVERCLLDMFGPLL
GGDGGGGWNDLFWAVHPGSSTIMDQVDAALGLEPGKLAASRRVLSDYGNMSGATVIFALD
ELRRQRKEAAAAGEWPELGVMMAFGPGMTVDAMLLHATSHVN*
SEQ ID NO: 2
   curcuminoid synthase (CUS) ("codon-optimized")
   Amino acid
   *Oryza sativa* 
MAPTTTMGSALYPLGEMRRSQRADGLAAVLAIGTANPPNCVTQEEFPDFYFRVTNSDHLT
ALKDKFKRICQEMGVQRRYLHHTEEMLSAHPEFVDRDAPSLDARLDIAADAVPELAAEAA
KKAIAEWGRPAADITHLVVTTNSGAHVPGVDFRLVPLLGLRPSVRRTMLHLNGCFAGCAA
LRLAKDLAENSRGARVLVVAAELTLMYFTGPDEGCFRTLLVQGLFGDGAAAVIVGADADD
VERPLFEIVSAAQTIIPESDHALNMRFTERRLDGVLGRQVPGLIGDNVERCLLDMFGPLL
GGDGGGGWNDLFWAVHPGSSTIMDQVDAALGLEPGKLAASRRVLSDYGNMSGATVIFALD
ELRRQRKEAAAAGEWPELGVMMAFGPGMTVDAMLLHATSHVN*
SEQ ID NO: 3
   diketide-CoA synthase (DCS)
   Amino acid
   *Curcuma longa* 
MEANGYRITHSADGPATILAIGTANPTNVVDQNAYPDFYFRVTNSEYLQELKAKFRRICE
KAAIRKRHLYLTEEILRENPSLLAPMAPSFDARQAIVVEAVPKLAKEAAEKAIKEWGRPK
SDITHLVFCSASGIDMPGSDLQLLKLLGLPPSVNRVMLYNVGCHAGGTALRVAKDLAENN
RGARVLAVCSEVTVLSYRGPHPAHIESLFVQALFGDGAAALVVGSDPVDGVERPIFEIAS
ASQVMLPESAEAVGGHLREIGLTFHLKSQLPSIIASNIEQSLTTACSPLGLSDWNQLFWA
VHPGGRAILDQVEARLGLEKDRLAATRHVLSEYGNMQSATVLFILDEMRNRSAAEGHATT
GEGLDWGVLLGFGPGLSIETVVLHSCRLN*
SEQ ID NO: 4
   diketide-CoA synthase (DCS) ("codon-optimized")
   Amino acid
   *Curcuma longa* 
MEANGYRITHSADGPATILAIGTANPTNVVDQNAYPDFYFRVTNSEYLQELKAKFRRICE
KAAIRKRHLYLTEEILRENPSLLAPMAPSFDARQAIVVEAVPKLAKEAAEKAIKEWGRPK
SDITHLVFCSASGIDMPGSDLQLLKLLGLPPSVNRVMLYNVGCHAGGTALRVAKDLAENN
RGARVLAVCSEVTVLSYRGPHPAHIESLFVQALFGDGAAALVVGSDPVDGVERPIFEIAS
ASQVMLPESAEAVGGHLREIGLTFHLKSQLPSIIASNIEQSLTTACSPLGLSDWNQLFWA
VHPGGRAILDQVEARLGLEKDRLAATRHVLSEYGNMQSATVLFILDEMRNRSAAEGHATT
GEGLDWGVLLGFGPGLSIETVVLHSCRLN*
SEQ ID NO: 5
   curcumin synthase 1 (CURS1)
   Amino acid
   *Curcuma longa* 
MANLHALRREQRAQGPATIMAIGTATPPNLYEQSTFPDFYFRVTNSDDKQELKKKFRRMC
EKTMVKKRYLHLTEEILKERPKLCSYKEASFDDRQDIVVEEIPRLAKEAAEKAIKEWGRP
KSEITHLVFCSISGIDMPGADYRLATLLGLPLTVNRLMIYSQACHMGAAMLRIAKDLAEN
NRGARVLVVACEITVLSFRGPNEGDFEALAGQAGFGDGAGAVVVGADPLEGIEKPIYEIA
AAMQETVAESQGAVGGHLRAFGWTFYFLNQLPAIIADNLGRSLERALAPLGVREWNDVFW
VAHPGNWAIMDAIEAKLQLSPDKLSTARHVFTEYGNMQSATVYFVMDELRKRSAVEGRST
TGDGLQWGVLLGFGPGLSIETVVLRSMPL*
SEQ ID NO: 6
   curcumin synthase 1 (CURS1) ("codon-optimized")
   Amino acid
   *Curcuma longa* 
MANLHALRREQRAQGPATIMAIGTATPPNLYEQSTFPDFYFRVTNSDDKQELKKKFRRMC
EKTMVKKRYLHLTEEILKERPKLCSYKEASFDDRQDIVVEEIPRLAKEAAEKAIKEWGRP
KSEITHLVFCSISGIDMPGADYRLATLLGLPLTVNRLMIYSQACHMGAAMLRIAKDLAEN
NRGARVLVVACEITVLSFRGPNEGDFEALAGQAGFGDGAGAVVVGADPLEGIEKPIYEIA
AAMQETVAESQGAVGGHLRAFGWTFYFLNQLPAIIADNLGRSLERALAPLGVREWNDVFW
VAHPGNWAIMDAIEAKLQLSPDKLSTARHVFTEYGNMQSATVYFVMDELRKRSAVEGRST
TGDGLQWGVLLGFGPGLSIETVVLRSMPL*
SEQ ID NO: 7
   curcumin synthase 2 (CURS2)
   Amino acid
   *Curcuma longa* 
MAMISLQAMRKAQRAQGPATILAVGTANPPNLYEQDTYPDYYFRVTNSEHKQELKNKFRLM
CEKTMVKRRYLYLTPEILKERPKLCSYMEPSFDDRQDIVVEEVPKLAAEAAENAIKEWGGDK
SAITHLVFCSISGIDMPGADYRLAQLLGLPLAVNRLMLYSQACHMGAAMLRIAKDIAENNRSA
RVLVVACEITVLSFRGPDERDFQALAGQAGFGDGAGAMIVGADPVLGVERPLYHIMSATQT
TVPESEKAVGGHLREVGLTFHFFNQLPAIIADNVGNSLAEAFEPIGIKDWNNIFWVAHPGNW
AIMDAIETKLGLEQSKLATARHVFSEFGNMQSATVYFVMDELRKRSAAENRATTGDGLRWG
VLFGFGPGISIETVVLQSVPL
SEQ ID NO: 8
   curcumin synthase 3 (CURS3)
   Amino acid
   *Curcuma longa* 
MGSLQAMRRAQRAQGPATIMAVGTSNPPNLYEQTSYPDFYFRVTNSDHKHALKNKFRVIC
EKTKVKRRYLHLTEEILKQRPKLCSYMEPSFDDRQDIVVEEIPKLAKEAAEKAIKEWGRPKSE
ITHLVFCSISGIDMPGADYRLATLLGLPLSVNRLMLYSQACHMGAQMLRIAKDLAENNRGAR
VLAVSCEITVLSFRGPDAGDFEALACQAGFGDGAAAVVVGADPLPGVERPIYEIAAAMQETV
PESERAVGGHLREIGWTFHFFNQLPKLIAENIEGSLARAFKPLGISEWNDVFWVAHPGNWGI
MDAIETKLGLEQGKLATARHVFSEYGNMQSATVYFVMDEVRKRSAAEGRATTGEGLEWGV
LFGFGPGLTIETVVLRSVPLP
SEQ ID NO: 9
   4-coumarate-CoA ligase (4CL)
   Amino acid
   *Arabidopsis thaliana* 
MAPQEQAVSQVMEKQSNNNNSDVIFRSKLPDIYIPNHLSLHDYIFQNISEFATKPCLING
PTGHVYTYSDVHVISRQIAANFHKLGVNQNDVVMLLLPNCPEFVLSFLAASFRGATATAA
NPFFTPAEIAKQAKASNTKLIITEARYVDKIKPLQNDDGVVIVCIDDNESVPIPEGCLRF
TELTQSTTEASEVIDSVEISPDDVVALPYSSGTTGLPKGVMLTHKGLVTSVAQQVDGENP
NLYFHSDDVILCVLPMFHIYALNSIMLCGLRVGAAILIMPKFEINLLLELIQRCKVTVAP
MVPPIVLAIAKSSETEKYDLSSIRVVKSGAAPLGKELEDAVNAKFPNAKLGQGYGMTEAG
PVLAMSLGFAKEPFPVKSGACGTVVRNAEMKIVDPDTGDSLSRNQPGEICIRGHQIMKGY
LNNPAATAETIDKDGWLHTGDIGLIDDDDELFIVDRLKELIKYKGFQVAPAELEALLIGH
PDITDVAVVAMKEEAAGEVPVAFVVKSKDSELSEDDVKQFVSKQVVFYKRINKVFFTESI
 PKAPSGKILRKDLRAKLANGL*
SEQ ID NO: 10
   4-coumarate-CoA ligase (4CL) ("codon-optimized")
   Amino acid
   *Arabidopsis thaliana* 
MAPQEQAVSQVMEKQSNNNNSDVIFRSKLPDIYIPNHLSLHDYIFQNISEFATKPCLING
PTGHVYTYSDVHVISRQIAANFHKLGVNQNDVVMLLLPNCPEFVLSFLAASFRGATATAA
NPFFTPAEIAKQAKASNTKLIITEARYVDKIKPLQNDDGVVIVCIDDNESVPIPEGCLRF
TELTQSTTEASEVIDSVEISPDDVVALPYSSGTTGLPKGVMLTHKGLVTSVAQQVDGENP
NLYFHSDDVILCVLPMFHIYALNSIMLCGLRVGAAILIMPKFEINLLLELIQRCKVTVAP
MVPPIVLAIAKSSETEKYDLSSIRVVKSGAAPLGKELEDAVNAKFPNAKLGQGYGMTEAG
PVLAMSLGFAKEPFPVKSGACGTVVRNAEMKIVDPDTGDSLSRNQPGEICIRGHQIMKGY
LNNPAATAETIDKDGWLHTGDIGLIDDDDELFIVDRLKELIKYKGFQVAPAELEALLIGH
PDITDVAVVAMKEEAAGEVPVAFVVKSKDSELSEDDVKQFVSKQVVFYKRINKVFFTESI
PKAPSGKILRKDLRAKLANGL*
SEQ ID NO: 11
   curcuminoid synthase (CUS)
   DNA
   *Oryza sativa* 
ATGGCACCGACGACGACCATGGGAAGCGCCTTATACCCGCTCGGCGAAATGCGGCGG
TCGCAGCGCGCCGACGGGCTCGCCGCCGTGCTCGCCATCGGCACCGCCAACCCTCCC
AACTGCGTTACCCAGGAGGAGTTCCCCGACTTCTACTTCCGCGTCACCAACAGCGACC
ACCTCACTGCCCTCAAGGACAAGTTCAAGCGTATCTGTCAGGAAATGGGCGTGCAGAG
GCGGTACCTGCACCACACGGAGGAGATGCTGTCCGCACACCCGGAGTTCGTGGACCG
CGACGCGCCGTCGCTCGACGCGCGGCTGGACATCGCCGCGGACGCCGTGCCGGAGC
TGGCGGCGGAGGCCGCCAAGAAGGCGATCGCCGAGTGGGGCCGCCCGGCCGCCGAC
ATCACCCACCTCGTCGTCACCACCAACTCCGGCGCCCACGTCCCGGGTGTCGACTTCC
GCCTCGTCCCGCTCCTCGGCCTCCGCCCCTCCGTGCGCCGCACCATGCTCCACCTCAA
CGGCTGCTTCGCCGGCTGCGCCGCGCTCCGCCTCGCCAAGGACCTCGCCGAGAACAG
CCGCGGCGCGCGCGTCCTCGTCGTCGCCGCCGAGCTCACCCTCATGTACTTCACCGG
GCCCGACGAGGGCTGCTTCAGGACGCTCCTCGTCCAGGGCCTGTTCGGCGACGGCGC
GGCCGCCGTCATTGTCGGCGCCGACGCCGACGACGTCGAGCGCCCGCTGTTCGAGAT
AGTGTCGGCGGCGCAGACGATCATCCCGGAGTCGGACCACGCCCTGAACATGCGGTT
CACGGAGCGCCGCTTGGACGGCGTCCTCGGGCGGCAGGTCCCGGGGCTCATCGGGG
ACAACGTCGAGCGGTGCCTCCTGGACATGTTCGGCCCACTTCTCGGCGGCGACGGCG
 
GCGGCGGGTGGAACGACCTGTTCTGGGCGGTGCACCCGGGCTCGTCGACGATCATGG
ACCAGGTCGACGCGGCGCTCGGCCTCGAGCCCGGCAAGCTGGCGGCGAGCCGCCGT
GTGCTCAGCGACTACGGGAACATGTCTGGCGCTACGGTGATCTTCGCGCTGGACGAGC
TGCGCCGGCAGCGCAAGGAGGCGGCGGCGGGTGAATGGCCGGAGTTGGGAGTG
ATGATGGCGTTCGGCCCGGGGATGACCGTTGATGCGATGCTGTTGCACGCCACCTCTC
ATGTGAATTAA
SEQ ID NO: 12
   curcuminoid synthase (CUS), codon-optimized
   DNA
   *artificial* 
ATGGCCCCAACCACCACCATGGGCTCCGCACTGTACCCACTGGGTGAAATGCGCCGCT
CCCAGCGTGCTGATGGTCTGGCAGCTGTGCTGGCTATCGGTACCGCCAACCCACCAAA
CTGCGTCACCCAGGAAGAGTTCCCAGATTTCTACTTCCGCGTTACCAACTCCGACCACC
TGACCGCACTGAAGGATAAGTTCAAGCGCATCTGCCAGGAAATGGGCGTGCAGCGCCG
CTACCTGCACCACACCGAAGAGATGCTGTCCGCTCACCCAGAGTTCGTTGATCGTGATG
CACCATCCCTGGATGCACGTCTGGACATCGCCGCAGATGCAGTGCCAGAACTGGCTGC
CGAGGCAGCTAAGAAGGCAATCGCTGAATGGGGTCGTCCAGCAGCAGACATCACCCAC
CTGGTGGTCACCACCAACTCCGGCGCTCACGTGCCAGGTGTCGATTTCCGTCTGGTGC
CTCTGCTGGGTCTGCGTCCATCCGTCCGTCGCACCATGCTGCACCTGAACGGTTGCTT
CGCTGGTTGCGCTGCACTGCGTCTGGCAAAGGACCTGGCTGAGAACTCCCGCGGCGC
ACGTGTCCTGGTTGTGGCAGCTGAACTGACCCTGATGTACTTCACCGGCCCAGATGAG
GGTTGCTTCCGCACCCTGCTGGTGCAGGGTCTGTTCGGTGATGGTGCAGCAGCTGTCA
TCGTTGGTGCCGATGCAGATGACGTCGAACGCCCACTGTTCGAGATCGTTTCCGCCGC
ACAGACCATCATCCCAGAATCCGACCACGCTCTGAACATGCGTTTCACCGAGCGCCGC
CTGGATGGTGTTCTGGGTCGTCAGGTGCCAGGTCTGATCGGTGACAACGTGGAACGCT
GCCTGCTGGATATGTTCGGTCCACTGCTGGGCGGTGACGGCGGTGGCGGTTGGAACG
ACCTGTTCTGGGCAGTCCACCCAGGTTCCTCCACCATCATGGATCAGGTTGATGCTGCA
CTGGGTCTGGAGCCAGGCAAGCTGGCAGCTTCCCGTCGCGTGCTGTCCGACTACGGC
AACATGTCCGGTGCTACCGTCATCTTCGCCCTGGATGAACTGCGTCGCCAGCGTAAGG
AAGCAGCAGCTGCTGGTGAATGGCCAGAGCTGGGTGTTATGATGGCATTCGGCCCTGG
TATGACCGTGGACGCAATGCTGCTGCACGCTACCTCTCACGTCAACTAA
SEQ ID NO: 13
   diketide-CoA synthase (DCS)
   DNA
   *Curcuma longa* 
ATGGAAGCGAACGGCTACCGCATAACTCACAGCGCCGACGGGCCGGCGACGATCTTG
GCCATCGGCACCGCCAACCCCACCAACGTCGTCGATCAGAACGCTTATCCCGACTTCT
ATTTCCGGGTCACCAACTCCGAGTATCTGCAGGAACTCAAAGCCAAGTTTAGGCGCATC
TGTGAGAAAGCGGCCATCAGGAAGAGGCACTTGTACTTGACTGAGGAGATTTTGCGGG
AGAATCCTAGCTTGCTGGCTCCCATGGCGCCGTCGTTCGACGCGCGGCAGGCGATCGT
GGTGGAGGCGGTGCCGAAGCTGGCGAAGGAGGCGGCGGAGAAGGCGATCAAGGAGT
GGGGCCGCCCCAAATCGGACATCACGCACCTCGTCTTCTGCTCCGCGAGCGGAATCGA
CATGCCCGGCTCCGACCTGCAGCTTCTCAAGCTGCTCGGGCTCCCGCCGAGCGTCAAT
CGCGTCATGCTCTACAACGTCGGGTGCCACGCCGGTGGCACCGCCCTCCGCGTCGCC
AAGGACCTCGCGGAGAACAACCGCGGCGCGCGGGTGCTCGCCGTCTGCTCCGAGGTC
ACCGTGCTCTCCTACCGCGGCCCCCACCCCGCCCACATCGAGAGCCTCTTCGTCCAAG
CTCTGTTTGGCGACGGCGCCGCCGCGCTCGTGGTCGGGTCCGACCCCGTCGATGGCG
TCGAGCGCCCCATCTTCGAAATCGCCTCGGCATCCCAAGTGATGCTTCCGGAGAGCGC
AGAGGCGGTGGGCGGCCACCTCCGCGAAATTGGGCTGACCTTCCACCTCAAGAGCCA
GCTTCCGTCGATCATCGCGAGCAACATCGAGCAGAGCCTGACGACTGCGTGCTCGCCG
CTGGGGCTGTCGGACTGGAACCAGCTGTTCTGGGCGGTTCACCCCGGCGGCCGAGCG
ATCCTGGACCAGGTGGAGGCGCGGCTCGGACTGGAGAAGGACCGGCTCGCCGCGAC
GCGGCACGTACTCAGCGAGTACGGCAACATGCAGAGCGCCACGGTGCTGTTCATCCTG
GACGAGATGCGGAACCGCTCGGCTGCGGAGGGCCACGCCACCACCGGCGAGGGGCT
CGACTGGGGCGTGCTGTTGGGCTTCGGCCCGGGACTCTCCATCGAGACCGTCGTCCT
CCATAGTTGCAGACTGAACTAG
SEQ ID NO: 14
   diketide-CoA synthase (DCS), codon-optimized
   DNA
   *artificial* 
ATGGAAGCAAACGGCTACCGCATCACCCACTCCGCTGATGGTCCAGCCACCATCCTGG
CAATCGGCACCGCTAACCCAACCAACGTGGTCGATCAGAACGCCTACCCAGACTTCTAC
TTCCGCGTCACCAACTCCGAATACCTGCAGGAGCTGAAGGCTAAGTTCCGCCGCATCT
GCGAAAAGGCAGCTATCCGCAAGCGCCACCTGTACCTGACCGAAGAGATCCTGCGCGA
GAACCCATCCCTGCTGGCACCAATGGCTCCATCCTTCGATGCCCGCCAGGCAATCGTT
GTGGAAGCCGTGCCAAAGCTGGCAAAGGAAGCCGCAGAGAAGGCAATCAAGGAGTGG
GGCCGCCCAAAGTCCGACATCACCCACCTGGTCTTCTGCTCCGCTTCCGGTATCGATAT
GCCAGGCTCCGACCTGCAGCTGCTGAAGCTGCTGGGTCTGCCACCATCCGTCAACCGC
GTTATGCTCTACAACGTTGGTTGCCACGCCGGCGGTACCGCACTGCGCGTCGCTAAGG
ATCTGGCCGAAAACAACCGCGGTGCACGTGTGCTGGCTGTCTGCTCCGAGGTTACCGT
GCTGTCCTACCGTGGTCCACACCCAGCTCACATCGAATCCCTGTTCGTGCAGGCACTGT
 
TCGGTGATGGTGCTGCAGCACTGGTCGTTGGTTCCGATCCAGTTGACGGCGTGGAACG
CCCAATCTTCGAGATCGCCTCCGCATCCCAGGTCATGCTGCCAGAATCCGCTGAGGCT
GTTGGCGGTCACCTGCGCGAAATCGGTCTGACCTTCCACCTGAAGTCCCAGCTGCCAT
CCATCATCGCATCCAACATCGAGCAGTCCCTCACCACCGCTTGCTCCCCACTGGGTCTG
TCCGACTGGAACCAGCTGTTCTGGGCTGTTCACCCAGGCGGTCGTGCAATCCTGGATC
AGGTGGAAGCACGCCTGGGTCTGGAGAAGGACCGTCTGGCTGCAACCCGTCACGTCC
TGTCCGAATACGGCAACATGCAGTCCGCAACCGTTCTGTTCATCCTGGATGAGATGCGC
AACCGCTCCGCAGCTGAAGGTCACGCTACCACCGGTGAAGGTCTGGACTGGGGCGTG
CTGCTGGGTTTCGGTCCAGGTCTGTCCATCGAGACCGTGGTCCTGCACTCCTGCCGCC
TGAACTAA
SEQ ID NO: 15
   curcumin synthase 1 (CURS1)
   DNA
   *Curcuma longa* 
ATGGCCAACCTCCACGCGTTGCGCAGGGAGCAGAGGGCTCAAGGTCCCGCCACCATC
ATGGCCATCGGGACGGCCACCCCTCCCAACCTCTACGAGCAGAGCACCTTCCCGGACT
TCTACTTCCGCGTCACCAACTCCGACGACAAGCAGGAGCTCAAGAAAAAGTTCCGCCG
CATGTGCGAGAAGACAATGGTGAAGAAGCGGTACCTGCACTTGACCGAGGAGATCCTG
AAGGAGAGGCCCAAGCTCTGCTCCTACAAGGAGGCGTCGTTCGACGACCGGCAGGAC
ATCGTGGTGGAGGAGATACCGAGATTGGCTAAGGAAGCTGCGGAGAAGGCCATCAAGG
AGTGGGGGCGGCCCAAATCGGAGATCACCCACCTGGTCTTCTGTTCCATCAGCGGCAT
CGACATGCCCGGCGCCGACTACCGCCTCGCGACGCTCCTCGGCCTCCCTCTCACCGT
CAACCGCCTCATGATCTACAGCCAGGCCTGCCACATGGGCGCCGCCATGCTCCGCATC
GCCAAGGACCTCGCCGAGAACAACAGGGGCGCGCGCGTGCTGGTGGTCGCCTGCGAG
ATCACCGTGCTCAGCTTCCGCGGCCCGAACGAGGGTGACTTCGAGGCGCTCGCGGGG
CAGGCCGGCTTCGGCGACGGCGCGGGGGCCGTCGTCGTCGGGGCCGACCCGCTGGA
AGGAATTGAAAAACCCATCTACGAGATCGCGGCGGCGATGCAGGAGACGGTGGCGGA
GAGCCAGGGGGCGGTGGGCGGCCACCTGCGGGCGTTCGGCTGGACGTTCTACTTCCT
GAACCAGCTGCCGGCGATCATCGCCGACAACCTCGGGAGGAGCCTGGAGCGGGCGTT
GGCGCCGCTGGGGGTGAGGGAGTGGAACGACGTCTTCTGGGTGGCGCACCCGGGCA
ACTGGGCCATCATGGACGCCATCGAAGCCAAGCTGCAGCTGAGCCCGGACAAGCTCAG
CACCGCCCGCCACGTCTTCACAGAGTACGGCAACATGCAGAGCGCCACCGTGTACTTC
GTGATGGATGAGCTGAGGAAGCGGTCGGCGGTGGAGGGGCGGAGCACCACCGGAGA
CGGCTTGCAGTGGGGAGTTCTCCTCGGTTTTGGGCCGGGCCTCAGCATCGAGACCGTT
GTACTGCGCAGTATGCCACTGTAG
SEQ ID NO: 16
   curcumin synthase 1 (CURS1), codon-optimized
   DNA
   *artificial* 
ATGGCCAACCTGCACGCACTGCGTCGCGAACAGCGTGCTCAGGGTCCAGCAACCATCA
TGGCAATCGGCACCGCTACCCCACCAAACCTGTACGAGCAGTCCACCTTCCCAGATTTC
TACTTCCGCGTCACCAACTCCGATGACAAGCAGGAACTGAAGAAGTTCCGTCGCAT
GTGCGAGAAGACCATGGTTAAGAAGCGCTACCTGCACCTGACCGAAGAGATCCTGAAG
GAACGCCCAAAGCTGTGCTCCTACAAGGAAGCATCCTTCGATGACCGCCAGGACATCG
TGGTCGAAGAGATCCCACGCCTGGCAAAGGAAGCAGCTGAGAAGGCTATCAAGGAATG
GGGCCGCCCAAAGTCCGAGATCACCCACCTGGTGTTCTGCTCCATCTCCGGTATCGAT
ATGCCAGGTGCTGATTACCGTCTGGCAACCCTGCTGGGTCTGCCACTGACCGTCAACC
GCCTGATGATCTACTCCCAGGCATGCCACATGGGTGCCGCAATGCTGCGCATCGCCAA
GGATCTGGCAGAAAACAACCGCGGCGCACGTGTGCTGGTTGTGGCATGCGAGATCACC
GTCCTGTCCTTCCGCGGTCCAAACGAAGGCGACTTCGAGGCTCTGGCTGGTCAGGCTG
GTTTCGGTGATGGCGCAGGTGCTGTCGTTGTGGGTGCAGACCCACTGGAAGGCATCGA
GAAGCCAATCTACGAAATCGCTGCCGCAATGCAGGAAACCGTCGCAGAGTCCCAGGGT
GCTGTTGGCGGTCACCTGCGCGCTTTCGGTTGGACCTTCTACTTCCTGAACCAGCTGC
CAGCTATCATCGCCGATAACCTCGGTCGCTCCCTCGAACGTGCACTGGCACCACTGGG
CGTTCGTGAGTGGAACGACGTGTTCTGGGTCGCTCACCCTGGTAACTGGGCCATCATG
GATGCAATCGAAGCTAAGCTGCAGCTGTCCCCAGACAAGCTGTCCACCGCTCGCCACG
TGTTCACCGAGTACGGCAACATGCAGTCCGCCACCGTTTACTTCGTGATGGATGAACTG
CGCAAGCGCTCCGCAGTTGAAGGTCGCTCCACCACCGGTGATGGTCTGCAGTGGGGC
GTGCTGCTGGGTTTCGGTCCAGGTCTGTCCATCGAGACCGTCGTTCTGCGCTCCATGC
CACTGTAA
SEQ ID NO: 17
   curcumin synthase 2 (CURS2)
   DNA
   *Curcuma longa* 
ATGGCGATGATCAGCTTGCAGGCGATGCGCAAGGCGCAGAGAGCTCAAGGTCCGGCC
ACCATCTTGGCCGTCGGCACCGCCAACCCGCCCAATCTCTACGAGCAGGACACGTATC
CCGACTACTACTTCCGCGTCACCAACTCCGAGCACAAGCAGGAGCTCAAGAACAAGTTC
CGCCTCATGTGCGAGAAGACGATGGTGAAGAGGCGGTATCTTTACCTGACGCCGGAGA
TCCTGAAGGAGCGGCCGAAGCTGTGCTCGTACATGGAGCCGTCGTTCGACGACCGGC
AGGACATCGTGGTGGAGGAGGTGCCGAAGCTGGCCGCGGAGGCAGCGGAGAACGCC
ATCAAGGAGTGGGGCGGCGACAAGTCCGCCATCACCCACCTGGTCTTCTGCTCCATCA
 
GCGGCATCGACATGCCCGGAGCTGACTACCGCCTCGCCCAGCTCCTCGGACTCCCGC
TCGCCGTCAACCGCCTGATGCTCTACAGCCAGGCCTGCCACATGGGCGCCGCCATGCT
GCGCATAGCCAAGGACATCGCCGAGAACAACCGCTCCGCGCGCGTCCTCGTCGTCGC
CTGCGAGATCACCGTGCTCAGCTTCCGCGGCCCGGACGAGCGCGACTTCCAGGCGCT
GGCCGGCCAGGCCGGCTTCGGGGACGGCGCCGGCGCGATGATCGTCGGGGCCGACC
CCGTCCTCGGCGTCGAGCGGCCGCTCTACCACATCATGTCGGCGACTCAGACGACGGT
ACCGGAGAGCGAGAAGGCGGTGGGGGGCCACCTCCGCGAGGTGGGGCTGACCTTCC
ACTTCTTCAACCAGCTGCCGGCGATCATCGCCGACAACGTGGGGAACAGCCTGGCGGA
GGCGTTCGAACCGATCGGGATCAAGGACTGGAACAACATCTTCTGGGTGGCGCACCCG
GGCAACTGGGCCATCATGGACGCCATCGAGACCAAGCTGGGCCTGGAACAGAGCAAG
CTGGCCACCGCACGCCACGTCTTCTCCGAGTTCGGCAACATGCAGAGCGCCACCGTCT
ACTTCGTGATGGACGAGCTCAGGAAACGGTCGGCGGCGGAGAACCGGGCGACCACCG
GCGACGGGCTCCGGTGGGGCGTGCTCTTCGGCTTCGGCCCGGGCATCAGCATCGAAA
CCGTCGTGCTCCAAAGCGTGCCGCTTTAG
SEQ ID NO: 18
   curcumin synthase 3 (CURS3)
   DNA
   *Curcuma longa* 
ATGAATACAATTAACGAATATTTAAGTTTAGAAGAATTTGAGGCCATAATCTTTGGAAATC
AAAAAGTTACAATTAGCGACGTTGTCGTAAACCGAGTTAATGAAAGTTTCAATTTCTTAAA
AGAATTCTCTGGGAATAAAGTAATTTACGGCGTAAACACTGGTTTTGGACCAATGGCGC
AGTATAGAATTAAAGAGTCTGATCAAATTCAGCTTCAATATAATTTAATTAGAAGCCACTC
TTCGGGAACCGGAAAGCCATTAAGCCCGGTTTGTGCTAAAGCAGCAATTTTAGCTCGTT
TAAACACTCTTTCATTAGGAAATTCAGGCGTTCACCCGTCAGTAATCAACCTAATGTCTG
AGCTTATCAATAAAGATATTACCCCTTTAATCTTCGAACACGGAGGCGTTGGTGCCAGC
GGTGACTTAGTACAGCTGTCACATTTAGCTTTAGTGTTAATTGGCGAAGGTGAAGTTTTT
TATAAAGGTGAAAGAAGACCAACTCCTGAAGTTTTTGAAATTGAAGGTTTAAAACCAATT
CAGGTAGAAATTAGAGAAGGTCTGGCATTAATTAACGGAACTTCAGTAATGACTGGAATT
GGAGTTGTAAATGTTTATCATGCTAAAAAATTATTAGACTGGTCATTAAAATCTTCTTGTG
CAATTAACGAATTGGTTCAGGCTTATGACGATCATTTCTCAGCAGAACTAAACCAGACTA
AACGTCATAAAGGACAGCAGGAAATTGCGTTGAAAATGCGTCAAAATCTTTCAGACAGT
ACTTTAATCCGTAAAAGAGAAGATCATTTATATTCTGGTGAAAATACCGAAGAAATCTTCA
AAGAAAAAGTTCAGGAATATTATTCATTAAGATGCGTTCCTCAAATTTTAGGACCGGTTTT
AGAAACTATTAATAATGTAGCTTCTATTCTGGAAGACGAATTTAACTCGGCAAACGATAA
CCCAATTATCGATGTAAAAAACCAGCATGTTTACCACGGCGGAAATTTCCACGGAGATTA
TATTTCGCTTGAAATGGATAAACTAAAAATTGTTATTACCAAATTAACAATGCTGGCAGAA
 
CGTCAGTTGAACTATTTATTAAATTCAAAAATCAACGAATTACTTCCTCCGTTTGTAAACT
TAGGAACATTAGGATTTAATTTCGGAATGCAGGGTGTTCAGTTTACAGCAACATCTACAA
CGGCAGAAAGTCAAATGCTGTCGAACCCAATGTATGTTCACAGTATTCCAAACAACAAC
GACAATCAGGATATCGTAAGTATGGGAACTAACTCGGCAGTAATTACCTCAAAAGTAATC
GAAAATGCTTTTGAAGTTCTGGCTATCGAAATGATTACTATCGTTCAGGCAATTGATTATT
TAGGACAAAAAGACAAAATTTCATCTGTTTCTAAAAAATGGTACGATGAAATTAGAAATAT
AATTCCAACATTTAAGGAAGATCAGGTAATGTATCCTTTCGTTCAAAAAGTAAAAGATCAT
TTGATCAACAATTAA
SEQ ID NO: 19
   4-coumarate-CoA ligase (4CL)
   DNA
   *Arabidopsis thaliana* 
ATGGCGCCACAAGAACAAGCAGTTTCTCAGGTGATGGAGAAACAGAGCAACAACAACA
ACAGTGACGTCATTTTCCGATCAAAGTTACCGGATATTTACATCCCGAACCACCTATCTC
TCCACGACTACATCTTCCAAAACATCTCCGAATTCGCCACTAAGCCTTGCCTAATCAACG
GACCAACCGGCCACGTGTACACTTACTCCGACGTCCACGTCATCTCCCGCCAAATCGC
CGCCAATTTTCACAAACTCGGCGTTAACCAAAACGACGTCGTCATGCTCCTCCTCCCAA
ACTGTCCCGAATTCGTCCTCTCTTTCCTCGCCGCCTCCTTCCGCGGCGCAACCGCCAC
CGCCGCAAACCCTTTCTTCACTCCGGCGGAGATAGCTAAACAAGCCAAAGCCTCCAACA
CCAAACTCATAATCACCGAAGCTCGTTACGTCGACAAAATCAAACCACTTCAAAACGAC
GACGGAGTAGTCATCGTCTGCATCGACGACAACGAATCCGTGCCAATCCCTGAAGGCT
GCCTCCGCTTCACCGAGTTGACTCAGTCGACAACCGAGGCATCAGAAGTCATCGACTC
GGTGGAGATTTCACCGGACGACGTGGTGGCACTACCTTACTCCTCTGGCACGACGGGA
TTACCAAAAGGAGTGATGCTGACTCACAAGGGACTAGTCACGAGCGTTGCTCAGCAAGT
CGACGGCGAGAACCCGAATCTTTATTTCCACAGCGATGACGTCATACTCTGTGTTTTGC
CCATGTTTCATATCTACGCTTTGAACTCGATCATGTTGTGTGGTCTTAGAGTTGGTGCGG
CGATTCTGATAATGCCGAAGTTTGAGATCAATCTGCTATTGGAGCTGATCCAGAGGTGT
AAAGTGACGGTGGCTCCGATGGTTCCGCCGATTGTGTTGGCCATTGCGAAGTCTTCGG
AGACGGAGAAGTATGATTTGAGCTCGATAAGAGTGGTGAAATCTGGTGCTGCTCCTCTT
GGTAAAGAACTTGAAGATGCCGTTAATGCCAAGTTTCCTAATGCCAAACTCGGTCAGGG
ATACGGAATGACGGAAGCAGGTCCAGTGCTAGCAATGTCGTTAGGTTTTGCAAAGGAAC
CTTTTCCGGTTAAGTCAGGAGCTTGTGGTACTGTTGTAAGAAATGCTGAGATGAAAATAG
TTGATCCAGACACCGGAGATTCTCTTTCGAGGAATCAACCCGGTGAGATTTGTATTCGT
GGTCACCAGATCATGAAAGGTTACCTCAACAATCCGGCAGCTACAGCAGAGACCATTGA
TAAAGACGGTTGGCTTCATACTGGAGATATTGGATTGATCGATGACGATGACGAGCTTT
TCATCGTTGATCGATTGAAAGAACTTATCAAGTATAAAGGTTTTCAGGTAGCTCCGGCTG
 
AGCTAGAGGCTTTGCTCATCGGTCATCCTGACATTACTGATGTTGCTGTTGTCGCAATG
AAAGAAGAAGCAGCTGGTGAAGTTCCTGTTGCATTTGTGGTGAAATCGAAGGATTCGGA
GTTATCAGAAGATGATGTGAAGCAATTCGTGTCGAAACAGGTTGTGTTTTACAAGAGAAT
CAACAAAGTGTTCTTCACTGAATCCATTCCTAAAGCTCCATCAGGGAAGATATTGAGGAA
AGATCTGAGGGCAAAACTAGCAAATGGATTGTGA
SEQ ID NO: 20
   4-coumarate-CoA ligase (4CL), codon-optimized
   DNA
   *artificial* 
ATGGCACCACAGGAACAGGCTGTCTCCCAGGTTATGGAGAAGCAGTCCAACAACAACA
ACTCCGATGTTATCTTCCGCTCCAAGCTGCCAGACATCTACATCCCAAACCACCTGTCC
CTGCACGATTACATCTTCCAGAACATCTCCGAATTCGCAACCAAGCCATGCCTGATCAA
CGGTCCAACCGGCCACGTGTACACCTACTCCGATGTCCACGTTATCTCCCGCCAGATC
GCAGCTAACTTCCACAAGCTGGGCGTGAACCAGAACGACGTGGTCATGCTGCTGCTGC
CAAACTGCCCAGAGTTCGTCCTGTCCTTCCTGGCTGCATCCTTCCGCGGTGCCACCGC
AACCGCTGCAAACCCATTCTTCACCCCAGCTGAAATCGCCAAGCAGGCTAAGGCCTCCA
ACACCAAGCTGATCATCACCGAGGCTCGCTACGTTGACAAGATCAAGCCACTGCAGAAC
GATGACGGCGTTGTGATCGTCTGCATCGATGACAACGAATCCGTTCCAATCCCAGAGG
GTTGCCTGCGCTTCACCGAACTGACCCAGTCCACCACCGAAGCATCCGAGGTTATCGA
TTCCGTGGAGATCTCCCCAGATGACGTCGTTGCTCTGCCATACTCCTCCGGTACCACCG
GTCTGCCAAAGGGCGTCATGCTGACCCACAAGGGTCTGGTGACCTCTGTGGCACAGCA
GGTTGACGGCGAAAACCCAAACCTGTACTTCCACTCCGATGACGTCATCCTGTGCGTTC
TGCCAATGTTCCACATCTACGCTCTGAACTCCATCATGCTGTGCGGTCTGCGTGTTGGT
GCAGCTATCCTGATCATGCCAAAGTTCGAAATCAACCTGCTGCTGGAGCTGATCCAGCG
CTGCAAGGTGACCGTCGCACCAATGGTGCCACCAATCGTCCTGGCAATCGCTAAGTCC
TCCGAAACCGAGAAGTACGATCTGTCCTCCATCCGCGTGGTCAAGTCCGGTGCCGCAC
CACTGGGCAAGGAACTGGAGGACGCAGTGAACGCTAAGTTCCCAAACGCCAAGCTGGG
CCAGGGTTACGGTATGACCGAAGCTGGTCCAGTGCTGGCAATGTCCCTGGGTTTCGCA
AAGGAGCCATTCCCAGTCAAGTCCGGTGCTTGCGGCACCGTTGTGCGCAACGCCGAAA
TGAAGATCGTCGATCCAGACACCGGCGATTCCCTGTCCCGCAACCAGCCAGGCGAGAT
CTGCATCCGCGGCCACCAGATCATGAAGGGTTACCTGAACAACCCAGCTGCCACCGCA
GAGACCATCGATAAGGACGGCTGGCTGCACACCGGTGACATCGGCCTGATCGATGACG
ATGACGAACTGTTCATCGTTGACCGCCTGAAGGAGCTGATCAAGTACAAGGGCTTCCAG
GTGGCCCCAGCAGAACTGGAGGCTCTGCTGATCGGTCACCCAGACATCACCGACGTCG
CCGTCGTTGCAATGAAGGAAGAGGCAGCTGGCGAAGTTCCAGTGGCCTTCGTGGTCAA
GTCCAAGGATTCCGAACTGTCCGAGGATGACGTTAAGCAGTTCGTGTCCAAGCAGGTT
 
GTGTTCTACAAGCGCATCAACAAGGTTTTCTTCACCGAGTCCATCCCAAAGGCTCCATC
CGGCAAGATCCTGCGCAAGGACCTGCGCGCTAAGCTGGCCAACGGTCTGTAA
SEQ ID NO: 21
   *phdB*
   DNA
   Corynebacterium sp. 
ATGTCTCTCAATGGAAAAGTCGCCATCGTTACCGGATCTGGTGCAGGACTTGGTCGTTC
CTTCGCTCAGGAGCTTGCCCGTCAGGGTGCATCTGTCATCGTCAATGACGTAAACCAG
GCAGCCGCAGATGAGACTGTCGCAGCAATCACCGAAGCCGGCGGCAAAGCCGCCGCC
GTTATCGCCCCCGTTGGACCCTCTGAAAGCGCCGCATTGCTGGTGCGGGAGGCCGTC
GACAAGTTCGGTTCTTTGGACATTCTTGTCACAAACGCGGGCATCCTTCGTGATAGGTC
CCTGCTGAAGATGACGGACGATGATTTCGATGCAGTCATTAACGTGCACCTCAAGGGCA
CTTTCACCTGTGTTCGCGAGGCATTTGGATACTTCAAGGAGAATGGAATCGCGGGGCG
CATCGTCACGATTGGTTCTCCCACCGGGCAGCGCGGCAACTTCGGACAGAGCAATTAC
GCTGCAGCTAAGGCGGGCATTGTGGGTATGGTTCGCACGTGGGCGCTGGAGATGAAG
CGCGCAGGTGTCACCATTAACGCGATCATTCCGGAAGCAGCCACCGATATGACCAAGA
CGGTGCCATATTTCCAGAAGGCTGTAGAGGCCGATGAGCGTGGCGAGGCCATGCCAG
CATTCTTCCGCGAGACCCTAGGTTTTGGCACTCCTCAGGATGTTGCGGGACTTGTGGCC
TTCCTTTCCTCTGATGAGGCAGCGAATATTTCTGGACAGGCCATCGGTGCAGGCGGCG
ACCGCATGCAGGTGTGGAAGCACCCAGAGCCAGCAGTTACTGAATTTAACCCAGGTGG
CTGGACCTATGAAGCACTGCAGGAACGTGGCAAGAACATTATTGAGGGCAACCTGCAG
TCCGTCGGTGTCGTTTTCCCTGAACTGCCGGCAGAGCTTCAGCCACAAATCCCAGTCAA
GGCATAA
SEQ ID NO: 22
   *phdC*
   DNA
   Corynebacterium sp. 
ATGAGCAACAACGTAGTGAAATATGAGTGCGCGGTCGACGCCGACAACATTGTCGCAG
TCGATATGCATGTGCACTTGGAAGTCGACAGCTGCGGACACAAATCGATGCCGGCAGA
CATCATGGCGGCATCCTCGAAGTACTTTAAGACCGCGGAACGAACTCCCTCAGCAGAT
GCCATTGCTGATATTTATAGGGAACACAAGATGGCGGCGGTGGTTTTCACCATCGATGC
GCGGACCCAAATGGGGCATCTGCCGAACTCGATTGATGATTTGGTGGCAAGCTGTGCC
CGCAACAATGACGTGCTGATCCCTTTTGGCAGTGTGGATCCTCGTACCGGCGAGGACG
CGCTGGTGGAAGCTCGCCGACAGGTGGAAGAACTCGGGGTGCGAGGCTTCAAATTCCA
TCCATCGGTTCAAGGATTCGACCCATCCGCGCCAGAGTTCTACCCACTGTGGGAATTGC
 
TCGAAAGTTTTGGATTGCCATGCGTGTTCCATACCGGACAAAACGGCATGGGTGCAGGT
CTTCCAGGTGGTCGAGGCATTAAGCTGCGCTTCTCCAACCCAATGTTGCTTGATGATGT
TGCGGCGGACTTCCCGAACCTGACCATCATCATGGCGCACCCTTCTGTTCCTTGGCAG
GATGAGGCTAACTCGATTGCCACCCACAAGGCCAATGTGTTCATTGATCTTTCCGGCTG
GTCGCCGAAGTATTTCCCAGAGTCTTTGGTCAGACAGTCCAATAACGTGCTATCCAAGA
AGGTGCTGTTTGGCACGGACTTCCCGCTGATTACCCCAGAGAAATGGCTTGCGGCTTTC
GCGAATCTGCCACTGAAGGATGAGGTTCGTCCGGGAATCCTCAAAGACAATGCGGTGA
AGGTACTTGGCCTAGCCGCTAGCACTGAGCGCGGATCTCAAGCAGAAAAGGTCGTGCA
ACATGCGTGA
SEQ ID NO: 23
   *phdD*
   DNA
   Corynebacterium sp. 
ATGCGTGATCCCATTCAAGGTGCTGTTATTCCTTCTGATCTTTTTGGTTTCGCAGAAGTT
CTCACCGAAGCCGAACGCGCAGTTCTTCTGGAAACCCGCAGGGTGCTTGAGGAAGAGG
TGAAGCCTTATATTAATGAGGCCTGGGATAAGGCAGTCTTCCCCGATGAGATCGTGCAG
CCCCTCCAAGATCTGCAATTGCTTGATCCGCCTGCACTTCGGGAAGCAGGGGAGTCGG
TTCGAGACATTTTCACTGGTTTCCGCAATTTTGAACTCGCGCGCTGTGACATCAATGTTG
GTACCTATTACAACGCATCTGCTGGTCTCTTCCGAACGGCCTGCATGGTTGGTGGCTCC
CCGGAGCAGGCGCAGCGATTGGATGCGCAGATCAAATCTGGTGAGGTCAAGGGCGTTT
TTGCACTGACGGAACCTGATCATGGCTCTGATATCGCAGGTGGTCTGGCAACCACGGC
CACTAAGGACGCAGACACCGGCGAGTGGATTATCAATGGTGAAAAACGGTGGATCGGT
GGTGCTTCCACTGCTGATTTGATCGCTACCTTCGCCAGGGATACAGCCGATAACCAGGT
GAAATGCTTCCTCGTGGCACCTCAGGCAGAGGGCGTGTCCATGGAGATTATTGATCGC
AAAGCCTCACTGCGCATCATGCAAAATGCACACATTACCTATAACAATGTCCGGGTGTC
TGGGGATGCGCGGCTGCACAACATCAATTCTTTCAAGGATGTTTCGGAATGCCTGCGCC
GTATGCGTTCCGATGTGGCGTGGATGGCGGTCGGTGCGCAGGCAGGTGCCTATGAAG
CAGCCGTGAAGTATGTGCGCAGCAGGGAACAGTTTGGCCGTCCGATCGCGGGGTTCCA
GTTGATTCAGGAAAAGCTCGCGCTCATGCTGGGCAATCTCACGGCGTCGCTGGGCATG
ATGGTCAAACTCACCGATCAGCAGCAGGCGGGAATTTTCAAAGAGGAAAACTCCGCGC
TGGCGAAAATGTTTACCTCGCTCAAACTTCGGGAGACCGCTAGTTGGGCGCGGGAAAT
CTGCGGAGGCAACGGCATCATTTTGGACAACGATGTTGCCCGGTTCCATGCCGATGCA
GAAGCCGTCTATTCATATGAAGGCACCCACGAAATCAATGCACTCATCGTTGGTCGCGC
CATCACGGGCGTCAGCGCTTTTATCTAA
SEQ ID NO: 24
   *phdE*
   DNA
   Corynebacterium sp. 
ATGACTACTTCCACCACCCCAAACACCATCGTTTCTTTCGAAGACGCACCAACCCTCAC
CGGCCAGGACCTGGGCTTTTCGCAGTGGCGCACTGTCACCCAGGAGATGGTGAACACC
TTCGCGGACGCAACTGATGATCAGCAGTGGATTCACACTGATCCTGAGCGCGCCAAGG
ACGGTCCTTTTGGTGGCGCAATTGCCCACGGTTTCCTCACCTTGTCCATGATCATTCCG
TTCTGGGGCGAGCTTCTCGATGTCACCGGCGTGACCACCAAGGTGAACTATGGCCTGG
ATAAGGTGCGTTTCACCTCTCCCGTCAAGGTCGGTTCCCGCATCCGCATGGGCGCTGT
GGTCCGTGAGATCTCTGAGGTGAAGGGCAATGGCCTGCACCTGGTCGCCGATGGCACT
ATTGAGATCGAAGGGCAGGAGCGCCCGGCCGTCGTAGCTACCTTCCTCACCCGCTTCT
ACGCTTAA
SEQ ID NO: 25
   *phdR*
   DNA
   Corynebacterium sp. 
ATGACCACCAGCAACCCCACCGCCGAGATCATTGGCGGACCAGAACGATTCCTCGAGG
CCGAATTGTCCCAGCAGATTCAATTCCTCACTGCCCGCGCACGAGCCAAGGGATCCGC
CAAAGGAAACGAAGCCTTAGTCGACCTCGGACTTAAAGTTCGCCAATACTCCACACTGT
CCCTAGCGGCCAGCGGATTAAAACCAACCCAACGAGAATTGGGAGCATTTCTCGACCTA
GACCCAAGTCAGATTGTTGCCTTGGTCGATTTCCTAGAAAAGCGCGGATTAGTGGCCCG
GGAAGTTGACCCCCGGGATAGGCGCTCGAAGATCATCATCGCCACCGAAAAAGGTCTG
GAAATTCACGACGAAGCCACCAAACGCCTCCTCATCGCCGAGGGTGAATCTCTAAAAAA
CCTCACCTCCGACGAGCAAGAACAACTAAGGGAACTGCTGCTCAAAATCGCCTTTTAA
SEQ ID NO: 26
   *phdA*
   DNA
   Corynebacterium sp. 
ATGAAAGTGAACCTCGGAATAGGAAGCTACCCACGACGCAGGGCAACTGTTCGACCAG
AGTCCACTGCAATCGAATTCGAAGGCACCAGCATCACCTACGGAGAATTCAGCAAACGA
GTCAATCGGCTTGGACATGCTCTTCTAGACCTCGGCGTTGCGCACCAAGATCGAGTAG
CTTATGTCGGATTCAACCACCCTGCCCTGCTAGAAGTGTTCTTTTCAACGAACCTCATTG
GGGCAACACCCGTGCTTGTTAACCCTCGCCTATCGGCAAACGAAATCGATTACATCATC
CAAGACAGCGGTGCGAGCATCGTGTTTTACGGAATCGACCTCATCGAGCACGCCACTT
ACCTCCAAGAACTCCATCCAGAGATCATCATGGTGGCCGTTGAAGGCGATGAGGGTCC
 
AGGTTTGCGTCGAAAAGCGCTTATTGAAGCGGCGAGCGACGCCGACATCGACCTAGAA
GTCAGCGATGATGACATCGCGCTGCTCATGTACACCTCCGGAACCACTGGCCGCCCAA
AGGGCGCCATGTTATCCCACCGAAACCTCTTCTTTAACTACTTCAATGCCCTGCTCAGC
CAGGAAATTGAACAAGGCGCGGTACTTTTATCCACTGCGCCGTTATTCCACATCGCGGG
CCTCAACATGACCACCATCCCGGTGATGATGAAGGGCGGAAAGGTGATCATCCACCGC
GAATTCCGGGCAGAGCACGTCCTCGACGAAATCGAACGCTCCAAGGTATCCGAATCCT
TCATGGTGCCAGCGATGATCGACATGTTGTCCAACCACCCATCATTTGCCGAACGCGAC
CTTTCTTCCCTTCGCGCCATCATGGTGGGCGGCTCTCCCCTTAGCGAACGTGCGCTGC
GAATCTGGCAAGGACGCGACGTAAAAATTGTCCAAGGCTTCGGCATGACAGAAACCGC
ACCGGGCGCCTGTATCCTCGAGGCAACAGACACAAGCACACACCTTGGAACCGCAGGT
CGAGCCCACTTCTTCACCGACATCAAACTAGTGGACCCGAAAACCGGCGAAGAAGTCC
CCACCGGAGAGGCCGGCGAAGTTCTCATCCGCGGACCACATGTGATGACCGGATACTG
GAACCGACCAGAAGACACCGCCAGCGCACTACAAAATGGCTGGTACCACTCCGGAGAT
ATCGCCATCAAAGATGAAGACGGCTACTACACCATCAAAGACCGCATCAAAGACATGTA
CATCTCCGGCGGCGAAAACATTTACCCCGCAGAAGTCGAACAAGCACTCCAGGAACTG
GAAGCAGTCCTTGATGCCGCAGTCATCGGAGTCCCCGATGAACGATGGGGCGAAACTG
GCATCGCCTTTGTCTCCATCCGAGAAAGCTACCTGACCAACCCACCAACCGGACCGGA
ACTACGAGAACTACTAGGCAGTGTCCTAGCCAGATACAAACTTCCACGAGAAATCCACA
TCATCGAAGAACTCCCCCGCAACGCCACAGGCAAAATCCAAAAGAACATCCTGCGAGA
CTTCACCATCCCCGTTTCATAA
SEQ ID NO: 27
   *benA*
   DNA
   Corynebacterium sp. 
ATGTCCACACCAGTTTCAAATTTGGCAAGCGTTCAGAAAACTCTGGACCATGCGCTTGA
GGACCGCCCTGAAGAGGGAATCGTCCGCGTCAACCGCAACATCTTCACTGACCCTGAG
ATCTTCGAGCTGGAGATGCGCCACATCTTCGAAGGCAACTGGATCTACCTGGCTCACGA
GTCCCAGATCCCTAACGCTGGCGACTACTTCACCACCTACATTGGCCGCCAGCCGATC
ATGATCACCCGTTCCAAGGACGGCACACTCAACTGCCTGATCAACGCGTGTTCTCACCG
TGGCGCCATGCTCTGCCGTCGCAAGACTGACAACCGCACCACCTTGACCTGCCCATTC
CACGGCTGGACATTCAGCAACGACGGCGCACTGCTCAAGGTCAAGGACGAAAAAGAAG
GCGCCTACCCAGAGAATTTCCGCACCGACGGCTCCCACGATCTGCGTCGCGTTCCTAA
GTTCGAGTCCTACCGTGGCTTCCTCTTCGGCTCCCTCAACGATGATGTCGTTTCTTTGG
AAGAGCACCTCGGCGACACCCGTACCGTCATTGACATGCTGGTTGACCAATCCCCAGA
AGGCCTCGAAGTACTGCGCGGATCCTCCACCTACACCTACGACGGCAACTGGAAGCTC
CAGACCGAAAACGGTGCAGACGGCTACCACGTTTCCTCCACCCACTGGAACTACGCTG
 
CAACCACCTCCCGCCGTGGCACTGGTGAATCCGCGAACGAAACCAAGGCAATGGACGC
TGGTACCTGGGGCAAGCAGGGTGGCGGATACTTCTCCTACCCTTACGGCCACATGCTG
CTGTGGATGTGGTGGGGCAACCCAGAAGACCGCCCACTGTTCGAGCGCCGCGACGAG
TTCAAGAAGGAATTCGGCGAAGAAAAGGGCGAGTTCATGGTTGGTGCTTCCCGCAATCT
GTGCCTCTACCCCAATGTTTACCTGATGGATCAGTTCTCCTCACAGATCCGCCACATCC
GCCCAATCTCAGTTGATCAGACCGAAGTCACCATCTACTGCATCGCACCTAAGGGCGAG
TCCGCGGAAGCACGTGCAAACCGCATCCGCCAGTACGAAGACTTCTTCAACGCAACGG
GCATGGCAACCCCAGATGACCTGGAGGAATTCCGCTCCTGCCAGAAGACCTACCAGGC
ATCTGCCTTCCCATGGAATGACATGACCCGCGGTTTGGGCCACCAGGTACAGGGACCA
AACGAGGTTGCCAAGGGCCTAGGCATGAACGAAGTTCTTTCCTCCGGAGCACGCACCG
AAGATGAAGGCCTCTACCCAATCCAGCACGGCTTCTGGCATGAACTCATGCAGGAGGC
TGTGAATAAGCAGAGCATCAAGGAAAAGGAATTGGCTGACGATACCGCTTCTTCCCTTG
CCACCGTAGCTGCAGCCAAAATCCGTGAGGAAGCAAAGGCAGCCGCGAAGTCCGACG
CTGGAGAGCCTCGCCGCCGTCGTCGCACCCGCGGTTAG
SEQ ID NO: 28
   *benB*
   DNA
   Corynebacterium sp. 
ATGTCTGAAATCACCCGCTCTGAGATCGAAGCTTTCCTCTACTACGAGGCTCGCCTGCT
CGATGACCGTAAATTTGAAGAATGGCTCGAATGCTACCGCGAGGACGCCGAGTTTTGG
ATGCCAGCCTGGGACGACAACGGTGAACTGACTGAAGATCCACAGTCTGAAATTTCCCT
CATCTACTACCCAAACCGTGGTGGACTTGAGGACCGCGTCTTCCGCATCCGCACCGAA
CGCTCCTCTGCAACTTCCCTACCTGAACCACGCACCGGCCACAGCACCACCAACGTGG
AAATCCTGGAGCGCCGCGACGGCGAAGTAGATATCCGCTTCAACTGGATCACCTTCTAC
TACCGTTACAACACCACCGACACCTACTTCGGCACCACGTTTATCACCCTTGATGTCAG
TGGTGAAACCCCGAAGATCGTCAAGAAGAAGGTCGTTTTGAAGAACGACTACATCCACC
ACATCGTCGATATTTACCACGTCTAG
SEQ ID NO: 29
   *benC*
   DNA
   Corynebacterium sp. 
ATGACTCACCAAGTTGCACTTGCCTTTGAAGACGGCATCACCCGATTCATCGACTGCGA
AGATGACCAAACTGTTGCAGATGCCGCCTACCAGGCACGCATCAACATTCCTTTCGACT
GCCGCGACGGCGCCTGCGGAACCTGCAAAGCGTTCTGCGAATCCGGCGACTTTGACG
AAGGCGACTACATCGACGACGCCCTGTCCGAAGATGAAGCAGCCGACGGCTACTGCCT
 
GCCTTGCCAGATGACCCCAAAGACCGACCTCATCTTGCAGATCGCCACCACCTCCGTG
CTGGCAAAGACCGGCGCATCCACTTTCGATGGCGAGTTGAAGGAGATCAATCACTTCTC
TGATTCCACCATCGGCATTGAGATCGAACTGGAAAACCGCCAAGATTTGGCGTTCCTCC
CTGGTCAATACATGAACATCCAGGTTCCAGGCAGCGACCAGACTCGTTCCTACTCTTTC
TCCTGCGCTCAAGATTCCGGCAACGTGCAGTTCCTGATCAAGGTAACCCCAGGTGGAC
TCATGACCACCTATCTCACCGATCACGCGAAGGTCGGCGACAAGCTCACCTTGACCGG
CCCGATGGGTTCCTTCTTCCTGCGTGAACCTGTCCGCCCGATCCTGCTGCTCGCCGGC
GGAACTGGACTTGCACCGATCTTGGCTATTTTGGAAAAGCTTTCCCGCGATGAGCTTCT
CGACGTCCCAATCCGCCTGGTTTACGGCGCGAACTTCACCCACGATCTGGTGGAATTG
GATCGACTTGATGCCTTCAAGGACAAGTTCGACTTCGATTACATCACCGTGCTTTCCGA
CAAGGACACCGAGCATCCACGCAAGGGCTACGTCCCAGCACACCTGACCGGCGAATAT
GAGCCAGATGAGGACACTGATGTGTACCTCTGCGGCCCTCCTCCAATGGTCGAGGCCG
TGCGCCAATTCCTGGGCACCCTGGAGCATCCTCCGCTGGACTTTTATTACGAGAAGTTC
ACTTCCGCCGCTGCCCCTGCTGCTGGTAAGCCAGAGATCACCGTGGAGACCAGCGAAG
TTGCAGAGGATTTCAACCTGGTCGAGGTGTCCACTCCAGGCATGTCTTCCGGCGAGGT
GCACTCTTCTGCAACCCAGCTGCAGGCCCGCATGGCTCTGGAGCTCGGCGCGCTGGA
GCTTGCGATCAACAAACTCGGCGAGCGCGACATCGAGCGATTCCGCAACTTGGCCGAC
ATCGCGAACTCCTTCATCGACGGCGATAAGTTTATCGACGCGGTGAAGTTCACCGAGG
CCAACGCCGATTTCCACGAGTTCCTCTTCCGCCGCGCAAACAACGAGGCGCTGCTTGC
GGCGTACCAGAACCTCCAGGTTGTTCAAGAAATGAACGCAACCCTTCCAGGCGCCGAG
TGGATTGATCCGGCAATTGCCACCGAGCACTTGGCGCTTGTCGACGCCGTCTCCCAGA
ATGATCTCGAGACCGCGAGAACAATCATTCGTGAACACGCGGAGCACGGCATTGACAC
TATGGTTAAGGCCCTCGAGAAATGA
SEQ ID NO: 30
   *benD*
   DNA
   Corynebacterium sp. 
ATGAGCGCGCCAGTAGGACAAGGCATCGAAGGTCGCCCCACTCACGTCACCCCGGAA
CGCTTCTTTGGCCAGGGTGTTGTGGTTACCGGAGCGGCTCAAGGCATCGGCATGGCAG
TGGCACACCGCATCGCATATGAGGATGGCAACCTAGTGTTGGTGGACCGTTCCCCGCT
GGTGCATGAAGTTGCCGAAGAGCTGCGTAAAGCAGGCGCCGGAACGGTGGATTCTTTC
ATCGCCGATCTGGAAACCTTTGAAGGCGCAACTGATGCTTTGGAATTTGCCGGGCAGAA
GTTGAAGAACCTTGATGTGGTGATCAACAACGTTGGTGGCACCATCTGGGCGAAGCCTT
ATCAGGAATACTCTGAGGAAGAGATCCGCAAGGAAATCAACCGAAGCCTCTTCCCCACC
TTGTGGATGTGCCGGGCCGCGTTGCCAATTCTGATCGGAAATGGTGGTGGAACGATCG
TTAATGTGTCCTCCATCGCGACCGGTGGTATCAACCGTGTTCCTTATGCTGCAGCAAAG
 
GGTGGCGTCAACGGCATTGTTTCTGCCATGGCCCGCGAAGCTGCACCGCATAATGTGC
GCGTGGTGGCAACGGCTCCCGGTGGCACGCTCGCTCCGGAACGTGCCGTCAAACGAG
GCCCTGGGCCAGAGGGCGAATTGGAAGAAAAGTGGTATCAGCAAATCGTTGATCAAAC
CATTGATTCCAGTTTGATGAAGCGCTACGGCACCCTAGAGGAGCAGGTCGCGCCGATC
TGTTTCCTCGCTTCTGAGGAAGCTTCCTACATCACTGGATCAGTCATGCCAGTCGGTGG
AGGCGACCAGGGATAA
SEQ ID NO: 31
   citrate synthase (*gltA*)
   DNA
   Corynebacterium sp.

ATGTTTGAAAGGGATATCGTGGCTACTGATAACAACAAGGCTGTCCTGCACTACCCCGG
TGGCGAGTTCGAAATGGACATCATCGAGGCTTCTGAGGGTAACAACGGTGTTGTCCTG
GGCAAGATGCTGTCTGAGACTGGACTGATCACTTTTGACCCAGGTTATGTGAGCACTGG
CTCCACCGAGTCGAAGATCACCTACATCGATGGCGATGCGGGAATCCTGCGTTACCGC
GGCTATGACATCGCTGATCTGGCTGAGAATGCCACCTTCAACGAGGTTTCTTACCTACT
TATCAACGGTGAGCTACCAACCCCAGATGAGCTTCACAAGTTTAACGACGAGATTCGCC
ACCACACCCTTCTGGACGAGGACTTCAAGTCCCAGTTCAACGTGTTCCCACGCGACGCT
CACCCAATGGCAACCTTGGCTTCCTCGGTTAACATTTTGTCTACCTACTACCAGGACCA
GCTGAACCCACTCGATGAGGCACAGCTTGATAAGGCAACCGTTCGCCTCATGGCAAAG
GTTCCAATGCTGGCTGCGTACGCACACCGCGCACGCAAGGGTGCTCCTTACATGTACC
CAGACAACTCCCTCAATGCGCGTGAGAACTTCCTGCGCATGATGTTCGGTTACCCAACC
GAGCCATACGAGATCGACCCAATCATGGTCAAGGCTCTGGACAAGCTGCTCATCCTGC
ACGCTGACCACGAGCAGAACTGCTCCACCTCCACCGTTCGTATGATCGGTTCCGCACA
GGCCAACATGTTTGTCTCCATCGCTGGTGGCATCAACGCTCTGTCCGGCCCACTGCAC
GGTGGCGCAAACCAGGCTGTTCTGGAGATGCTCGAAGACATCAAGAGCAACCACGGTG
GCGACGCAACCGAGTTCATGAACAAGGTCAAGAACAAGGAAGACGGCGTCCGCCTCAT
GGGCTTCGGACACCGCGTTTACAAGAACTACGATCCACGTGCAGCAATCGTCAAGGAG
ACCGCACACGAGATCCTCGAGCACCTCGGTGGCGACGATCTTCTGGATCTGGCAATCA
AGCTGGAAGAAATTGCACTGGCTGATGATTACTTCATCTCCCGCAAGCTCTACCCGAAC
GTAGACTTCTACACCGGCCTGATCTACCGCGCAATGGGCTTCCCAACTGACTTCTTCAC
CGTATTGTTCGCAATCGGTCGTCTGCCAGGATGGATCGCTCACTACCGCGAGCAGCTC
GGTGCAGCAGGCAACAAGATCAACCGCCCACGCCAGGTCTACACCGGCAACGAATCCC
GCAAGTTGGTTCCTCGCGAGGAGCGCTAA

### Figures

The Figures show:
- **Figure 1**: **Curcumin production by engineered C. *glutamicum* strains from ferulate during batch cultivation in shake flaks**
Cultivation profiles in minimal medium with 10 g L⁻¹ of glucose (55 m) and 5 mM ferulate of CUR-6, of the wild type derivative expressing dcs and curs1 genes from *C*. *longa* (**A**); and CUR-4_nat, a phdBCDE-deficient strain expressing the dcs and curs1 genes from C. longa (**B**). Increase of biomass (g L⁻¹ of CDW), consumption of glucose (mM) and ferulate (mM), and formation of curcumin (mg L⁻¹) over time are displayed. The data comprise mean values and standard deviations from three biological replicates.
- **Figure 2**: **Curcumin production by engineered *C. glutamicum* CUR-8 from ferulate during batch cultivation in shake flaks**
The data displays cultivation profile in minimal medium with 10 g L⁻¹ of glucose (55 m) and 5 mM ferulate of CUR-8 strain expressing the dcs and curs1 genes from C. longa, with deletion of phdRBCDE genes. Increase of biomass (g L⁻¹ of CDW), consumption of glucose (mM) and ferulate (mM), and formation of curcumin (mg L⁻¹) over time are displayed. The data comprise mean values and standard deviations from three biological replicates.

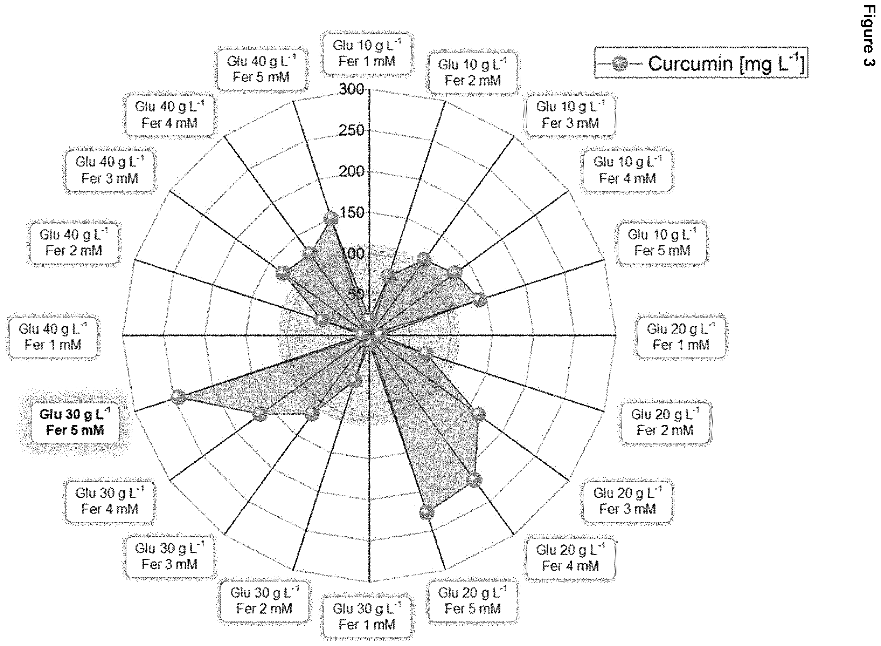

- **Figure 3**: **Optimization of growth and biotransformation substrate concentration for curcumin production by genetically engineered *C. glutamicum***
The CUR-8 strain was cultured in minimal medium with varying starting concentrations of glucose (10, 20, 30, and 40 g L⁻¹) and ferulate (1, 2, 3, 4, and 5 mM) in glass test tubes. The curcumin titres were compared based on the end-point sampling and extraction after 72 hours of cultivation. The data represent average values from three biological replicates.
- **Figure 4**: **High-level curcumin production by engineered *C. glutamicum* strains from ferulate during batch cultivation in shake**
The data displays cultivation profiles in minimal medium with 30 g L-1 of glucose (55 m) and 5 mM ferulate of CUR-8, CUR-12, and CUR-15 (A, C, E, respectively); and their respective dynamics of intracellular CoA-thioester concentrations (B, D, F). Increase of biomass (g L-1 of CDW), consumption of glucose (mM) and ferulate (mM), and formation of curcumin (mg L-1) over time are displayed. Samples for the absolute quantification of CoA-thioesters were taken at the beginning, in the middle, and at the end of the exponential growth, here indicated as phase II. The data comprise mean values and standard deviations from three biological replicates.
- **Figure 5**: **Bioprocess optimization of curcumin production in stirred-tank bioreactors by metabolically engineered *C. glutamicum* CUR-8**
The data represents cultivation profiles under high oxygen, low pH (20% dO₂; pH=6.8) (**A**); oxygen limiting, high pH (2.5% dO₂; pH=6.8) (**B**); and oxygen limiting, low pH (2.5% dO₂; pH=6.0) (**C**) conditions. The arrows indicate feeding with ferulate. The data comprises single biological replicates.
- **Figure 6**: **Fed-batch production of curcumin by metabolically engineered *C. glutamicum* CUR-8**
Data represents growth and production profiles of the cultures from low cell density (A) and high cell density (B) inoculums. After depletion of initially provided glucose and ferulate, additional pulses were added manually from concentrated stock solutions. First ferulate feed is indicated by an arrow. Concentrations of substrates were monitored by simultaneous HPLC measurements. Data comprises mean values from two biological replicates for the high cell density inoculum fermentation, whereas low cell density inoculum approach was carried out in one biological replicate.

The present invention may also be characterized by the following items:
(1) A gram-positive bacterial host cell, which naturally has a phenylpropanoid pathway, wherein said bacterial host cell is genetically engineered to express curcuminoid biosynthetic genes, thereby being capable of producing curcumin.
(2) The bacterial host cell of item 1, wherein said curcuminoid biosynthetic genes encode one or more selected from the group consisting of phenylalanine hydroxylase (PAH), phenylalanine ammonia lyase (PAL), tyrosine ammonia lyase (TAL), Cinnamate 4-hydroxylase (C4H), 4-coumarate 3-hydroxylase (C3H), Caffeic acid O-methlytransferase (COMT), Caffeoyl-CoA O-methyltransferase (CCoAOMT), 4-coumarate-CoA ligase (4CL), diketide-CoA synthase (DCS), curcuminoid synthase (CUS), curcumin synthase 1 (CURS1), curcumin synthase 2 (CURS2), and curcumin synthase 3 (CURS3).
(3) The bacterial host cell of any one of the preceding items, wherein said curcuminoid biosynthetic genes are codon optimized.
(4) The bacterial host cell of item 2 or 3, wherein said
   a) curcuminoid synthase (CUS) has a polypeptide sequence at least about 70% similar to the polypeptide sequence shown in SEQ ID: 1 or 2,
   b) diketide-CoA synthase (DCS) has a polypeptide sequence at least about 70% similar to the polypeptide sequence shown in SEQ ID: 3 or 4,
   c) curcumin synthase 1 (CURS1) has a polypeptide sequence at least about 70% similar to the polypeptide sequence shown in SEQ ID: 5 or 6,
   d) curcumin synthase 2 (CURS2) has a polypeptide sequence at least about 70% similar to the polypeptide sequence shown in SEQ ID: 7,
   e) curcumin synthase 3 (CURS3) has a polypeptide sequence at least about 70% similar to the polypeptide sequence shown in SEQ ID: 8, and/or
   f) 4-coumarate-CoA ligase (4CL) has a polypeptide sequence at least about 70% similar to the polypeptide sequence shown in SEQ ID: 9 or 10.
(5) The bacterial host cell of any one of the preceding items, said curcuminoid biosynthetic genes expressed in the corynebacterium (e.g., *C*. *glutamicum)* host cell encode
   a) a polypeptide sequence at least about 70% similar to the polypeptide sequence shown in SEQ ID: 3 or 4,
   b) a polypeptide sequence at least about 70% similar to the polypeptide sequence shown in SEQ ID: 5 or 6, and
   c) optionally a polypeptide sequence at least about 70% similar to the polypeptide sequence shown in SEQ ID: 9 or 10.
(6) The bacterial host cell of any one of the preceding items, wherein said curcuminoid biosynthetic genes are under the control of a homologous or heterologous promoter, preferably the tuf promoter.
(7) The bacterial host cell of any one of the preceding items, wherein said biosynthetic genes are extrachromosomal expressed, preferably by a plasmid, more preferably a low copy number plasmid.
(8) The bacterial host cell of any one of the preceding items, wherein said bacterial host cell is genetically engineered to be reduced in catabolizing phenylpropanoids into further metabolic products for energy production.
(9) The bacterial host cell of item 8, wherein in said bacterial host cell *phdBCDE* genes encoding 3-hydroxyacyl-CoA dehydrogenase, metal-dependent hydrolase, acyl-CoA dehydrogenase, and enoyl-CoA hydratase, respectively, are inactivated.
(10) The bacterial host cell of any one of the preceding items, wherein in said bacterial host cell the functional gene encoding PhdR is inactivated.
(11) The bacterial host cell of any one of the preceding items, wherein said bacterial host cell is genetically engineered to have increased expression of the *phdA* gene encoding long-chain fatty acid-CoA ligase.
(12) The bacterial host cell of any one of the preceding items, wherein in said bacterial host cell *benABCD* genes encoding encoding benzoate 1,2-dioxygenase large subunit, benzoate 1,2-dioxygenase small subunit, benzoate 1,2-dioxygenase electron transfer component, 1,6-dihydroxycyclohexa-2,4-diene-1-carboxylate dehydrogenase, respectively, are inactivated.
(13) The bacterial host cell of any one of the preceding items, wherein said bacterial host cell is genetically engineered to have attenuated expression of the gene encoding citrate synthase.
(14) The bacterial host cell of item 13, wherein said bacterial host cell is genetically engineered to have GTG as start codon of the gene encoding citrate synthase.
(15) The bacterial host cell of any one of the preceding items, which is a Corynebacterium host cell, Bacillus host cell, Actinobacter host cell, or Streptomycetes host cell.
(16) The bacterial host cell of item 15, wherein said Corynebacterium host cell is a Corynebacterium glutamicum host cell.
(17) The bacterial host cell of any one of the preceding items, which is cultured in the presence of ferulate, preferably up to about 5 mM ferulate.
(18) The bacterial host cell of any one of the preceding items, which is cultured in the presence of glucose, preferably about 30 g/L glucose.
(19) The bacterial host cell of any one of the preceding items, which is cultured using fed-batch fermentation.
(20) A method for producing curcumin, comprising culturing the bacterial host cell of any one of items 1 to 19 under suitable conditions in liquid medium and harvesting curcumin.
(21) The method of item 20, wherein said liquid medium comprises ferulate, preferably up to 5 mM ferulate.
(22) The method of item 20 or 21, wherein said liquid medium comprises glucose, preferably up to 30 g/L glucose.
(23) The method of any one of items 20 to 22, wherein bacterial host cells are cultivated at a pH of about 6 to 7.
(24) The method of any one of items 20 to 23, wherein bacterial host cells are cultivated at an O₂ concentration of about 10-22%.
(25) The method of any one of items 20 to 24, wherein culturing comprises a two-phase fermentation.
(26) Use of the bacterial host cell of any one of items 1 to 19 for the production of curcumin.

The present invention is further illustrated by the following examples. Yet, the examples and specific embodiments described therein must not be construed as limiting the invention to such specific embodiments.

### Examples

### 1. Materials & Methods

### 1. Microorganisms and plasmids

*C*. *glutamicum* ATCC 13032 (DSM 20300) was obtained from the Deutsche Stammsammlung für Mikroorganismen und Zellkulturen (DSMZ, Braunschweig, Germany). E. coli DH10B (Life GmbH, Darmstadt, Germany) and NM522 (Invitrogen, Carlsbad, CA, USA) were used for the propagation of plasmids. *E. coli* NM522 co-expressed the plasmid pTC expressing a C. glutamicum-specific DNA-methyltransferase for methylation of plasmid DNA (Kind et al., Metab Eng (2010). 12: 341-351). For plasmid-based expression of genes, the episomal vector pClik 5α plasmid was used (Buschke et al., Biotechnol J (2011), 6: 306-317). For genome-based modification, including the deletion and integration of genes, the integrative pClik int sacB plasmid was applied (Becker et al., App Environ Microbiol (2005), 71: 8587-8596; Kind et al., Metab Eng (2010). 12: 341-351). All strains and plasmids, including newly created derivatives, were maintained in 30% glycerol at -80 °C. They are listed in Table 1.

**Table 1. Bacterial strains and plasmids used herein.**

| **Strain or plasmid** | | **Characteristics** | **Reference** |
|---|---|---|---|
| ***C*. *glutamicum*** | | | |
| | **ATCC 13032** | Wild type strain | American Type Strain and Culture Collection, Manassas, VA, USA |
| | **CUR-1** | ATCC 13032 derivative with deletion of *phdBCDE* | This application |
| | **CUR-2_nat** | CUR-1 harbouring the pClik 5α MCS_OsCUS_nat | This application |
| | **CUR-2_co** | CUR-1 harbouring the pClik 5α MCS_OsCUS_co | This application |
| | **CUR-3_nat** | CUR-1 harbouring the pClik 5α MCS_SbCUS_nat | This application |
| | **CUR-3_co** | CUR-1 harbouring the pClik 5α MCS_SbCUS_co | This application |
| | **CUR-4_nat** | CUR-1 harbouring the pClik 5α MCS_CIDCS_CURS1_nat | This application |
| | **CUR-4_co** | CUR-1 harbouring the pClik 5α MCS_CIDCS_CURS1_co | This application |
| | **CUR-5_nat** | CUR-1 harbouring the pClik 5α MCS_At_4CL_CIDCS_CURS1_nat | This application |
| | **CUR-5_co** | CUR-1 harbouring the pClik 5α MCS_At_4CL_CIDCS_CURS1_co | This application |
| | **CUR-6** | ATCC13032 harbouring the pClik 5α MCS_CIDCS_CURS1_nat | This application |
| | **CUR-7** | CUR-1 derivative with deletion *of phdR* | This application |
| | **CUR-8** | CUR-7 harbouring pClik 5α MCS_CIDCS_CURS1_nat | This application |
| | **CUR-9** | ATCC 13032 derivative with the deletion of the *phdRBCDE* and exchange of native promoter of *phdAT* with the *tuf* promoter | This application |
| | **CUR-10** | CUR-7 derivative with the deletion of *phdR* and replacement of native promoter of *phdA* with the *tuf* promoter | This application |
| | **CUR-11** | CUR-6 derivative with ATG start codon of citrate synthase replaced with GTG | This application |
| | **CUR-12** | CUR-11 derivative harbouring pClik 5α MCS_CIDCS_CURS1_nat | This application |
| | **CUR-13** | CUR-7 derivative with accBC *fasO* site mutated | This application |
| | **CUR-14** | CUR-13 derivative with accD1fasO site mutated | This application |
| | **CUR-15** | CUR-14 derivative harbouring pClik 5α MCS_CIDCS_CURS1_nat | This application |

| ***E. coli*** | | | |
|---|---|---|---|
| | **DH10B** | F⁻ mcrA Δ(*mrr-hsd*RMS-*mcr*BC) Φ80d*lac*ZΔM15 Δ*l*acX74 *end*A1 *rec*A1 *deo*R Δ(*ara,leu*)7697 *ara*D139 *gal*U *gal*k *nupG rps*L λ⁻ | Invitrogen, Carlsbad, CA, USA |
| | **NM522** | *sup*E, *thi,* Δ(*lac*-*pro*AB), *hsd*5 (r⁻, m⁻) | Invitrogen, Carlsbad, CA, USA |

| **Plasmids** | | | |
|---|---|---|---|
| | **pClik 5α MCS** | Episomal transformation vector *for C. glutamicum* with *E*. *coli* and *C*. *glutamicum* MCS and ORI, and Kan^{R} and *sacB* as selection markers | (Becker et al., Metab Eng (2011), 13: 159-168 |
| | **pClik 5α MCS_CIDCS_CURS1_nat** | pClik 5α MCS with native *C. longa* DCS and CURS1 genes | GenScript Biotech, Rijswijk, Netherlands |
| | **pClik 5α MCS_CIDCS_CURS1_co** | pClik 5α MCS with *C. longa* DCS and CURS1 genes codon-optimized for *C*. *glutamicum* | GenScript Biotech, Rijswijk, Netherlands |
| | **pClik 5α** | pClik 5α MCS with native *Arabidopsis* | GenScript Biotech, |
| | **MCS_At4CL_CIDCS_CURS1_nat** | *thaliana* 4CL and *C. longa* DCS and CURS1 genes | Rijswijk, Netherlands |
| | **pClik 5α** | pClik 5α MCS with *A*. *thaliana* 4CL and | GenScript Biotech, |
| | **MCS_At4CL_CIDCS_CURS1_nat** | *C. longa* DCS and CURS1 genes codon-optimized for *C*. *glutamicum* | Rijswijk, Netherlands |
| | **pClik 5α MCS_OsCUS_nat** | pClik 5α MCS with native *Oryza sativa* CUS gene | GenScript Biotech, Rijswijk, Netherlands |
| | **pClik 5α MCS_OsCUS_co** | pClik 5α MCS with *O*. *sativa* CUS gene codon-optimized for *C*. *glutamicum* | GenScript Biotech, Rijswijk, Netherlands |
| | **pClik 5α MCS_SbCUS_nat** | pClik 5α MCS with native *Sorghum bicolor* putative curcumin synthase | GenScript Biotech, Rijswijk, Netherlands |
| | **pClik 5α MCS_SbCUS_co** | pClik 5α MCS with *S*. *bicolor* putative curcumin synthase codon-optimized for *C. glutamicum* | GenScript Biotech, Rijswijk, Netherlands |
| | **pClik int *sacB*** | Integrative transformation vector for *C*. *glutamicum* with *E*. *coli* MCS and ORI, and Kan^{R} and *sacB* as selection markers | (Becker et al., Metab Eng (2011), 13: 159-168) |
| | **pClik int *sacB_*Δ*phdBCDE*** | pClik int *sacB* for the deletion of the *C*. *glutamicum phdBCDE* genes | This application |
| | **pClik int *sacB_*Δ*phdR*** | pClik int *sacB* for the deletion of the *C*. *glutamicum phdR* gene | This application |
| | **pClik int *sacB* Δ*phdRBCDE_phdAT_{Ptuf}*** | pClik int *sacB* for the deletion of the *C*. *glutamicum phdR*BCDE genes and the replacement of the native *C. glutamicum phdAT* promoter with the *C. glutamicum* structural *tuf* promoter | This application |
| | **pClik int *sacB_phdA_{Ptuf}*** | pClik int *sacB* for the deletion of the *C*. *glutamicum* phdR gene and replacement of the native *C. glutamicum phdA* promoter with the *C. glutamicum* structural *tuf* promoter and reversed orientation of the *phdA* gene | This application |
| | **pClik int *sacB_cs*_{GTG}** | pClik int *sacB* for the exchange of the *C*. *glutamicum* citrate synthase ATG start codon with GTG | * |
| | **pClik int *sacB_accBC*_fasO_mut** | pClik int *sacB* for the mutation of the *fasO* site of the *C. glutamicum accBC* gene | This application |
| | **pClik int *sacB_accD1*_fasO_mut** | pClik int *sacB* for the mutation of the *fasO* site of the C. glutamicum *accD1* gene | This application |

| | | | |
|---|---|---|---|
| *The plasmid was kindly provided from the in-house strain collection at the Institute for Systems Biotechnology (University of Saarland, Saarbrücken, Germany). | | | |

### 2. Genetic engineering application

Cloning strategies were designed using the SnapGene software (GSL, Biotech, LLC, Insightful Science, Chicago, IL, USA). DNA fragments were amplified by PCR (2x Phusion High-Fidelity PCR Master Mix with GC Buffer, Thermo Fischer Scientific, Waltham, MA, USA) using specific primers. DNA with heterologous genes for curcumin expression was synthetized in either native or codon-optimized form. The cloning vector was linearized using Smal restriction digestion (FastDigest, Thermo Fischer Scientific). The linearized plasmid and the DNA fragments of interests were purified (Wizard SV Gel, PCR Clean-UP System, Promega, Mannheim, Germany) and then assembled in vitro (Rohles et al., Microbial Cell Factories (2016), 15: 154).

The assembled plasmids were transformed into E. coli DH10B using heat shock and verified for correctness using analytical PCR (2x Phire Hot Start II DNA-polymerase, Thermo Fischer Scientific), isolated (QIAprep Spin Miniprep Kit, Qiagen, Hilden, Germany), and verified by sequencing (Azenta Life Sciences, Genewiz Germany, Leipzig, Germany) (Becker et al., 2010). Integrative plasmids were additionally transformed into E. coli NM522 pTC for the generation of the C. glutamicum-specific methylation pattern (Kind et al., 2010). Subsequently, the plasmid were transformed into C. glutamicum by electroporation, and the desired mutants were obtained by homologous recombination as described earlier (Becker et al., Eng Life Sci (2010), 10: 430-438; Kind et al., Metab Eng (2010), 12: 341-351; Rohles et al., Metab Eng (2022), 73: 168-181). All recombinant strains were verified by PCR and sequencing.

### 3. Media

For cell propagation during cloning and pre-culturing for curcumin production, the cells were grown in complex BHI medium (37 g L-1, brain heart infusion, Becton Dickinson, Franklin Lakes, NJ, USA). For plate cultures, the BHI medium was solidified by the addition of 15 g L-1 of agar (Becton Dickinson). For plasmid maintenance, growth media were supplemented with kanamycin (25- 50 µg mL-1) and tetracycline (12.5 µg mL-1). In addition, a glucose-based minimal medium was used for the cultivation of C. glutamicum. The medium contained per litre: 30 g of glucose, 15 g of (NH4)2SO4, 1 g of NaCl, 0.2 g of MgSO4·7H2O, 0.055 g of CaCl2, 34.8 g of K2HPO4, 27.2 g of KH2PO4, 2 mg of FeCl3·6H2O, 2 mg of MnSO4·H2O, 0.5 mg of ZnSO4·H2O, 0.2 mg CuCl2·2H2O, 0.2 mg of Na2B4O7·10H2O, 0.1 mg (NH4)6Mo7O24·4H2O, 20 mg FeSO4·7H2O, 1 mg of thiamine-HCl, 1 mg of Ca-pantothenate, 0.5 mg of biotin and 30 mg of 3,4-dihydroxybenzoic acid (Rohles et al., Metab Eng (2022), 73: 168-181; Weiland et al., Metab Eng (2023), 75: 153-169). In addition, ferulate was added as substrate for curcumin production, as given below.

### 4. Batch cultivation in shake flasks

*C*. *glutamicum* was incubated in an orbital shaker (Multitron, Infors AG, Bottmingen, Switzerland) at 30 °C, 230 rpm, and 80% humidity, using non-baffled shake flasks (10% filling volume). Regarding the light-sensitivity of curcumin (Mondal et al., Biology (2016), 158: 212-218), the glass window of the incubator was covered with aluminum foil to enable cultivation in the dark. The cultivation involved two subsequent pre-cultures, followed by the main culture. The first pre-culture (10 mL BHI medium) was inoculated in with a single colony from a BHI-agar plate, pre-grown for 48 h at 30 °C and incubated 12 h. Then, the cells were harvested (6,000 × g, 20 °C, 3 min), washed twice with minimal glucose medium, and used to inoculate the second pre-culture (50 mL minimal glucose medium) which was grown for another 7 h. Finally, the main culture in minimal glucose medium (50 mL) was inoculated from the second pre-culture as described above. The main culture was inoculated to an initial optical density (OD660) of 1.0. For the production of curcumin, ferulate was added only to the main culture. All experiments were conducted in biological triplicate.

### 5. Screening of curcumin producers and medium tests in reagent tubes

Small scale screening experiments of curcumin-producing strains and tests for medium optimization were performed in glass reagent tubes (25 mL) filled with 5 mL of inoculated main culture medium (see above). Different combinations of glucose (10, 20, 30, and 40 g L-1, corresponding to 55, 110, 165, and 220 mM) and ferulate (1, 2, 3, 4, and 5 mM) were tested.

### 6. Batch fermentation in stirred tank bioreactor

For selection of appropriate aeration and pH, curcumin production was carried out in batch mode in 1 L stirred-tank reactors (SR0700DLS, DASGIP AG, Jülich, Germany) under different pH and oxygen saturation. To start the process, a BHI pre-culture (100 mL supplemented with 25 µg mL-1 kanamycin) was inoculated and cultivated at 30 °C. The cells were harvested (5,000 × g, 5 min, room temperature) and used to inoculate a second pre-culture in a chemically defined medium containing 25 µg mL-1 kanamycin. The second pre-culture was grown for 10 hours. The exponentially growing cells were harvested (5,000 × g, 5 min, room temperature) and resuspended in batch medium. The inoculum was injected through the septum of the bioreactor, and inoculated in 300 mL of batch medium to OD660 = 2.0. Fermentation was carried out under three process conditions: 20% dO2, pH = 6.8; 2.5% dO2, pH = 6.8; and 2.5% dO2, pH = 6.0. The batch medium had the composition as described herein, with increased glucose concentration to 90 g L-1, and 2.5 g L-1 yeast extract. The pH was monitored online using a pH electrode (Mettler Toledo 405-DPAS-SC-K8S/225, Mettler Toledo, Giessen, Germany). 6 M NaOH and 6 M HCl were used to maintain pH value of 6.8 (MP8 pump system, Eppendorf, Hamburg, Germany). Oxygen was also monitored online (Hamilton, Höchst, Germany), and saturation of 2.5% and 20% was maintained by automatic adjustments of agitation and gas inflow. DASGIP control software (DASGIP AG) was applied for data acquisition and process control. Ferulate feed phase was initiated manually by injecting 15 mM ferulate from a 500 mM stock after 4 h of growth.

### 7. Fed-batch fermentation in stirred tank reactors

The selected curcumin producer was further evaluated in stirred tank 1 L bioreactors (SR0700DLS, DASGIP AG) in a fed-batch mode. The preparation of first and second pre-cultures was carried out as described herein. The initial batch medium had composition as described herein, whereby glucose was supplemented in concentration of 30 and 20 g L-1, and kanamycin was omitted (small scale test tube test revealed no statistical difference in curcumin production with and without supplementation of kanamycin. Two different sizes of inoculum were tested (OD660 = 2 and OD660 = 8, corresponding to low and high cell density inoculum, respectively). The process was maintained at 30 °C, 20% dO2 (dissolved oxygen), and pH = 6.8. Ferulate feed phase was initiated manually by injecting 5 mM ferulate from a 500 mM stock after 4 h of batch phase. Ferulate and glucose concentrations were monitored using HPLC (Agilent Technologies). Glucose and ferulate feeds were given manually upon exhaustion of the provided substrates. Thereby glucose feed was given from a 550 mM stock of glucose monohydrate solution.

### 8. Quantification of cell concentration

The cell concentration was inferred from spectrophotometric analysis of the optical density at 660 nm (OD660). A previously established correlation was used to convert OD-readings into cell dry weight (CDW) levels: CDW [g L-1] = 0.32 × OD660 (Becker et al., Appl Environ Microbiol (2009), 75: 7866-7869).

### 9. Quantification of substrates and products

Glucose and lactate were quantified by isocratic HPLC (Agilent 1260 Infinity Series, Agilent Technologies, Waldbronn, Germany). The analytes were separated using a reversed phase column (Aminex HPX-87H, 300 × 7.8 mm, Bio-Rad, Hercules, CA, USA) at 40 °C as the stationary phase, and 3.5 mM H2SO4 at a flow rate of 0.50 mL min-1 as the mobile phase. Detection was based on refractive index measurement at 55 °C (Agilent 1260 RID, G1362A, Agilent Technologies). External standards were applied for quantification.

Ferulate was quantified by gradient HPLC (Agilent 1260 Infinity Series, Agilent Technologies), using separation done at 25°C (Nucleodur EC 4/3 C18 Isis, 3 µm × 4 mm, Nucleodur EC 100/3 C18 Isis, 3 µm × 100 mm, Macherey-Nagel, Düren, Germany) and following gradient of 0.025% H3PO4 (eluent A) and acetonitrile (eluent B) at a continuous flow rate of 1.0 mL min-1: 0 - 13.8 min, 99 - 67% A; 13.8 - 14.3 min, 67 - 0% A; 14.3 - 17.3 min, 0% A; 17.3 - 17.8, 0 -99% A; 17.8 - 24.3 min, 99% A. Ferulate was detected by UV absorption at 325 nm (Agilent 1260 DAD, G4212B, Agilent Technologies) and quantified using external standards.

Curcumin was quantified by gradient HPLC (Agilent 1260 Infinity Series, Agilent Technologies). For this purpose, 50 to 500 µL of cell broth was acidified to pH 3.0 (6 M HCl), subsequently mixed with two volumes of ethyl acetate, followed by phase separation (15,000 × g, 4°C, 2 min). The organic phase was transferred into a fresh tube. The extraction of the sample was repeated until the obtained organic phase appeared no longer yellow. All extracts were combined, dried under a stream of nitrogen, dissolved in 200 µL of acetonitrile, and analyzed by HPLC. In short, 10 µL sample was injected and separated on reversed phase column (Nucleodur EC 100/3 C18 Isis, 3.5 µm × 100 mm, Macherey Nagel) at 25 °C, using a gradient of 0.025% H3PO4 (eluent A) and acetonitrile (eluent B) at a constant flow rate of 1.0 mL min-1: 0 - 22.0 min, 100-0% A; 22 - 22.5 min, 27.4 - 72.6% A; 22.5 - 24.5 min, 0-100% A; 24.5 - 27.0 min, 100% A. Curcumin was detected by UV absorption at 426 nm (Agilent 1290 DAD, G4212A, Agilent Technologies). External standards were applied for quantification.

### 10. Quantification of intracellular CoA-thioesters

The protocol for the quantification of intracellular CoA-thioesters was adapted from previous application (Gläser et al., Microbial Cell Factories (2020), 19: 160). Briefly, culture samples (10 mg CDW) were quenched with an adequate amount of quenching and extraction buffer (25 mM formic acid, 95% acetonitrile, -20°C). Extraction of the intracellular CoA-thioesters was achieved through mixing and cooling steps on ice for 10 minutes, followed by centrifugation (15,000 × g, 4 °C, 10 min) and collection of the obtained supernatant into 10 mL ice-cold deionized water. The cell pellets were washed two times with 8 mL of ice-cold water, and all supernatants were combined afterwards. The extract was frozen in liquid nitrogen, freeze-dried, and subsequently resuspended in 500 µL of pre-cooled buffer (25 mM ammonium formate, 2% MeOH, pH 5.6, 4 °C). Prior to analysis, the buffered extract was clarified from debris by centrifugation (15,000 × g, 4 °C, 10 minutes).

The analysis was carried out using a triple quadrupole MS (QTRAP 6500+, AB Sciex, Darmstadt, Germany), coupled to an LC system (Agilent 1290 Infinity System, Agilent). The analytes of interest were separated on a reversed phase column (Gemini, C18, 100 mm × 2.1 mm, 1.5 µm, 110 Å, Phenomenex, Aschaffenburg, Germany) at 40 °C and a flow rate of 0.60 mL min 1, using a gradient of eluent A (50 mM formic acid, pH 8.1) and eluent B (methanol). The fraction of eluent B was as follows: 0-12 min, 0-15%; 12-16 min, 15-100%; 16-18 min, 100%; 18-20 min, 100-0%; 20-25 min, 0%.

For absolute quantification, 13C-labelled cell extracts were used as internal standard (Gläser et al., *loc. cit.*)*.* For this purpose, *C*. *glutamicum* ATCC 13032 was grown on 99% [13C6] glucose (Omicron, Biochemicals, South Bend, United States). To obtain fully 13C-enriched cell extracts, the inoculum size for both the second pre-culture, as well as for the main culture, was kept below 1% of the finally harvested cell concentration (Wittmann, Microbial Cell Factories (2007), 6: 6). Extraction of the 13C CoA-thioesters was conducted as described above. The concentration of individual 13C CoA-thioesters was determined using synthetic standards. For subsequent absolute quantification, an appropriate amount of 13C extract was added to the samples during the initial quenching step (Gläser et al., *loc. cit.*)*.*

### II. Constructs and Results

### 1. Development of C. glutamicum chassis for curcumin production

The *phdBCDE* genes, coding for 3-hydroxyacyl-CoA dehydrogenase, metal-dependent hydrolase, acyl-CoA dehydrogenase, and enoyl-CoA hydratase, were tested whether they represent a bottleneck for efficient biotransformation of phenylpropanoids to aromatic plant polyphenols.

In an effort to delete *phdBCDE* genes at once, 485 bp sequence upstream from phdB gene and 608 bp sequence downstream from phdE gene were cloned into the non-replicative plasmid pClik_IntsacB_ΔphdBCDE. Methylated plasmid was used to transform *C*. *glutamicum* ATCC 13032 to kanamycin resistance, indicating successful first recombination and integration of the plasmid into the wild type genome. For markerless deletion and removal of the vector backbone, one positive merodiploid underwent the second recombination step. From approximately 50 clones analysed by PCR, 12 appeared to have phdBCDE genes deleted, indicated by truncated PCR product (289 bp, compared to 3841 bp in the wild type. One selected clone was further confirmed by sequencing, and designated as C. *glutamicum* CUR-1.

In order to assess the effects of genomic removal of the phdBCDE genes, the CUR-1 chassis strain was cultivated on minimal media agar plates supplemented individually with 5 mM p-coumarate, caffeate, and ferulate as a sole carbon and energy source. As expected, the growth of the CUR-1 strain was entirely abolished on all tested phenylpropanoids.

Although, *C*. *glutamicum* CUR-1 was not able to utilize *p*-coumarate, caffeate, or ferulate as a growth substrate, its fitness in glucose based minimal medium was rather not affected. The CUR-1 strain exhibited similar growth behaviour like its parental wild type strain. Both strains consumed entire 10 g of glucose within 9.5 hours without significant change in maximum growth rate. The wild type reached µmax of 0.39 ± 0.02 h-1, while the strain lacking the *phdBCDE* genes grew with comparable growth rate of 0.36 ± 0.02 h-1. Biomass yield and specific glucose uptake rates were also without notable difference.

The newly generated *phdBCDE* mutant (CUR-1) was further assessed in minimal medium containing glucose and additional 5 mM ferulate. When exposed to ferulate, the wild type experiences a 1.5-fold decrease in its maximum growth rate, as opposed to cultivation with glucose as the only carbon source. Furthermore, in the presence of ferulate, the glucose uptake rate is also reduced, effectively leading to nearly two hours of extended growth to consume the provided 10 g of glucose. Utilization of glucose was completed within 11.45 h. It is notable, that during this time, only 2 mM of ferulate were consumed with a rate of 0.18 mmol h-1. When glucose was exhausted, the remaining 3 mM ferulate were consumed within one hour. The observed slow uptake of ferulate while glucose is still present in the medium, can be attributable to carbon catabolite repression. For most of bacteria, glucose is often preferred growth substrate from the mixture of different carbon sources.

The *phd* operon which is responsible for catabolism of ferulate and other phenylpropanoids is transcriptionally controlled by *phdR,* a MarR-type repressor (Kallscheuer et al., 2016, loc. cit.). PhdR-mediated repression is relieved once glucose is no longer present.

The deletion of *phdBCDE* genes moderately impacted growth of *C*. *glutamicum.* Specifically, the CUR-1 strain exhibited a decreased maximum growth rate of 0.20 ± 0.0 h-1, and glucose uptake rate of 4.88 ± 0.06 mmol h-1, compared to the wild type (P: .0035 and .0036, respectively). It is apparent that the deletion of *phdBCDE* genes also resulted in reduced biomass formation. Notably, deletion of the ferulate-catabolizing genes did not entirely abolish utilization of ferulate. Although at a significantly reduced rate, the CUR-1 strain consumed nearly 0.7 mM during first 12.5 hours while glucose was available. Subsequently, upon complete exhaustion of glucose, and until 72 hours of cultivation, the level of ferulate in the medium remained close to 4 mM.

The observed growth status of the CUR-1 strain in glucose-based minimal medium, with and without supplementation of ferulate, indicated good fitness albeit deletion of the *phdBCDE* genes. Therefore, the first chassis strain CUR-1 was selected as a host for further screening of curcumin biosynthetic gene clusters.

### 2. Turmeric dcs and curs(1) genes supported strong curcumin production in genetically engineered C. glutamicum

Genes were under transcriptional control of the native, strong constitutive tuf promoter, controlling the expression of translational elongation factor gene. In the designs with multiple genes, promoter sequence was positioned in front of the first gene, while the rest contained its own ribosomal binding site (RBS) (the last 20 bp from the tuf promoter sequence).

In order to ensure sufficient copy number, the biosynthetic genes were not integrated into the host's genome, but rather episomally expressed from the low-copy pClik5α plasmid. In order to mitigate possible undesired effect of high-copy plasmids, the low-copy number pClik5α was used as an expression vector.

The first variant of biosynthetic gene cluster comprised of a single *cus* gene from rice (O. *sativa*)*.* This gene encodes curcuminoid synthase (CUS) (GenBank accession number: LOC4342896) that can catalyse the "one-pot" synthesis of curcumin (Katsuyama et al., Microbiology (2008), ), 154: 2620-2628), bisdemethoxycurcumin (Morita et al., PNAS (2010), 107: 19778-19783), and dicinnamoylmethane (Katsuyama et al., (2008), *loc. cit.*)*.* CUS exhibits substrate-dependent specificity. The preferred substrate is p-coumaroyl-CoA, leading to the synthesis of bisdemethoxycurcumin through the condensation of two molecules of p-coumaroyl-CoA and one molecule of malonyl-CoA. Although it displays lower specificity, the enzyme can also accept feruloyl-CoA and cinnamoyl-CoA as precursors, catalyzing the synthesis of curcumin and dicinnamoylmethane, respectively.

In the current application, cultivation of *C*. *glutamicum* CUR-2_nat and CUR-2_co, derivatives of *C*. *glutamicum* CUR-1 expressing the OsCUS cluster in native and codon-optimized form, respectively, resulted in curcumin titres of 3 and 5 mg L⁻¹, in the same order as first mentioned. The codon-optimized form exhibited slightly higher titre of curcumin (P= .001).

The next cluster variant contained two type III PKS genes responsible for curcumin synthesis in turmeric (*C*. *longa*): diketide synthase (dcs) and curcumin synthase 1 (*curs1*) (GenBank accession numbers: AB495006 and KM880189, respectively. Briefly, the DCS enzyme catalyses synthesis of feruloyl-diketide-CoA, from one molecule feruloyl-CoA and malonyl-CoA. The CURS1 enzyme exerts dual function during biosynthesis of curcumin. Firstly, it hydrolases feruloyl-diketide-CoA in a corresponding keto acid, and second extends it with another molecule of feruloyl-CoA, to yield one molecule of curcumin (Katsuyama et al., J Biol Chem (2009), 284: 11160-11170).

In the present application, cultivation of *C*. *glutamicum* CUR-4_nat resulted in a titre of 8 mg L⁻¹ curcumin. On the other hand, 7 mg L⁻¹ of curcumin was produced by the strain expressing the cluster in the codon-adapted form, *C*. *glutamicum* CUR-4_co. In contrast to the first cluster variant expressing the *cus* gene from rice, the combination of dcs and *curs1* from turmeric performed better in the native codon sequence. However, the difference between the two sequence types was not statistically significant (P: .23).

In addition to dcs and *curs1* genes from *C*. *longa,* the final cluster variant contained a copy of *p*-coumaroyl:CoA ligase (*4cl1*) gene from tale cress (*A*. *thaliana*) (GenBank accession number: NC_003070.9). In *A*. *thaliana*, there are four isoforms of *4cl* genes, and they play indispensable role in directing carbon flow from primary metabolism to different routes of secondary metabolism. Specifically, they catalyse activation of phenylpropanoids into corresponding CoA-thioesters that are used in downstream synthesis of numerous secondary metabolites. Among the four isoforms, *4cl1* was selected as an additional source of feruloyl-CoA-synthetizing enzyme. Despite supplemental source of enzyme that can perform CoA-activation of ferulate, *C*. *glutamicum* CUR-5_nat and CUR-5_co did not produce more curcumin than the strain with only endogenous acyl:CoA ligase (*phdA*) (*C. glutamicum* CUR-4_nat and CUR-4_co). Overall, the strains *C*. *glutamicum* CUR-5_nat and CUR5_co produced 0.4 and 1.6 mg L-1 of curcumin, respectively.

After conducting screening of four biosynthetic gene clusters, it has been determined that the combination including the dcs and *curs1* genes from *C*. *longa* showed supreme performance compared to other cluster combinations. For this reason, this cluster variant has been chosen as the most suitable candidate for engineering further curcumin production cells.

### 3. Deregulation of ferulate uptake boosts curcumin production

*C*. *glutamicum* can withstand and grow in the presence of even 30 mM ferulate supplemented to the glucose-based (55 mM) minimal medium. Nevertheless, at this high concentration of ferulate, the growth of *C*. *glutamicum* appeared to be limited. Therefore, a reduced level of 5 mM ferulate was selected as a starting concentration for biotransformation of ferulate to curcumin in a glucose-based (55 mM) minimal medium by genetically engineered *C*. *glutamicum.*

In both CUR-6, the wild type derivative heterologously expressing the *dcs* and *curs1* genes from *C*. *longa*, and CUR-4_nat, the maximum growth rate was further slowed to 0.16 and 0.17 h⁻¹, respectively. Both strains exhibited comparable rates of glucose consumption, approximately 3.8 mmol h⁻¹. Furthermore, the uptake rate of ferulate demonstrated a significant reduction by 3.6-fold in *C*. *glutamicum* CUR-6, as compared to the wild type strain (Figure 1). Interestingly, the uptake rate of ferulate remained unaffected upon expression of an empty pClik5α plasmid in the wild type (P: .341). Additionally, glucose consumption showed a moderately reduced rate of 4.85 ± 0.04 (P: .0019).

In this application, antibiotic kanamycin was utilized as a selection marker for the maintenance of pClik5α_CIDCS_CURS1_nat plasmid. Antibiotics are commonly employed during bacterial fermentation to select for the cells carrying the desired plasmid. However, addition of antibiotics can lead to delay in growth of bacterial cells, here reflected by decreased uptake rate of glucose in *C*. *glutamicum* ATCC 13032 :: pClik5α. Nevertheless, once the strain was adapted to the presence of the antibiotic, it displayed a comparable maximum growth rate of 0.25 ± 0.01 h⁻¹. Moreover, results suggest that the observed substantial reduction in the uptake rate of ferulate in the CUR-6 strain, as compared to the plasmid-free wild type, is not linked to the kanamycin-imposed selection pressure. Instead, it is primarily related to the expression of the *dcs* and *curs1* genes and formation of curcumin.

In the wild type derivative expressing the pClik5α_CIDCS_CURS1_nat plasmid (CUR-6), the production of curcumin was growth coupled, with initial traces of the product detectable as early as 1.5 hour of cultivation (Figure 1A). After 27 hours of cultivation, the final titre of curcumin achieved by this strain was 2.5 mg L⁻¹, coinciding with the depletion of ferulate from the medium. On the other hand, two distinct phases were observed in the production profile of *C*. *glutamicum* CUR-4_nat, with deleted *phdBCDE* genes. The first phase commenced after a brief non-productive period (first 2.75 hours) and persisted until the beginning of the stationary phase, achieving a peak titre of 0.5 mg L⁻¹. Subsequent second production phase, spanning from 17.5 to 72 hours (Figure 1B), was characterized by approximately 3 fold higher volumetric and specific productivity rate, resulting in a maximum titre of 6 mg L⁻¹ of curcumin. Throughout the production process, the concentration of unutilized ferulate remained close to 4 mM, indicating that considerable amount of the curcumin precursor remains untouched. Evidently, greater part of curcumin was formed during the stationary phase, after the supplied glucose as the primary growth substrate was exhausted. Formation of curcumin from existing pools of feruloyl-CoA and malonyl-CoA was clearly not able to sustain high-level curcumin production.

With the intention of deleting *phdR* gene, the non-replicative plasmid pClik_intSacB_ΔphdR was made. The plasmid harboured 521 and 551 bp fragments homologous to the upstream and downstream sequences flanking the phdR gene in the CUR-1 background. Methylated form of plasmid was transformed in *C*. *glutamicum* CUR-1. First recombination transformants were selected against kanamycin resistance markers, encoded within the plasmid backbone. One isolated clone was subjected to second recombination via sacB-mediated counter selection. From approximately 50 clones analysed by colony PCR, 19 had abbreviated PCR products (431 bp compared to 1129 bp in CUR-1 background), indicating deletion of the *phdR* gene. One of the clones was further validated by sequencing, and named as CUR-7 strain. Derivative of the CUR-7 strain expressing the episomal plasmid with dcs and *curs1* genes was designated as CUR-8 strain. With the aim of evaluating the effect of *phdR* deletion in CUR-1 strain background, the CUR-8 strain was cultivated in minimal medium containing glucose and ferulate, in the same biotransformation set-up as its ancestor CUR-4_nat.

In the growth profile of CUR-8 strain, three individual phases were notable (Figure 2). First phase, representing early curcumin production phase, manifested with low curcumin productivity and titre. During this phase that lasted first 16 hours less than 5 mg L⁻¹ of curcumin were formed. Interestingly, early curcumin production phase fully overlapped with extended lag phase. As most of curcumin was formed during second phase, this period was designated as major production phase. Notably, it commenced with the beginning of exponential growth. During this stage, greatest portion of supplemented ferulate was consumed (close to 3 mM). Upon exhaustion of glucose ferulate uptake considerably ceased. Only minor fractions were utilized during the final third phase, distinguished by reduced curcumin productivity, as compared to the previous major production phase. Nevertheless, maximum titre of 110 mg L⁻¹, a 13-fold increase as compared to the parental strain with intact regulation of ferulate uptake. Elimination of PhdR-mediated regulation of ferulate catabolism resulted in remarkable upgrade of curcumin production in metabolically engineered *C*. *glutamicum.* Nonetheless, strain with deleted *phdRBCDE* genes showed enhanced curcumin production abilities.

### 4. Native promoter architecture of phdAT genes was optimal for ferulate uptake

As described, *phdT* and *phdA* genes are part of the *phd* operon, involved in catabolism of phenylpropanoids in C. *glutamicum.* Briefly, PhdT is a membrane-bound transporter, facilitating the uptake of phenylpropanoids.

With the objective of achieving consistent and robust expression of the *phdTA* genes, along with increasing the rate of ferulate uptake and synthesis of the curcumin precursor, feruloyl-CoA, overexpression of the *phdTA* genes was performed. The overexpression was accomplished by replacing the original *phdTA* promoter with the strong, constitutive tuf promoter along with simultaneous deletion of *phdRBCDE* genes in the wild type.

The new strain *C*. *glutamicum* Δ*phdRBCDE_phdTAP*tuf was designated as CUR-9 strain. When cultured in a microbioreactor, it exhibited an exceptionally prolonged lag phase of nearly 60 hours during growth on the mixture of glucose and ferulate. In addition to delayed growth, the strain display overall reduction in biomass formation. This growth behaviour was not observed in the medium containing only glucose. The absence of ferulate in the medium completely restored the typical growth phenotype, which closely resembles that of its ancestor wild type strain. Hence, it appears that only in the presence of ferulate, the growth is severely impaired. These results lead to the conclusion that the tuf promoter exerts overly strong transcriptional control, enhancing the uptake of ferulate and synthesis of feruloyl-CoA at the expense of strain fitness.

Therefore, an alternative, less vigorous approach was taken to boost the activity of the *phdTA* genes. In this second metabolic engineering design, the original *phdTA* promoter was positioned in front of the *phdT* gene, while the orientation of the *phdA* gene was reversed, and the gene was placed under transcriptional control of the tuf promoter. Genetic engineering was performed using CUR-1 as a parental strain, and the newly obtained strain was designates as CUR-10. The genetic modification has only marginally lessened the effect of ferulate on growth, shortening the lag phase to approximately 55 hours. Similar to the previous approach, the growth in the medium containing only glucose, remained comparable to that of the wild type.

However, growth of both of the newly generated CUR-9 and CUR-10 strains in the medium containing ferulate was manifested with more than two days long lag phase and reduced biomass formation by almost 30%. The observed growth defects were indicators of drastically decreased cellular fitness. Hence, the undesired reduced cellular viability pointed out that the overexpression of *phdTA* genes using the strong, constitutive tuf promoter was not the most suitable strategy to increase supply of ferulate and shows confied likelihood for the improvement of the strain performance.

### 5. Optimization of medium conditions for curcumin production

110 mg L⁻¹ of curcumin was produced in a biotransformation setup with 10 g L⁻¹ of glucose and 5 mM ferulate in a minimal medium by the CUR-8 strain. For industrially feasible productivity, higher cell densities are often desired. Considering the fact that rate of enzymatic reactions depends on substrate concentrations, it appeared relevant to investigate impact of different concentrations of ferulate as well. In that regard, the initial glucose concentrations tested were set to 10, 20, 30, and 40 g L⁻¹. Each alternative was tested in a combination with a varying starting concentration of ferulate (1, 2, 3, 4, and 5 mM), comprising in total 20 different screening conditions. Given the elevated production capacity of CUR-8 strain, the screening was carried out using this strain.

Among the tested conditions, a combination of 30 g L⁻¹ glucose and 5 mM ferulate resulted in the highest curcumin titre of 298 mg L⁻¹. Furthermore, a distinct trend in the production pattern was observed. When glucose was supplemented in concentrations of 10, 30, and 40 g L⁻¹, an increase in the initially provided ferulate from 1 to 5 mM, matched with the increase in final curcumin titre. The condition with 20 g L⁻¹ glucose was the only exception. Hereby, the supplementation with 4 mM ferulate resulted in higher curcumin titre when compared to 5 mM, albeit without statistical difference (P: .37). Despite the natural antimicrobial activity of ferulate (Sova, 2012; Zduńska et al., 2018), curcumin production was more boosted at the elevated concentrations of the biotransformation substrate (Figure 3).

Overall, curcumin levels increased from 172, to 286, and 298 mg L⁻¹ at 10, 20, and 30 g L⁻¹ glucose respectively, but then sharply decreased to 182 mg L⁻¹ at 40 g L⁻¹ glucose (Figure 3). Therefore, the increase in curcumin production in biotransformation setups with 10 to 30 g L⁻¹ glucose can be associated with increased formed biomass that can accommodate membrane-bound curcumin. Similarly, production of membrane-bound β-carotene astaxanthin by *C*. *glutamicum* was increased under high glucose concentration at 40 g L⁻¹, compared to 20 g L⁻¹. However, the titres decreased at more elevated glucose concentration beyond 40 g L⁻¹.

Taking into account the fact that 30 g L⁻¹ glucose in combination with 5 mM ferulate resulted in 2.7-fold enhanced production of curcumin, when compared to initial biotransformation setup with 10 g L⁻¹ glucose and 5 mM ferulate, subsequent cultivations were carried out in the optimized minimal medium containing 30 g L⁻¹ glucose and 5 mM ferulate.

### 6. Modification of the start codon of citrate synthase gltA genes

The native ATG start codon of citrate synthase, encoded by *gltA* gene, was exchanged with the rare GTG codon in the CUR-7 strain, which is devoid of the *phdRBCDE* genes. Modification of the start codon is a tool that can be used to lessen the gene expression levels. The exchange of the start codon in *gltA* gene was accomplished by two rounds of *sacB*-mediated counter-selection, and one positive clone, confirmed by PCR and sequencing, was designated as CUR-11 strain. Derivative of CUR-11 strain expressing the pClik5α_CIDCS_CURS1 curcumin biosynthetic cluster was named as CUR-12 strain.

In parallel to the strategy that aimed to decrease withdrawal of acetyl-CoA by the TCA cycle, a second approach focusing rather on strengthening the malonyl-CoA-forming pathway was pursued. Carboxylation of acetyl-CoA to malonyl-CoA is catalyzed by the multi-enzyme acetyl-CoA carboxylase complex, which is in case of *C*. *glutamicum* comprised of a ATP-dependent biotin carboxylase and biotin carboxyl carrier protein that form the α-subunit, carboxyltransferase that makes the β-subunit, and the carboxylase-associated ε-subunit, encoded by the *accBC, accD1*, and *accE* genes, respectively. Expression of *accBC* and accD1, as well as of the fatty acid synthase I (fasA) and II (fasB), is regulated by FasR, a TetR-type transcriptional repressor. Typical for a TetR-type regulator, FasR directly binds to the promoter regions of *accBC* and *accD1*, designated as *fasO* sites, thereby inhibiting their transcription.

In that regard, point mutations were introduced into *fasO* sites of *accBC* and *accD1* genes in CUR-7 strain. The exchange of nucleotides was accomplished step-wise, by first replacing the nucleotides in the *fasO* site of *accBC* gene, thereby using the CUR-7 as a parental strain. After two rounds of *sacB*-mediated counter-selection, one positive clone, validated by PCR and sequencing, named CUR-13, was used as a parent strain to introduce mutations in the *fasO* site of accD1 gene in the same manner, by using the sacB counter selection. A correct clone, obtained by two round of homologous recombination, and proven by PCR and sequencing, was named as CUR-14 strain. Derivative of CUR-14 strain expressing the pClik5α_CIDCS_CURS1 curcumin biosynthetic cluster was designated as CUR-15 strain.

In the interest of investigating the effects of introduced genetic mutations, CUR-12, CUR-15, as well as CUR-8, an ancestor in the strain lineage, were cultivated in shake flasks in minimal medium with supplementation of 30 g L⁻¹ glucose and 5 mM ferulate.

First insight into production performance of the tested strains revealed increased curcumin titre in the cultures of CUR-12 strain. Specifically, the strain produced 249 mg L⁻¹ curcumin, which is 18 mg more than the CUR-8 strain (curcumin producer in which start codon of citrate synthase as unchanged), demonstrating the value of engineering supply of acetyl-CoA in production of curcumin. CUR-15 strain, the curcumin producer with deregulated expression of *accBCD1* genes, produced slightly less, 233 mg L⁻¹. At the first sight, genetic engineering of acetyl-CoA and malonyl-CoA pathways had only a minor influence on production of curcumin.

First, a substantially long lag phase was apparent in growth profiles of all three strains, whereby first doubling of CUR-12 and CUR-15 strains occurred 16 hours after main cultures were inoculated. At this point, CUR-8 strain formed around 1.4-fold more biomass, when compared to CUR-12 and CUR-15 curcumin producers (Figure 4A, C, E). Slightly higher biomass formation at this stage of cultivation is likely linked to the exchange of start codon in *gltA* gene and derepression of *accBCD1* transcriptional control. These mutations perturbed native influx of acetyl-CoA into the TCA cycle and acetyl-CoA carboxylation rate, respectively. Once the strains adapted to the mutations, they caught up with the ancestor strain in terms of growth rate and maximum biomass formed.

In all three cultivated strains, three distinct productivity phases were apparent. First, phase I, an early production phase, which lasted until the strains began to grow exponentially. This took place after 27 hours in case of CUR-8, and slightly later, after 34 hours in case of CUR-12 and CUR-15 (Figure 3A, C, E). For all three strains, specific curcumin formation rate (qCur) and volumetric productivity (Pvol) were lower during the early production phase. Notably, this was most prominent in CUR-12 strain. The CUR-8 strain exhibited superior specific (Pspec) and volumetric productivity (Pvol) during this phase, when compared to CUR-12 and CUR-15 (Table S3), indicating potentially reduced fitness of acetyl-CoA and malonyl-CoA-engineered strains during an early stage of cultivation. Partially impaired cellular vitality of CUR-12 and CUR-15 was additionally demonstrated by less efficient utilization of glucose. This was reflected by reduced specific glucose uptake rate (qGlc).

Next, close to 80% of curcumin was produced during second phase, designated as major production phase. It coincided with the exponential growth phase, and lasted until the ferulate consumption rate ceased. Interestingly, in neither of tested strains, 5 mM ferulate were not completely depleted. Around 1.5 mM still remained untouched by the end of cultivation (Figure 3A, C, E). Moreover, uptake of ferulate in CUR-12 and CUR-15 strains diminished even before glucose was entirely consumed. Notably, curcumin productivity rates (Pvol and Pspec) were indistinguishable among the strains during the major production phase, whereby volumetric productivity was up to 7-fold higher, in comparison to the previous early production phase.

Finally, phase III, a late production phase, which manifested with lower volumetric and specific curcumin productivity than the previous phase. Production of curcumin plateaued during phase III (Figure 3A, C, E). Notably, in cultures of CUR-8 strain, with unmodified acetyl-CoA and malonyl-CoA pathways, levels of curcumin began to slightly drop towards the end of cultivation, indicating possible product degradation over time. However, in CUR-12 and CUR-15 strains curcumin degradation was not apparent. In contrast, the strains demonstrated moderate increase in concentration towards the last hours of cultivations. The marginally extended increase in produced curcumin was more evident in CUR-12 strain. It is during this stage that CUR-12 surpassed CUR-8 strain in terms of maximum titre, likely due to the increased concentration of intracellular malonyl-CoA, as demonstrated by the absolute quantification of intracellular-CoA thioesters (Figure 3B, D).

Sampling for the measurement of intracellular CoA-thioesters was carried out consistently at the beginning, in the middle, and at the end of exponential growth of all three cultivated strains. Evidently, expression of curcumin BGC strongly impacted levels of acetyl-CoA, malonyl-CoA, succinyl-CoA, and butyryl-CoA in the CUR-8 producer, as compared to the wild type expressing the empty pClik5α plasmid. Thereby concentration of malonyl-CoA plummeted by 20-fold during the major production phase. Diminished availability of the acetyl-CoA and ongoing channelling of malonyl-CoA into curcumin biosynthetic machinery, contributed to the dramatically reduced levels of one of the curcumin precursors. Disparate picture was seen in the levels of butyryl-CoA, which showed up to nearly 300-fold boost. Concentration of feruloyl-CoA was elevated by nearly 80% during at the beginning of the exponential growth phase, but declined sharply by the onset of the major production phase.

As demonstrated by enriched pools of malonyl-CoA in the CUR-12 curcumin producer, weakening the flow of acetyl-CoA in the TCA cycle, was beneficial for improving its supply and curcumin titre. The most notable increase of 11-fold was measured during mid-exponential phase. Overall, pools of butyryl-CoA in the CUR-12 strain were decreased, when compared to the CUR-8. The most prominent reduction of 16-fold was recorded at the end of exponential growth phase. Lower availability of butyryl-CoA in the samples from the TCA cycle-defective mutant can be potentially linked to replenishing of succinyl-CoA, the TCA cycle intermediate downstream from citrate, through methylmalonyl-CoA node.

### 7. Process optimization for curcumin production

*C*. *glutamicum* CUR-8 strain was grown in stirred-tank bioreactors, in three fermentation setups: high oxygen and high pH (20% dO₂; pH=6.8); low oxygen and high pH (2.5% dO₂; pH=6.8), and low oxygen and low pH (2.5% dO₂; pH=6.0). Taking into account that curcumin exhibits increased stability in the acidic environment, only pH values below 7.0 were considered. In each condition, the process started with the initial concentration of 90 g L⁻¹ glucose, as growth substrate, and 2.5 g L⁻¹ yeast extract, to support the cell vitality at acidic pH. In order to shorten the lag phase observed in cultivation profile of the CUR-8 strain in the shake flask experiments, ferulate was not added at beginning of the fermentation process. Instead, a 15 mM ferulate feed was added after hours of cultivation, which corresponds to two new generations, indicating good cell vitality. The goal of delayed addition of ferulate was to allow the cells to adapt to the conditions in the reactors, thereby reducing the stress from the exposure to the aromatic. In each set-up, kanamycin was added to the cultivation medium.

A highest curcumin titre of 392 mg L⁻¹ was obtained in the in the first condition where oxygen saturation was maintained at 20% level and pH at 6.8. It took the producer 88 hours to consume glucose entirely, whereas ferulate was depleted sooner, after 64 hours. Within this time, there was modest accumulation of 4.6 mM lactate (Figure 5A). Notably, the dynamics of ferulate consumption was peculiar. Namely, until 40 hours, the uptake rate was slow (0.17 h-1), suggesting that entire feed of 15 mM would not be utilized by the end of fermentation period. Surprisingly, HPLC measurements after 48 hours showed sharp decrease in the concentration of ferulate in the supernatant of culture broth. The acceleration of ferulate utilization also coincided with the revived growth of the cells that previously stopped already after 22 hours of fermentation. Moreover, maximum formed biomass was 15 g L⁻¹, which is by 33% more than in the other tested conditions. This brings an assumption that the during this fermentation period, a mutation, which allows the phdRBCDE-deficient strain to rapidly consume ferulate, occurred.

Lowering the oxygen saturation to 2.5% turned out to be unfavourable for curcumin production, reducing the curcumin titre by 42% to 224 mg L⁻¹. Given that nearly two thirds of the ferulate feed were still present after 65 hours, and that staggering amount of almost 500 mM lactate accumulated within this time, fermentation was terminated at this time point. The growth stopped fairly early after 22 hours, which exactly matches the time when lactate formation surged (Figure 5B). Curcumin production abated when lactate level reached 122 mM.

Combination of oxygen limitation and lower pH had even more detrimental effect on the production, resulting in 161 mg L⁻¹ of curcumin formed (Figure 5C). The trends in growth, lactate formation, ferulate utilization, and curcumin production are very alike to the ones in previous condition. However, within the 65 hours period, more than 200 mM glucose remained untouched by the strain, explaining the 2-fold lower accumulation of lactate, when compared to condition with higher pH.

This set of observations shows that overdone oxygen limitation and low pH are not advantageous for curcumin production. Rather, high oxygen levels and moderately acidic conditions support higher production of curcumin. For this reason, in the following fermentation experiments, dissolved oxygen and pH were set to 20% 6.8, respectively.

### 8. Fed-batch process

Given that increased concentration of glucose and ferulate did not result in considerable increase in the curcumin titre, the CUR-8 strain was further evaluated in a fed-batch process. Oxygen saturation and pH were maintained at 20% and 6.8, respectively.

The initial concentration of glucose was set to 30 g L⁻¹, and subsequent pulses of 30 g L⁻¹ glucose and 5 mM ferulate were given manually from concentrated stock solutions (500 g L⁻¹ and 500 mM, respectively). During the first batch, glucose was consumed within 30 hours with a rate of 6.63 mM h-1 (Figure 6A). First ferulate pulse was given after four hours, and was consumed in 26 hours with a rate of 0.22 mM h⁻¹. Notably, ferulate and glucose were co-utilized, and curcumin production set-on immediately after the ferulate feed. Throughout the first batch 338 m L⁻¹ of curcumin were produced. The second pulse of glucose was given once the initially provided glucose was depleted, indicated by increase in dissolved oxygen and confirmed by HPLC measurement. Given that the second ferulate pulse was given at the time when glucose was completely exhausted, the strain experienced plateau in growth, but picked up as soon as the second glucose pulse was injected. During the second phase, glucose uptake rate increased to 7.29 mM h⁻¹, whereas ferulate uptake slowed down, demonstrated by reduced rate of 0.13 mM h⁻¹. Glucose and ferulate were still co-consumed, and curcumin formation was growth coupled, reaching 692 mg L⁻¹ prior the third feed (Figure 6A). In order to provide strain with a sufficient amount of glucose, a third pulse was injected prior complete exhaustion of the second feed. In the course of third feed consumption of glucose was minors, whereas ferulate concentration did not decrease since the third pulse. Nonetheless, production of curcumin proceeded, likely from already created pools of feruloyl-CoA, reaching 806 mg L⁻¹ after 88 hours of cultivation. Notably, this is the highest curcumin titre obtained by microbial fermentation, reported to date.

In view of increasing productivity and reducing cultivation time, another fermentation with four times higher cell density of the inoculum was put to the test. Initial concentration of glucose was set to 20 g L⁻¹, and similar to the previous fermentation, 5 mM ferulate and 30 g L⁻¹ glucose pulses were added manually (Figure 6B). Thereby, first ferulate pulse was injected after four hours. Consumption of ferulate started without delay, with a 68% faster rate (0.37 mM h⁻¹) than in the fermentation with low cell density inoculum. Glucose was also utilized with a quicker rate (9.28 mM h⁻¹). Consequently, second pulse of ferulate was injected almost 12 hours earlier. By the time the first ferulate feed was nearly exhausted, 276 mg L⁻¹ of curcumin were formed. Notably, second glucose pulse was utilized with 64% higher rate than during the first batch (15.24 mM h⁻¹). As a results, another pulse of glucose was given to enable consumption of the second ferulate feed. Regardless, ferulate uptake rate plunged to 0.27 mM h⁻¹, and eventually the third pulse remained untouched. The highest curcumin titre of 612 mg L⁻¹ was recorded after 38 hours of cultivation (Figure 6B), which corroborates with the end of second feeding with ferulate. After 50 hours curcumin level slightly decreased, likely due to dilution effect of fourth glucose and third ferulate pulse. Although curcumin titre in this fermentation was nearly 200 mg L⁻¹ lower than in the previous set-up, in terms of volumetric productivity high cell density inoculum strategy outperformed the low cell density inoculum approach.

Overall, in both fermentation strategies the CUR-8 strain could sustain two 5 mM ferulate pulses. Apropos curcumin titre, both approaches surpassed the so far reported titres, indicating that the herein presented genetically engineered *C*. *glutamicum* is an excellent host for production of curcumin.

## Claims

1. A gram-positive bacterial host cell, which naturally has a phenylpropanoid pathway, wherein said bacterial host cell is genetically engineered to express curcuminoid biosynthetic genes, thereby being capable of producing curcumin.

2. The bacterial host cell of claim 1, wherein said curcuminoid biosynthetic genes encode one or more selected from the group consisting of phenylalanine hydroxylase (PAH), phenylalanine ammonia lyase (PAL), tyrosine ammonia lyase (TAL), Cinnamate 4-hydroxylase (C4H), 4-coumarate 3-hydroxylase (C3H), Caffeic acid O-methlytransferase (COMT), Caffeoyl-CoA O-methyltransferase (CCoAOMT), 4-coumarate-CoA ligase (4CL), diketide-CoA synthase (DCS), curcuminoid synthase (CUS), curcumin synthase 1 (CURS1), curcumin synthase 2 (CURS2), and curcumin synthase 3 (CURS3).

3. The bacterial host cell of claim 2, wherein said
g) curcuminoid synthase (CUS) has a polypeptide sequence at least about 70% similar to the polypeptide sequence shown in SEQ ID: 1 or 2,
h) diketide-CoA synthase (DCS) has a polypeptide sequence at least about 70% similar to the polypeptide sequence shown in SEQ ID: 3 or 4,
i) curcumin synthase 1 (CURS1) has a polypeptide sequence at least about 70% similar to the polypeptide sequence shown in SEQ ID: 5 or 6,
j) curcumin synthase 2 (CURS2) has a polypeptide sequence at least about 70% similar to the polypeptide sequence shown in SEQ ID: 7,
k) curcumin synthase 3 (CURS3) has a polypeptide sequence at least about 70% similar to the polypeptide sequence shown in SEQ ID: 8, and/or
l) 4-coumarate-CoA ligase (4CL) has a polypeptide sequence at least about 70% similar to the polypeptide sequence shown in SEQ ID: 9 or 10.

4. The bacterial host cell of any one of the preceding claims, said curcuminoid biosynthetic genes expressed in the corynebacterium (e.g., *C*. *glutamicum*) host cell encode
d) a polypeptide sequence at least about 70% similar to the polypeptide sequence shown in SEQ ID: 3 or 4,
e) a polypeptide sequence at least about 70% similar to the polypeptide sequence shown in SEQ ID: 5 or 6, and
f) optionally a polypeptide sequence at least about 70% similar to the polypeptide sequence shown in SEQ ID: 9 or 10.

5. The bacterial host cell of any one of the preceding claims, wherein said bacterial host cell is genetically engineered to be reduced in catabolizing phenylpropanoids into further metabolic products for energy production.

6. The bacterial host cell of claim 5, wherein in said bacterial host cell *phdBCDE* genes encoding 3-hydroxyacyl-CoA dehydrogenase, metal-dependent hydrolase, acyl-CoA dehydrogenase, and enoyl-CoA hydratase, respectively, are inactivated.

7. The bacterial host cell of any one of the preceding claims, wherein in said bacterial host cell the functional gene encoding PhdR is inactivated.

8. The bacterial host cell of any one of the preceding claims, wherein said bacterial host cell is genetically engineered to have increased expression of the *phdA* gene encoding long-chain fatty acid-CoA ligase.

9. The bacterial host cell of any one of the preceding claims, wherein in said bacterial host cell *benABCD* genes encoding encoding benzoate 1,2-dioxygenase large subunit, benzoate 1,2-dioxygenase small subunit, benzoate 1,2-dioxygenase electron transfer component, 1,6-dihydroxycyclohexa-2,4-diene-1-carboxylate dehydrogenase, respectively, are inactivated.

10. The bacterial host cell of any one of the preceding claims, wherein said bacterial host cell is genetically engineered to have attenuated expression of the gene encoding citrate synthase.

11. The bacterial host cell of claim 10, wherein said bacterial host cell is genetically engineered to have GTG as start codon of the gene encoding citrate synthase.

12. The bacterial host cell of any one of the preceding claims, which is a Corynebacterium host cell, Bacillus host cell, Actinobacter host cell, or Streptomycetes host cell.

13. A method for producing curcumin, comprising culturing the bacterial host cell of any one of claims 1 to 12 under suitable conditions in liquid medium and harvesting curcumin.

14. The method of claim 13, wherein said liquid medium comprises ferulate, preferably up to 5 mM ferulate.

15. The method of claim 13 or 14, wherein said liquid medium comprises glucose, preferably up to 30 g/L glucose.
